# EUROPEAN PATENT SPECIFICATION

(11) **EP 3 202 401 B1**
(45) Date of publication and mention of the grant of the patent: **23.10.2019**
(21) Application number: 16197227.8
(22) Date of filing: 15.12.2010
(51) Int. Cl.: A61K 31/426, A61K 31/4439, A61P 3/10

(54) **PPAR-SPARING THIAZOLIDINEDIONES AND COMBINATIONS FOR THE TREATMENT OF NEURODEGENERATIVE DISEASES**
PPAR-UMGEHENDE THIAZOLIDINEDIONE UND KOMBINATIONEN ZUR BEHANDLUNG NEURODEGENERATIVER ERKRANKUNGEN
THIAZOLIDINEDIONES PPAR-SPARING ET COMBINAISONS POUR LE TRAITEMENT DE MALADIES NEURODÉGÉNÉRATIVES

(30) Priority: 15.12.2009 US 286765 P; 15.12.2009 US 286713 P
(43) Date of publication of application: 09.08.2017
(62) Divisional of application: 10798884.2
(73) Proprietor: Cirius Therapeutics, Inc., Kalamazoo, MI 49007 (US)
(72) Inventor: COLCA, Gerard R., Kalamazoo MI 49007 (US); KLETZIEN, Rolf F., Richland MI 49083 (US); TANIS, Steven P., Carlsbad CA 92011 (US); LARSEN, Scott D., South Lyon MI 48178 (US)
(74) Representative: Cohausz & Florack

(56) References cited:
- WO-A1-2009/038681
- WO-A1-2010/105048
- WO-A2-2007/109024
- GELDMACHER D S ET AL: "P1-397 Pioglitazone in Alzheimer's disease: rationale and clinical trial design", NEUROBIOLOGY OF A, TARRYTOWN, NY, US, vol. 25, 1 July 2004 (2004-07-01), pages S211-S212, XP004625323, ISSN: 0197-4580, DOI: 10.1016/S0197-4580(04)80709-7

## Description

### SUMMARY OF THE INVENTION

The present invention relates to a compound that has reduced binding and/or activation of the nuclear transcription factor PPARy. Contrary to the teachings of the literature, the PPARy sparing compound of the present invention shows beneficial neurological properties.

The compound of this invention has reduced binding and/or activation of the nuclear transcription factor PPARy, does not augment sodium re-absorption, and is useful in treating or preventing several neurodegenerative disorders. Advantageously, the compound having lower PPARy activity exhibits fewer side effects than compounds having higher levels of PPAPγ activity.

The present invention provides a sodium salt of Compound X 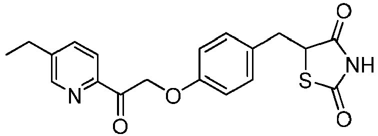 for use in treating Alzheimer's Disease.

Also described herein is a method for treating and/or preventing neurodegenerative diseases (e.g., Alzheimer's Disease, Parkinson's Disease, Amyotrophic Lateral Sclerosis (ALS), Muscular Sclerosis (MS), or Mild Cognitive Impairment (MCI)) comprising administering to a patient a compound of Formula I: 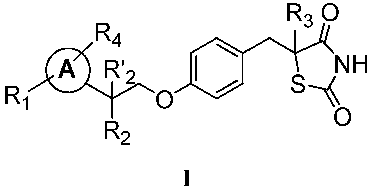 or a pharmaceutically acceptable salt thereof, wherein each of R₁ and R₄ is independently selected from H, halo, aliphatic, and alkoxy, wherein the aliphatic or alkoxy is optionally substituted with 1-3 of halo; R'₂ is H; R₂ is H, halo, hydroxy, or optionally substituted aliphatic, -O-acyl, -O-aroyl, -O-heteroaroyl, -O(SO₂)NH₂, -O-CH(Rₘ)OC(O)Rₙ, -O-CH(Rₘ)OP(O)(ORₙ)₂, -O-P(O)(ORₙ)₂, or 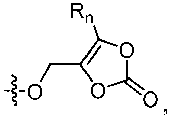 wherein each Rₘ is independently an optionally substituted C₁₋₆ alkyl, each Rₙ is independently C₁₋₁₂ alkyl, C₃₋₈ cycloalkyl, or phenyl, each of which is optionally substituted, or R₂ and R'₂ together form oxo; R₃ is H or optionally substituted C₁₋₃ alkyl; and ring A is a phenyl, pyridin-2-yl, pyridin-3-yl, or pyridin-4-yl, each of which is substituted with an R₁ group and an R₄ group at any chemically feasible position on ring A.

In some methods described herein, the neurodegenerative disorder comprises Alzheimer's Disease, Parkinson's Disease, ALS, MS, MCI, or any combination thereof.

In some instances, R₃ is H.

In some instances, R₃ is CH₃.

In some instances, R₄ is H, methyl, methoxy, ethyl, ethoxy, -O-isopropyl, -CF₃, - OCHF₂ or -OCF₃. For example, R₄ is H.

In some instances, R₁ is H, alkyl, halo or alkoxy. For example, R₁ is H. In other examples described herein, R₁ is halo. In some examples described herein, R₁ is C₁₋₃ alkyl.

In some instances, ring A is phenyl that is substituted with R₁ and R₄ groups at any chemically feasible position on ring A. In some examples described herein, ring A is phenyl, and one of R₁ or R₄ is attached to the para or meta position of ring A. In other examples described herein, ring A is phenyl, and one of R₁ or R₄ is attached to the meta position of ring A. In some examples described herein, R₁ is attached to the para or meta position of ring A. And, in some examples described herein, R₁ is F or Cl, either of which is attached to the para or meta position of ring A. In other examples described herein, R₁ is alkoxy (e.g., methoxy, ethoxy, propoxy, -O-isopropyl, butoxy, or -O-tertbutyl) that is attached to the para or meta position of ring A. In other examples described herein, ring A is phenyl, and R₁ is attached to the meta or ortho position of the phenyl ring. For instance as described herein, ring A is phenyl, and R₁ is attached to the ortho position of the phenyl ring. In some instances, ring A is phenyl, and R₁ is methoxy, ethoxy, or -O-isopropyl, any of which is attached to the ortho position of ring A. In other instances, R₁ is -CF₃, -OCHF₂ or -OCF₃.

In some instances, ring A is optionally substituted pyridin-2-yl or optionally substituted pyridin-3-yl, either of which is substituted with R₁ and R₄ groups at any chemically feasible position on ring A. In some examples described herein, ring A is pyridin-2-yl, and one of R₁ or R₄ is attached to the 5 position of the ring. In other examples described herein, ring A is pyridin-3-yl, and one of R₁ or R₄ is attached to the 6 position of the ring. In some examples described herein, ring A is pyridin-2-yl, and R₁ is attached to the 5 position of the ring. For instance, ring A is pyridin-2-yl, and R₁ is alkyl or alkoxy, either of which is attached to the 5 position of ring A. In other instances, ring A is pyridin-2-yl, and R₁ is methyl, ethyl, propyl, isopropyl, butyl, or tertbutyl, any of which are attached to the 5 position of ring A.

In some instances, R'₂ is H.

In some instances, R₂ is hydroxy.

In some instances, R₂ is -O-acyl, -O-aroyl, or -O-heteroaroyl.

In some instances, R₂ and R'₂ together form oxo.

In some instances, the compound of Formula I is one selected from:

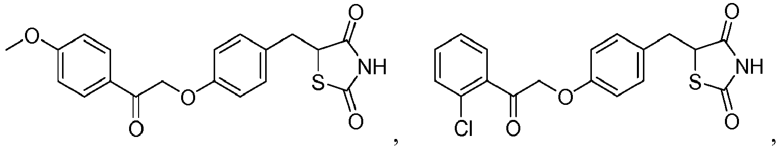

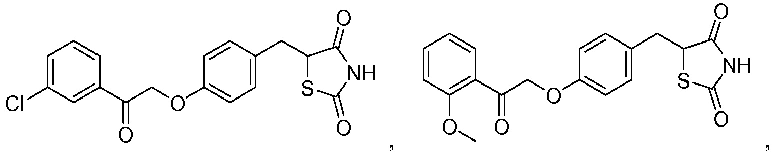

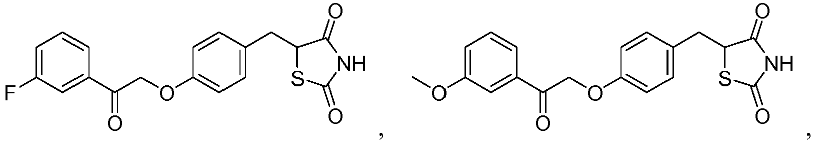

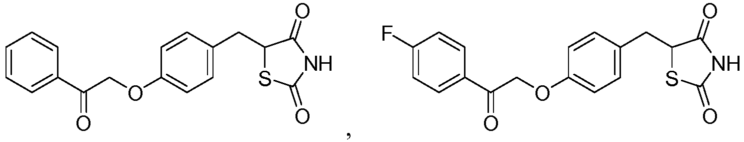

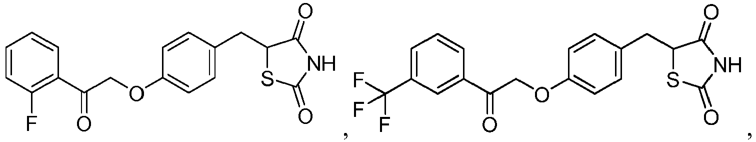

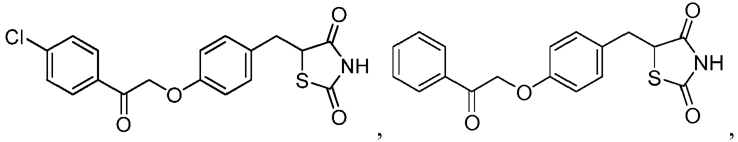

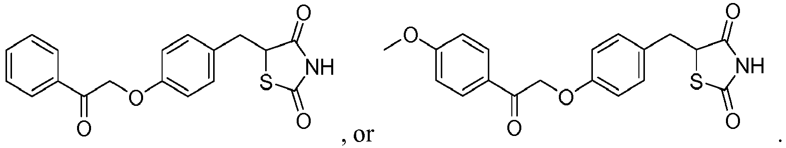

In some instances, the compound of Formula I is one selected from: 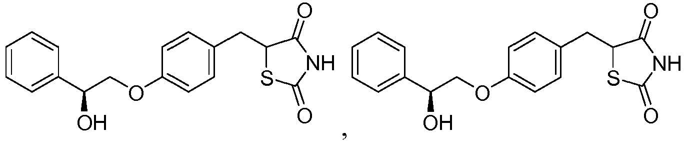 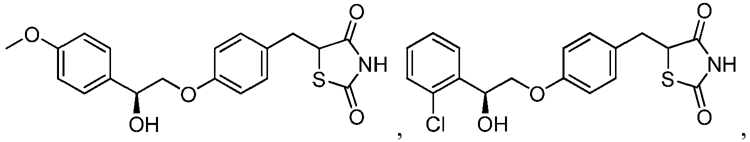 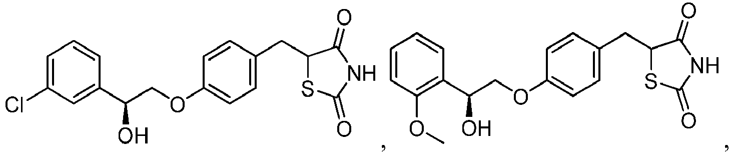 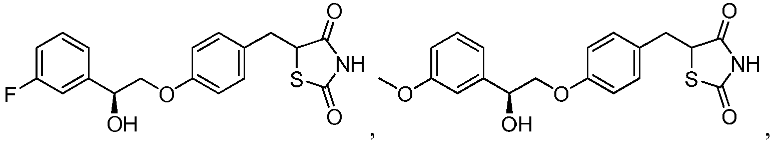 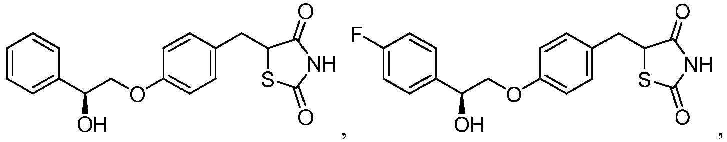 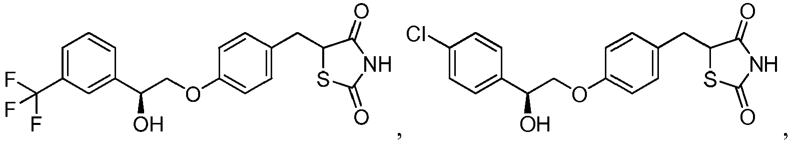 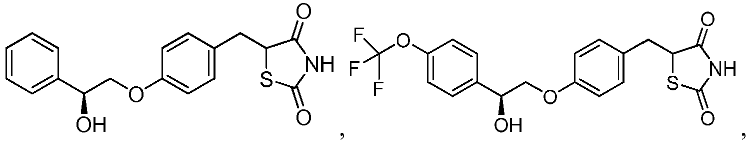 or

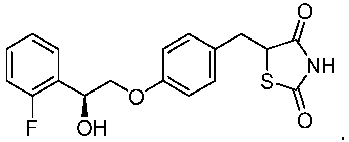

In some instances, the compound of Formula I is one selected from:

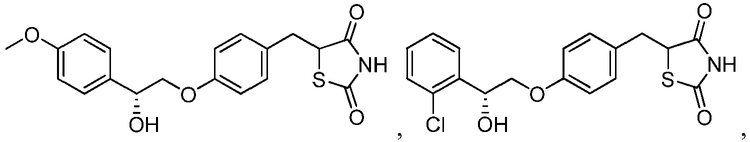

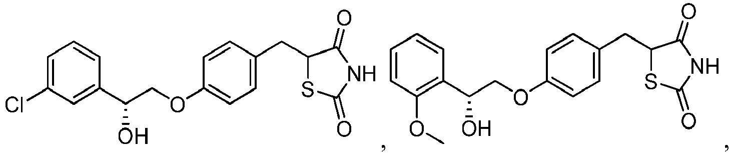

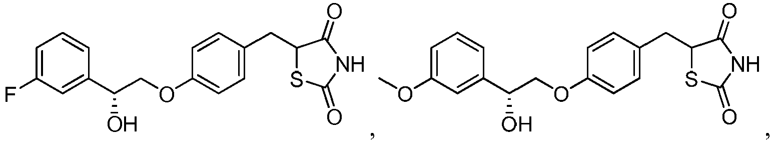

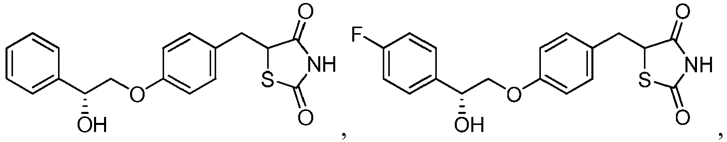

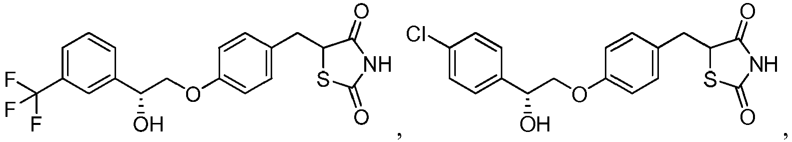

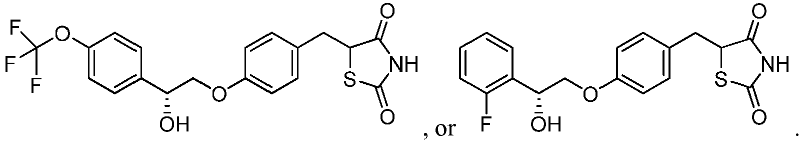

In some instances, the compound of Formula I is one selected from:

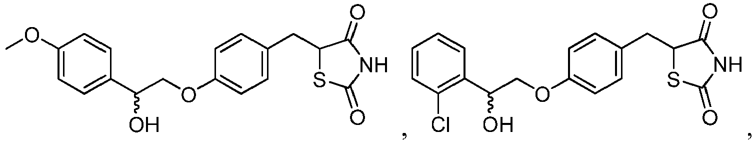

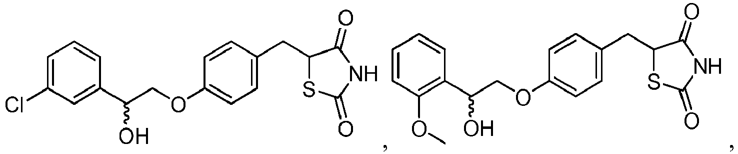

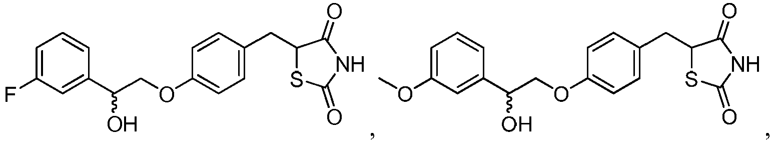

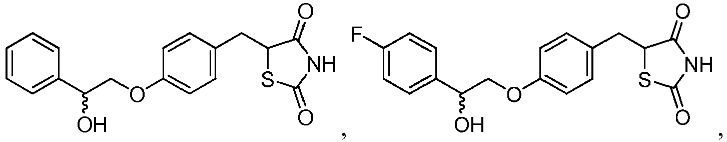

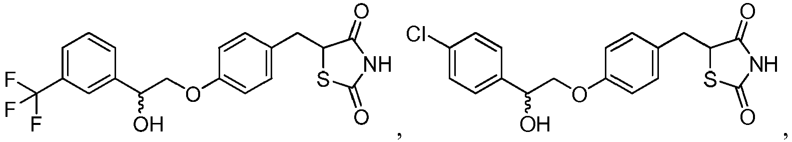

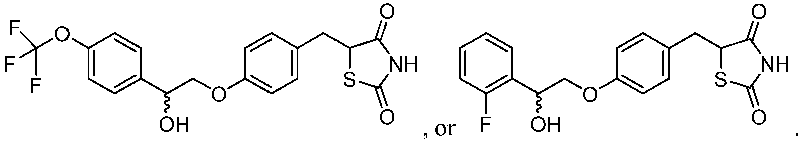

In some instances, the compound of Formula I is one selected from: 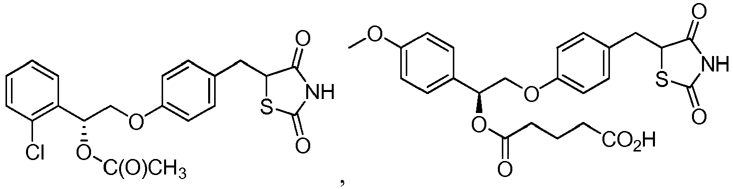 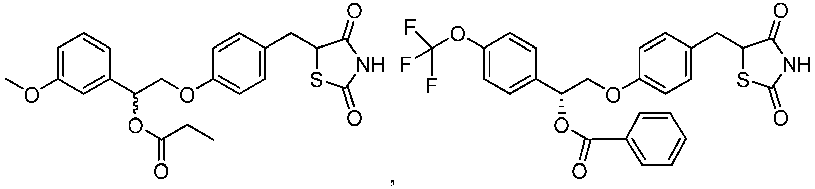 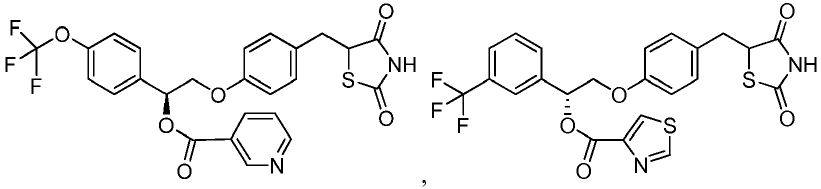 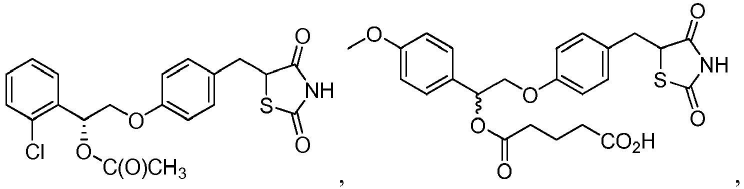 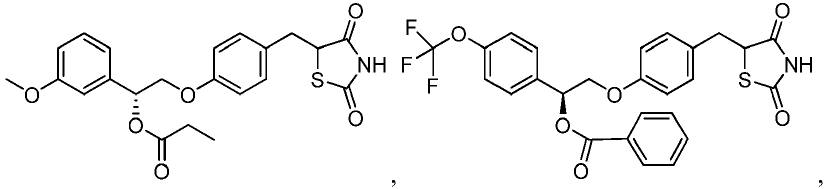 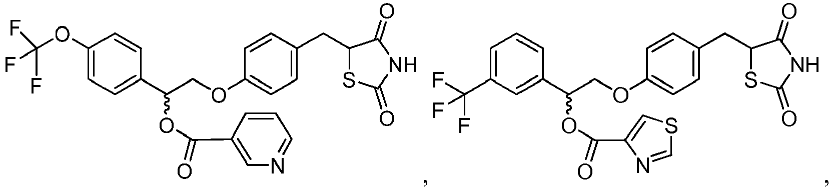 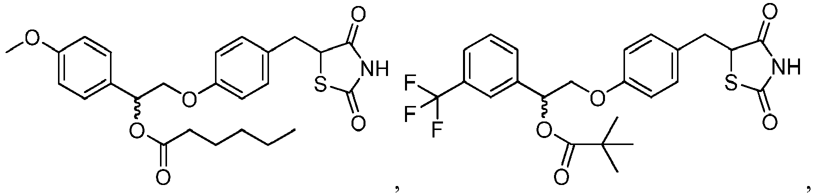 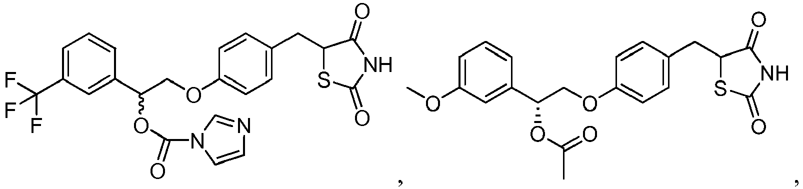 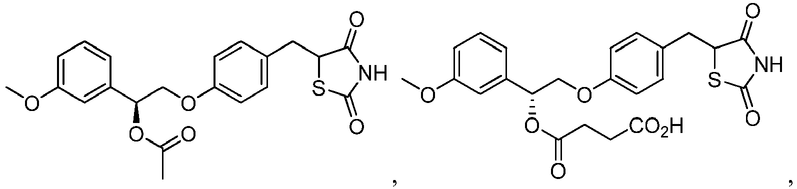 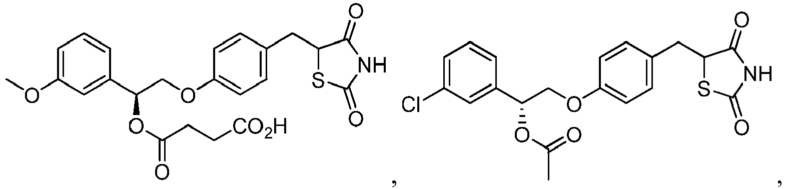 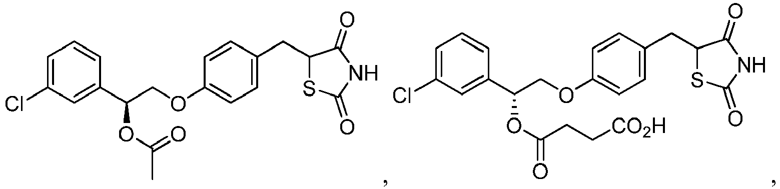 or

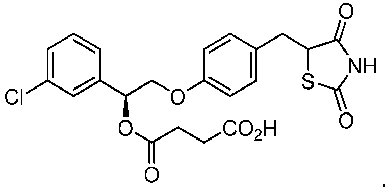

In some instances, the compound of Formula I is one selected from:

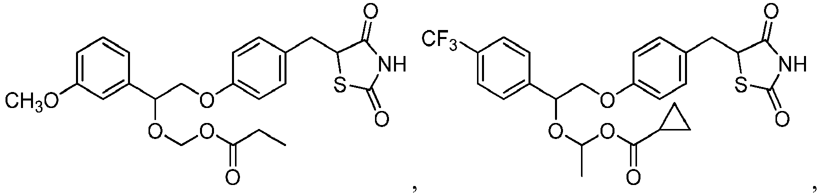

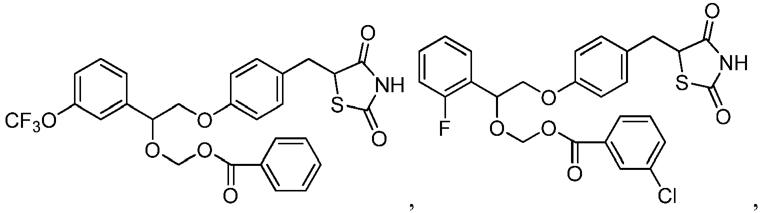

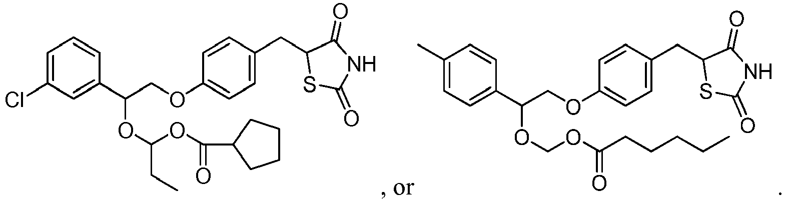

In some instances, the compound of Formula I is one selected from:

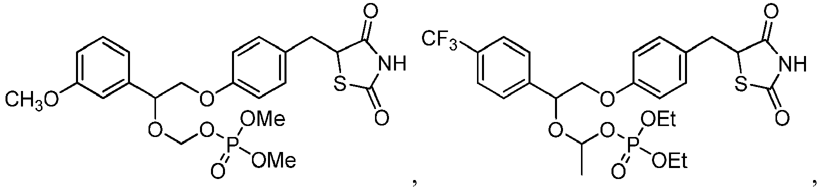

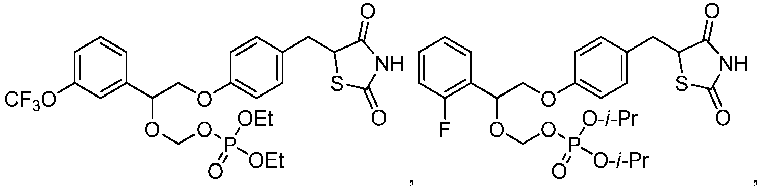

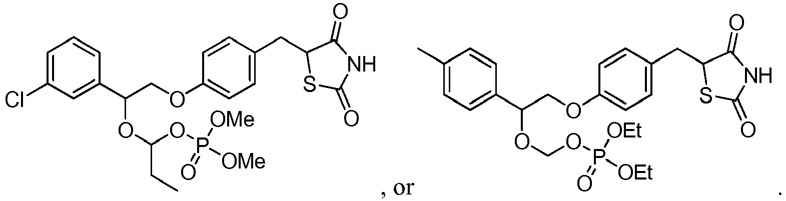

In some instances, the compound of Formula I is one selected from: 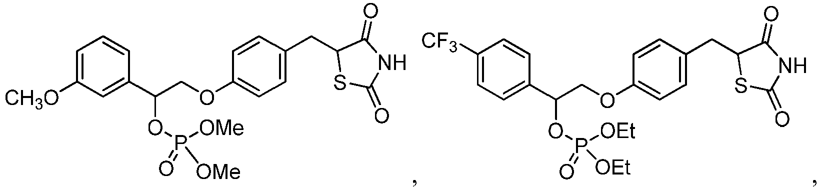 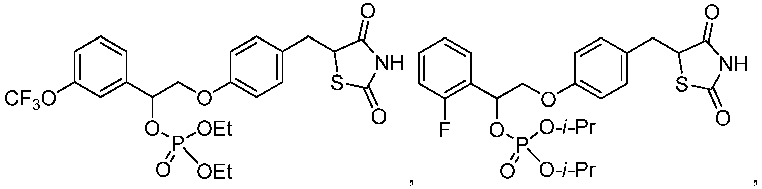 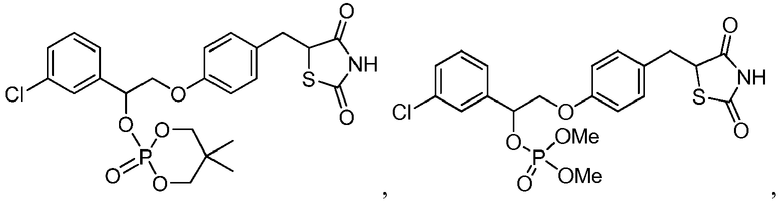 or

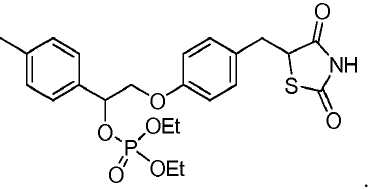

In some instances, the compound of Formula I is one selected from:

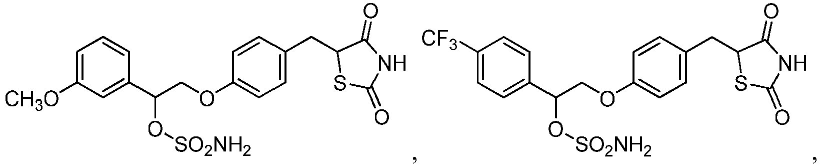

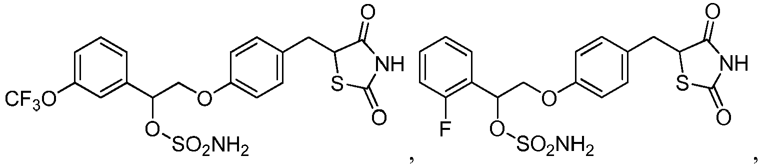

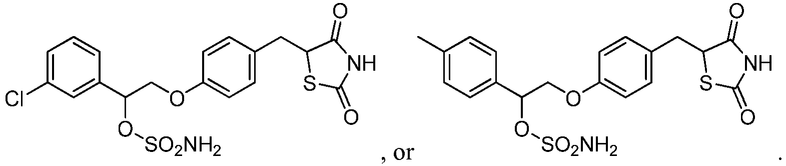

In some instances, the compound of Formula I is one selected from:

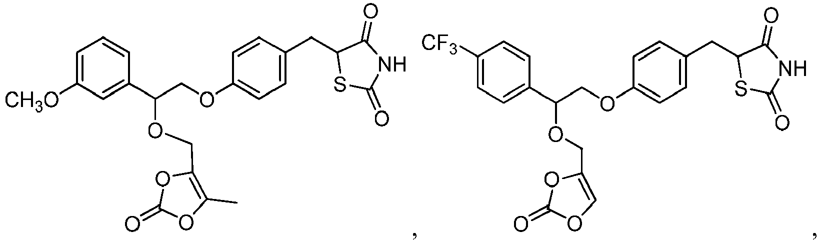

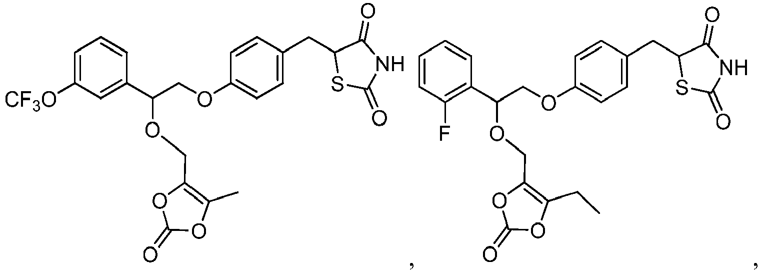

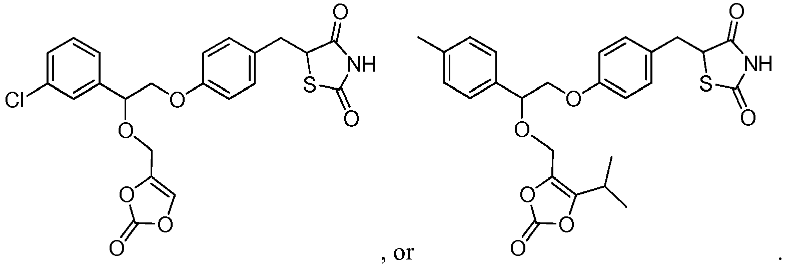

In some instances, the compound of Formula I is one selected from: 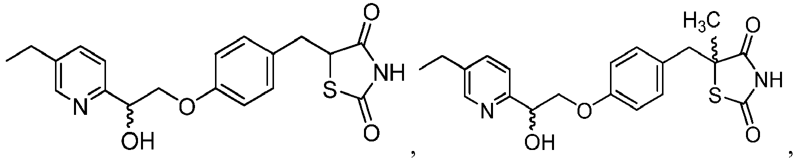 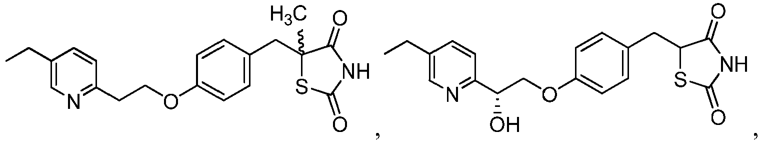 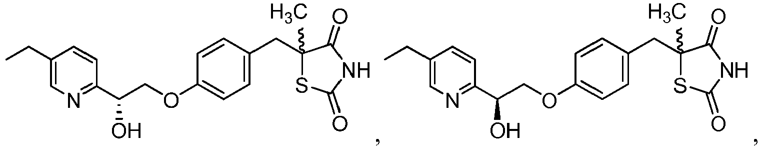 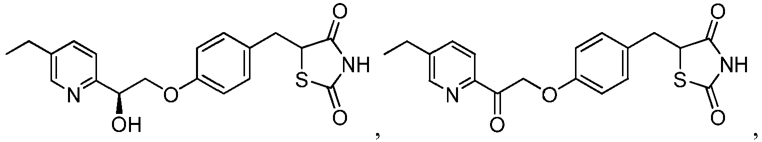 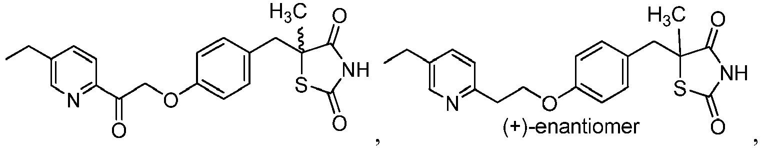 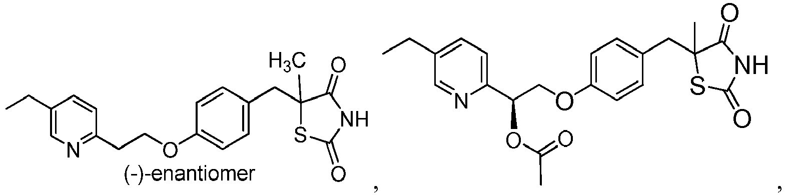 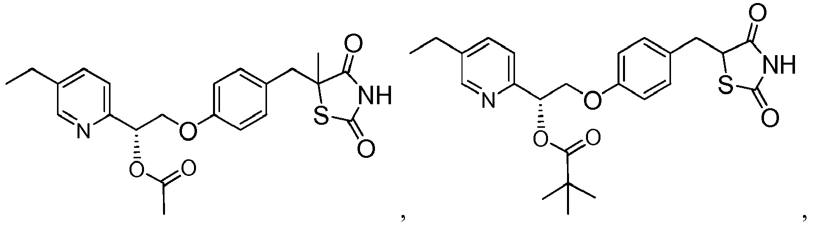 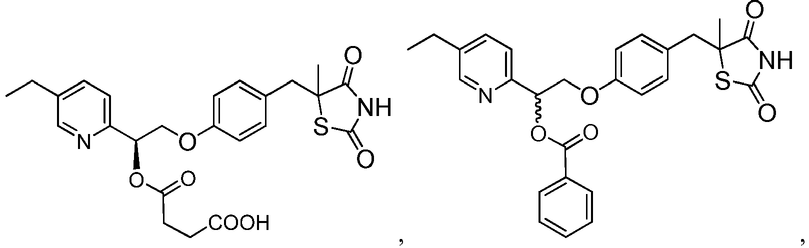 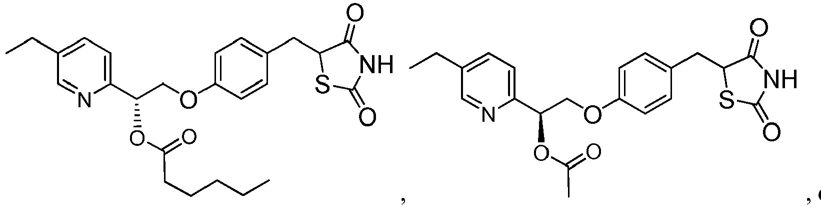 or

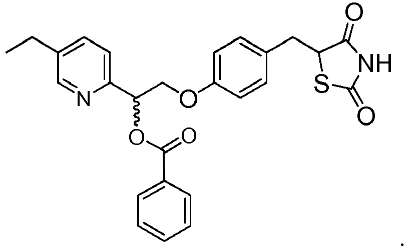

In some instances, the compound of Formula I is one selected from:

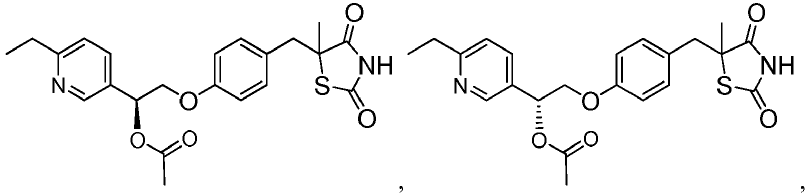

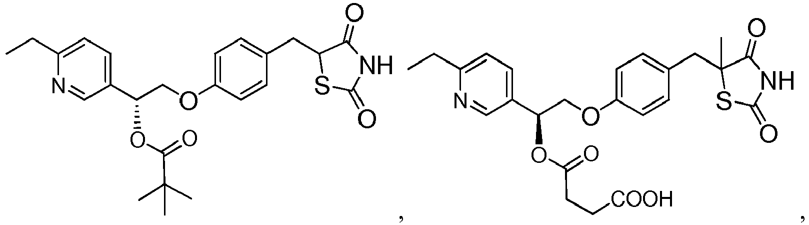

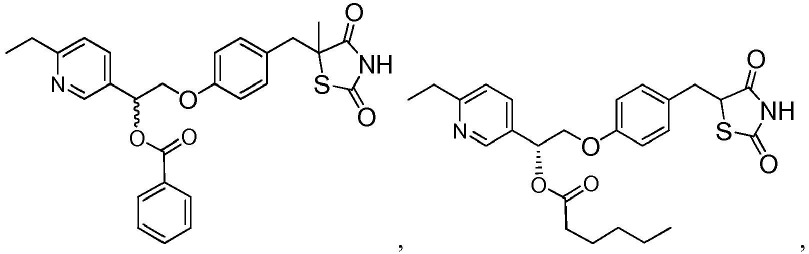

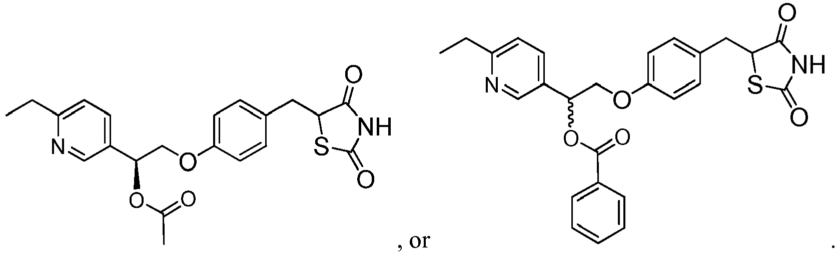

In some instances, the compound of Formula I is one selected from: 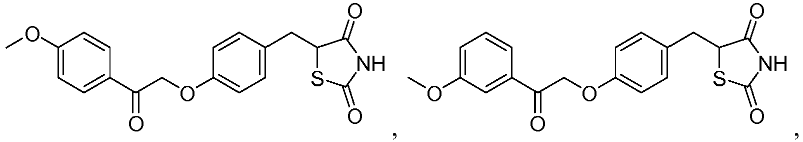 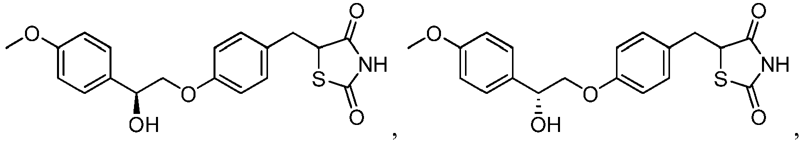 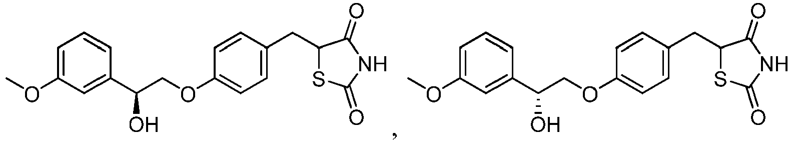 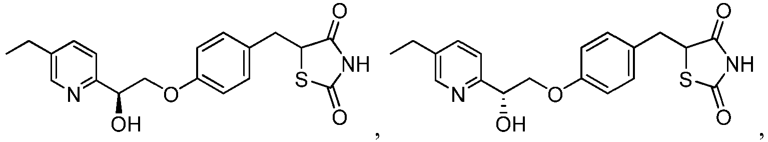 or

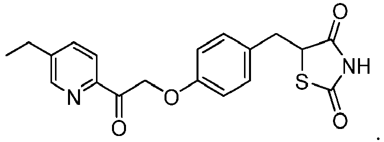

Some instances further comprise administering a phosphodiesterase inhibitor to the patient.

Some instances further comprise administering to the patient another pharmaceutical agent having an activity that increases cAMP in the patient.

In some instances, the second pharmaceutical agent further comprises a beta-adrenergic agonist. For example, the beta-adrenergic agonist comprises a beta-1- adrenergic agonist, a beta-2-adrenergic agonist, a beta-3-adrenergic agonist, or any combination thereof. In other examples, the beta-adrenergic agonist comprises noradrenaline, isoprenaline, dobutamine, salbutamol, levosalbutamol, terbutaline, pirbuterol, procaterol, metaproterenol, fenoterol, bitolterol mesylate, salmeterol, formoterol, bambuterol, clenbuterol, indacaterol, L-796568, amibegron, solabegron, isoproterenol, albuterol, metaproterenol, arbutamine, befunolol, bromoacetylalprenololmenthane, broxaterol, cimaterol, cirazoline, denopamine, dopexamine, epinephrine, etilefrine, hexoprenaline, higenamine, isoetharine, isoxsuprine, mabuterol, methoxyphenamine, nylidrin, oxyfedrine, prenalterol, ractopamine, reproterol, rimiterol, ritodrine, tretoquinol, tulobuterol, xamoterol, zilpaterol, zinterol, or any combination thereof.

Further described herein isa method of treating or preventing a neurodegenerative disorder comprising administering to a patient a pharmaceutical composition comprising a compound of Formula I, as described above, and a phosphodiesterase inhibitor.

In some instances, the phosphodiesterase inhibitor comprises a non-selective inhibitor. For example, the phosphodiesterase inhibitor comprises caffeine (1,3,7-trimethylxanthine), theobromine (3,7-dimethyl-2,3,6,7-tetrahydro-1H-purine-2,6-dione), theophylline (1,3-dimethyl-7H-purine-2,6-dione), IBMX (3-isobutyl-1-methylxanthine), or any combination thereof.

In some instances, the phosphodiesterase inhibitor comprises a selective inhibitor. For example, the selective phosphodiesterase inhibitor comprises Milrinone (2-methyl-6-oxo-1,6-dihydro-3,4'-bipyridine-5-carbonitrile), Cilostazol (6-[4-(1-cyclohexyl-1H-tetrazol-5-yl)butoxy]-3,4-dihydro-2(1H)-quinolinone), Cilomilast (4-cyano-4-(3-cyclopentyloxy-4-methoxyphenyl)cyclohexane-1-carboxylic acid), Rolipram (4-(3-cyclopentyloxy-4-methoxyphenyl)pyrrolidin-2-one), Roflumilast (3-(cyclopropylmethoxy)-N-(3,5-dichloropyridin-4-yl)-4-(difluoromethoxy)benzamide), or any combination thereof.

Also described herein is a method of treating or preventing a neurodegenerative disorder comprising administering to a patient a pharmaceutical composition comprising a compound selected from: 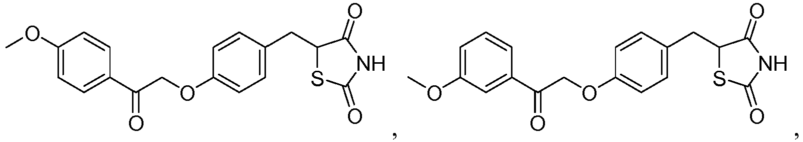 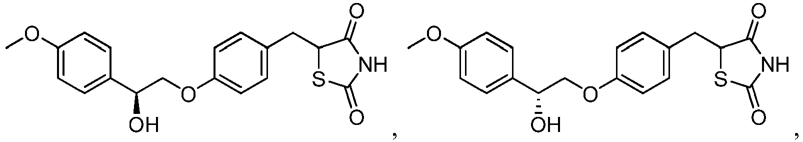 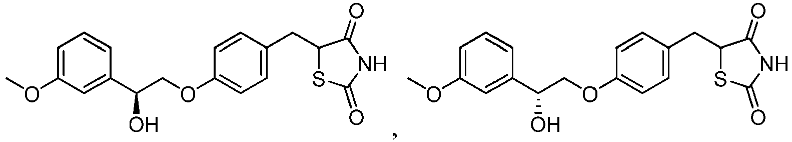 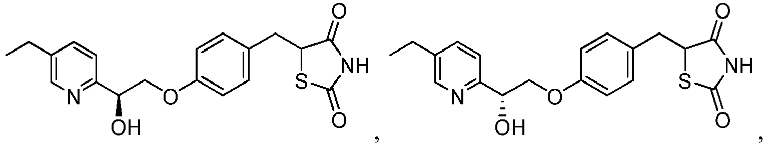 or 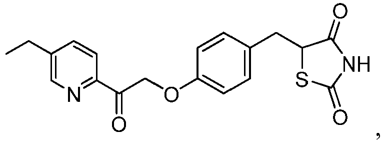 and a phosphodiesterase inhibitor.

In some instances, the phosphodiesterase inhibitor comprises a non-selective inhibitor. For example, the phosphodiesterase inhibitor comprises caffeine (1,3,7-trimethylxanthine), theobromine (3,7-dimethyl-2,3,6,7-tetrahydro-1H-purine-2,6-dione), theophylline (1,3-dimethyl-7H-purine-2,6-dione), IBMX (3-isobutyl-1-methylxanthine), or any combination thereof.

In some instances, the phosphodiesterase inhibitor comprises a selective inhibitor. For example, the selective phosphodiesterase inhibitor comprises Milrinone (2-methyl-6-oxo-1,6-dihydro-3,4'-bipyridine-5-carbonitrile), Cilostazol (6-[4-(1-cyclohexyl-1H-tetrazol-5-yl)butoxy]-3,4-dihydro-2(1H)-quinolinone), Cilomilast (4-cyano-4-(3-cyclopentyloxy-4-methoxyphenyl)cyclohexane-1-carboxylic acid), Rolipram (4-(3-cyclopentyloxy-4-methoxyphenyl)pyrrolidin-2-one), Roflumilast (3-(cyclopropylmethoxy)-N-(3,5-dichloropyridin-4-yl)-4-(difluoromethoxy)benzamide), or any combination thereof.

Further described herein is a method of treating and/or preventing a neurodegenerative disorder comprising administering to a patient a pharmaceutical composition comprising a co-crystal comprising a compound of Formula I, as described above, and a phosphodiesterase inhibitor.

In some instances, the phosphodiesterase inhibitor comprises a non-selective inhibitor. For example, the non-selective phosphodiesterase inhibitor includes caffeine (1,3,7-trimethylxanthine), theobromine (3,7-dimethyl-2,3,6,7-tetrahydro-1H-purine-2,6-dione), theophylline (1,3-dimethyl-7H-purine-2,6-dione), 3-isobutyl-1-methylxanthine, or any combination thereof.

In some instances, the phosphodiesterase inhibitor comprises a selective inhibitor. For example, the selective phosphodiesterase inhibitor comprises Milrinone (2-methyl-6-oxo-1,6-dihydro-3,4'-bipyridine-5-carbonitrile), Cilostazol (6-[4-(1-cyclohexyl-1H-tetrazol-5-yl)butoxy]-3,4-dihydro-2(1H)-quinolinone), Cilomilast (4-cyano-4-(3-cyclopentyloxy-4-methoxyphenyl)cyclohexane-1-carboxylic acid), Rolipram (4-(3-cyclopentyloxy-4-methoxyphenyl)pyrrolidin-2-one), Roflumilast (3-(cyclopropylmethoxy)-N-(3,5-dichloropyridin-4-yl)-4-(difluoromethoxy)benzamide), or any combinations thereof.

In some instances, the pharmaceutical composition further comprises a pharmaceutical agent having an activity that increases cAMP in the patient. For example, the pharmaceutical agent comprises a beta-adrenergic agonist. In some examples, the beta-adrenergic agonist comprises a beta-1-adrenergic agonist, a beta-2-adrenergic agonist, a beta-3-adrenergic agonist, or any combination thereof. In other examples, the beta-adrenergic agonist comprises noradrenaline, isoprenaline, dobutamine, salbutamol, levosalbutamol, terbutaline, pirbuterol, procaterol, metaproterenol, fenoterol, bitolterol mesylate, salmeterol, formoterol, bambuterol, clenbuterol, indacaterol, L-796568, amibegron, solabegron, isoproterenol, albuterol, metaproterenol, arbutamine, befunolol, bromoacetylalprenololmenthane, broxaterol, cimaterol, cirazoline, denopamine, dopexamine, epinephrine, etilefrine, hexoprenaline, higenamine, isoetharine, isoxsuprine, mabuterol, methoxyphenamine, nylidrin, oxyfedrine, prenalterol, ractopamine, reproterol, rimiterol, ritodrine, tretoquinol, tulobuterol, xamoterol, zilpaterol, zinterol, or any combination thereof.

Further described herein is a method of treating a neurodegenerative disorder in a patient comprising administering a pharmaceutical composition comprising
a co-crystal comprising the compound 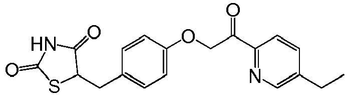 or a pharmaceutically acceptable salt thereof, and a phosphodiesterase inhibitor; and
a second pharmaceutical agent having an activity that increases cAMP in the patient, and a pharmaceutically acceptable carrier.

Also described herein is a method of treating a neurodegenerative disorder in a patient comprising administering a pharmaceutical composition comprising
a co-crystal comprising the compound 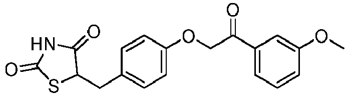 or a pharmaceutically acceptable salt thereof, and a phosphodiesterase inhibitor; and
a second pharmaceutical agent having an activity that increases cAMP in the patient, and a pharmaceutically acceptable carrier.

Further described herein is a method of treating or preventing a neurodegenerative disorder comprising administering to a patient an alkali earth metal salt of a compound of Formula I: 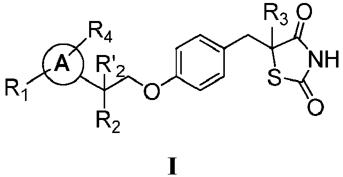 wherein each of R₁ and R₄ is independently selected from H, halo, aliphatic, and alkoxy, wherein the aliphatic or alkoxy is optionally substituted with 1-3 of halo; R'₂ is H; R₂ is H, halo, hydroxy, or optionally substituted aliphatic, -O-acyl, -O-aroyl, -O-heteroaroyl, -O(SO₂)NH₂, -O-CH(Rₘ)OC(O)Rₙ, -O-CH(Rₘ)OP(O)(ORₙ)₂, -O-P(O)(ORₙ)₂, or 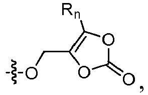 wherein each Rₘ is independently an optionally substituted C₁₋₆ alkyl, each Rₙ is independently C₁₋₁₂ alkyl, C₃₋₈ cycloalkyl, or phenyl, each of which is optionally substituted, or R₂ and R'₂ together form oxo; R₃ is H or optionally substituted C₁₋₃ alkyl; and ring A is a phenyl, pyridin-2-yl, pyridin-3-yl, or pyridin-4-yl, each of which is substituted with an R₁ group and an R₄ group at any chemically feasible position on ring A.

In some instances, the neurodegenerative disorder comprises Alzheimer's Disease, Parkinson's Disease, ALS, MS, MCI, or any combination thereof.

In some instances, the alkali earth metal is sodium or potassium.

In some instances, R₃ is H.

In some instances, R₃ is CH₃.

In some instances, R₄ is H, methyl, methoxy, ethyl, ethoxy, -O-isopropyl, -CF₃, -OCHF₂ or -OCF₃. For example, R₄ is H.

In some instances, R₁ is H, alkyl, halo or alkoxy. For example, R₁ is H. In other examples, R₁ is halo. In some examples, R₁ is C₁₋₃ alkyl.

In some instances, ring A is phenyl that is substituted with R₁ and R₄ groups at any chemically feasible position on ring A. In some examples, ring A is phenyl, and one of R₁ or R₄ is attached to the para or meta position of ring A. In other examples, ring A is phenyl, and one of R₁ or R₄ is attached to the meta position of ring A. In some examples, R₁ is attached to the para or meta position of ring A. And, in some examples, R₁ is F or Cl, either of which is attached to the para or meta position of ring A. In other examples, R₁ is alkoxy (e.g., methoxy, ethoxy, propoxy, -O-isopropyl, butoxy, or -O-tertbutyl) that is attached to the para or meta position of ring A. In other examples, ring A is phenyl, and R₁ is attached to the meta or ortho position of the phenyl ring. For instance, ring A is phenyl, and R₁ is attached to the ortho position of the phenyl ring. In some instances, ring A is phenyl, and R₁ is methoxy, ethoxy, or -O-isopropyl, any of which is attached to the ortho position of ring A. In other instances, R₁ is -CF₃, -OCHF₂ or -OCF₃.

In some instances, ring A is optionally substituted pyridin-2-yl or optionally substituted pyridin-3-yl, either of which is substituted with R₁ and R₄ groups at any chemically feasible position on ring A. In some examples, ring A is pyridin-2-yl, and one of R₁ or R₄ is attached to the 5 position of the ring. In other examples, ring A is pyridin-3-yl, and one of R₁ or R₄ is attached to the 6 position of the ring. In some examples, ring A is pyridin-2-yl, and R₁ is attached to the 5 position of the ring. For instance, ring A is pyridin-2-yl, and R₁ is alkyl or alkoxy, either of which is attached to the 5 position of ring A. In other instances, ring A is pyridin-2-yl, and R₁ is methyl, ethyl, propyl, isopropyl, butyl, or tertbutyl, any of which are attached to the 5 position of ring A.

In some instances, R'₂ is H.

In some instances, R₂ is hydroxy.

In some instances, R₂ is -O-acyl, -O-aroyl, or -O-heteroaroyl.

In some instances, R₂ and R'₂ together form oxo.

In some instances, the compound of Formula I is one selected from:

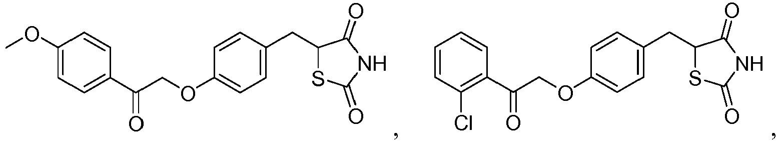

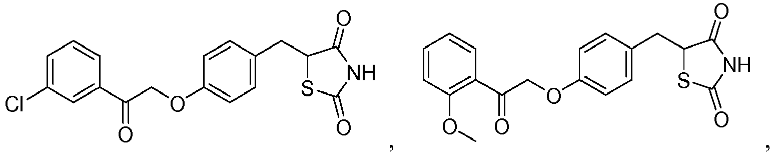

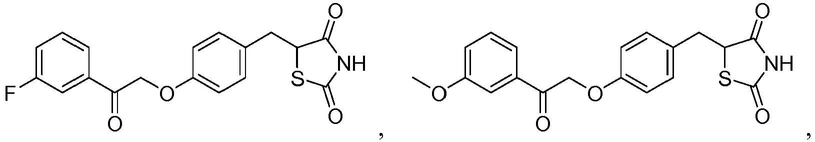

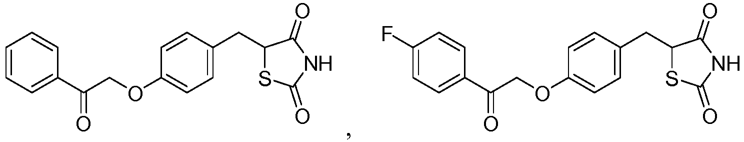

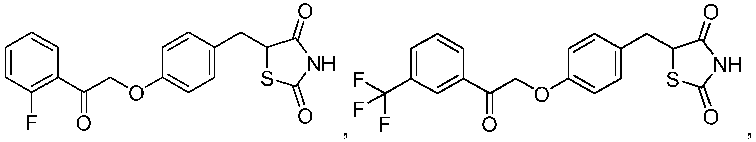

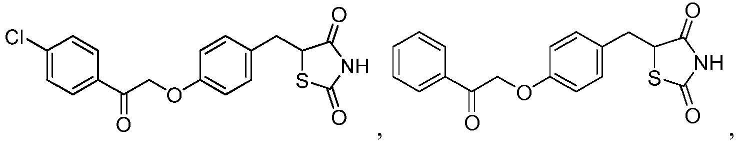

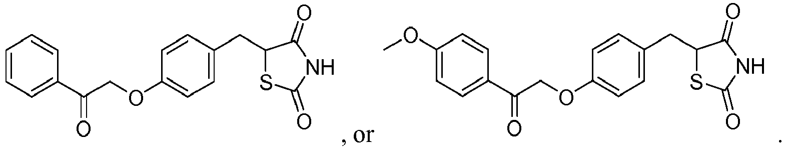

In some instances, the compound of Formula I is one selected from: 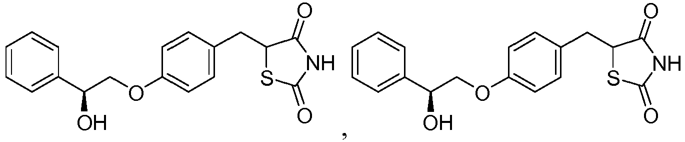 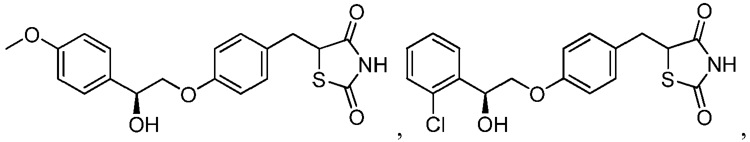 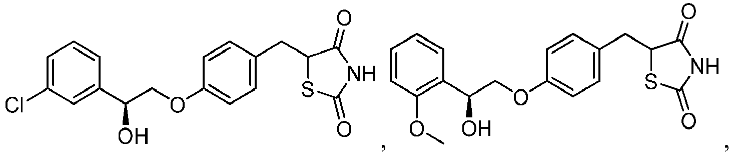 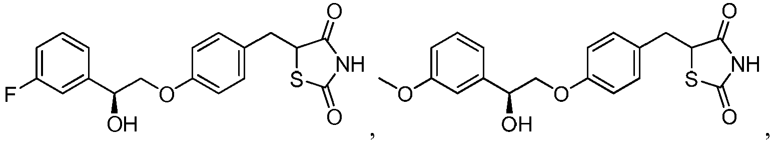 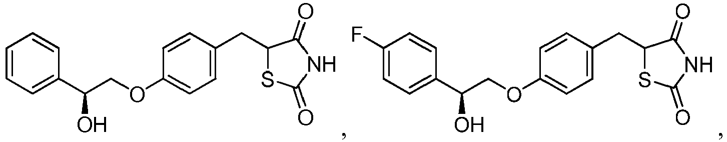 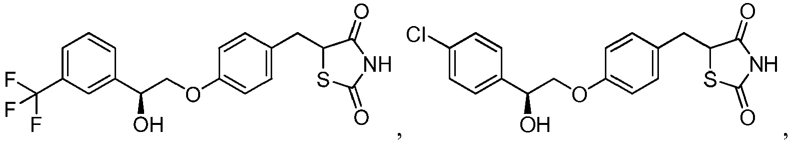 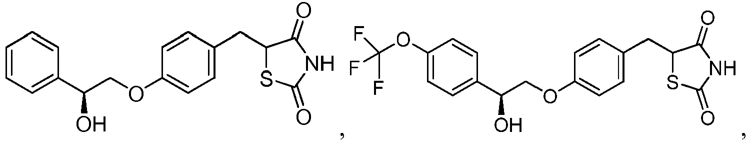 or

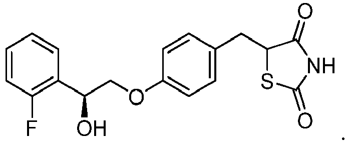

In some instances, the compound of Formula I is one selected from:

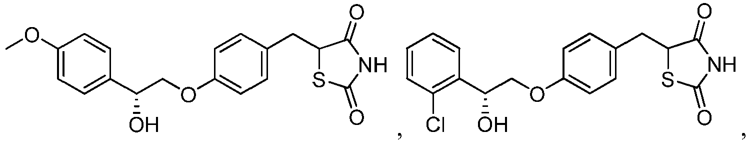

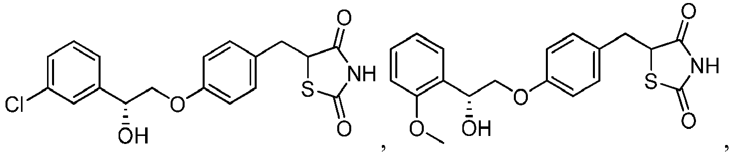

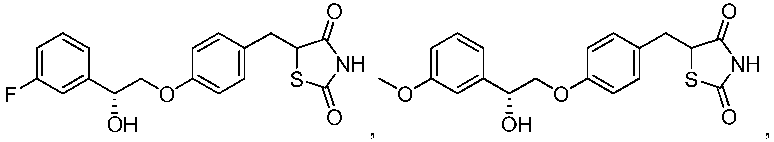

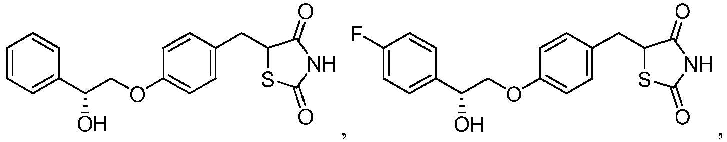

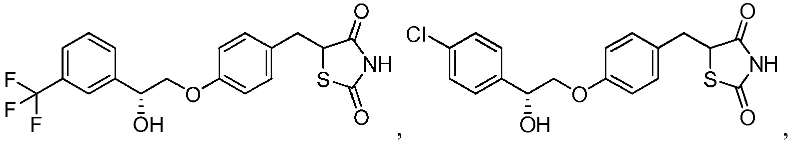

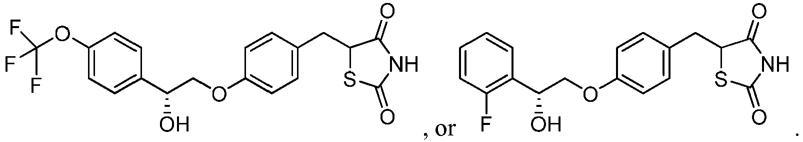

In some instances, the compound of Formula I is one selected from:

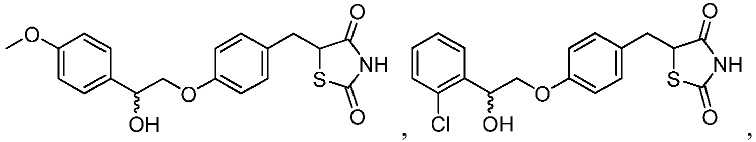

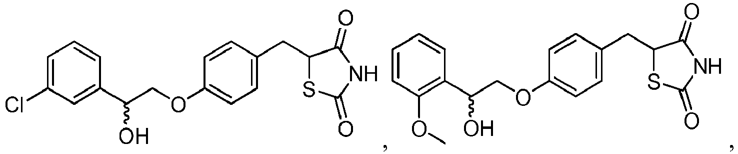

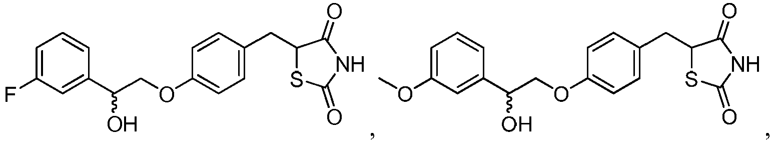

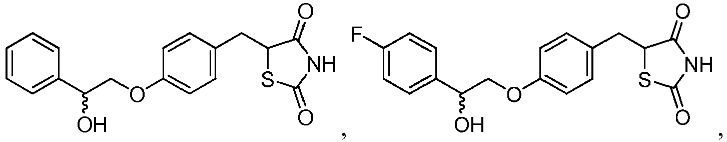

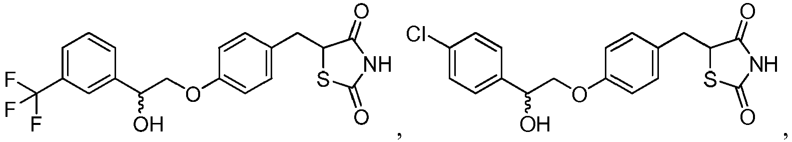

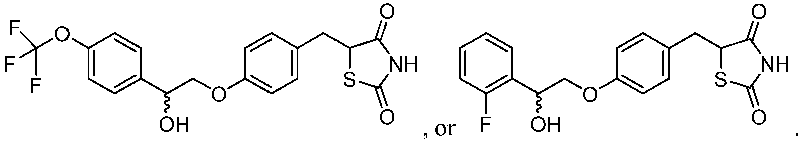

In some instances, the compound of Formula I is one selected from: 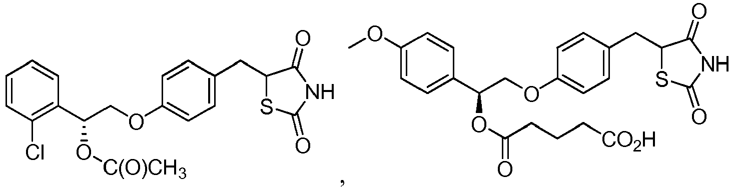 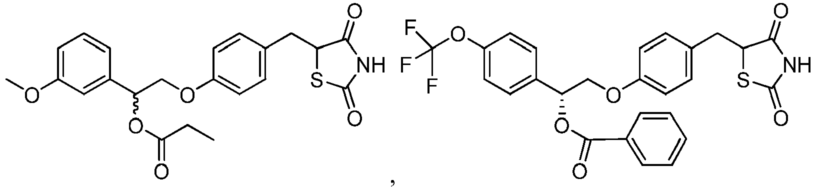 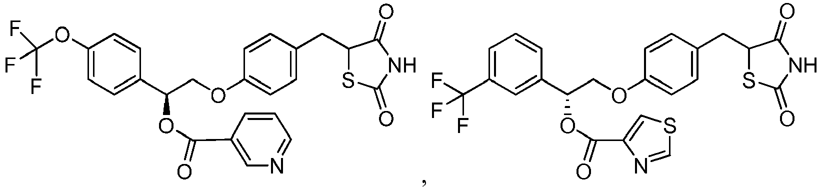 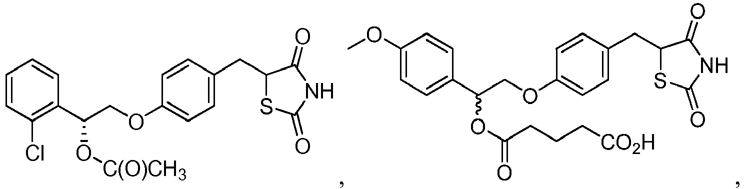 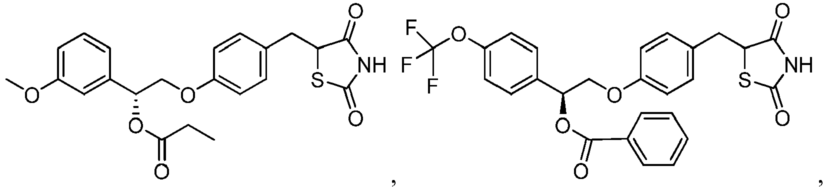 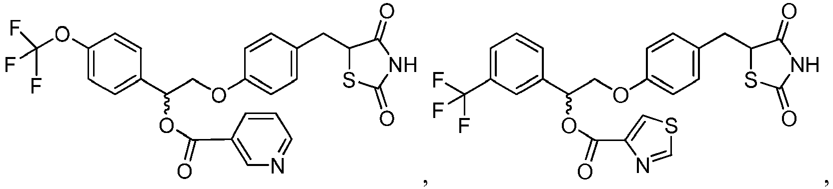 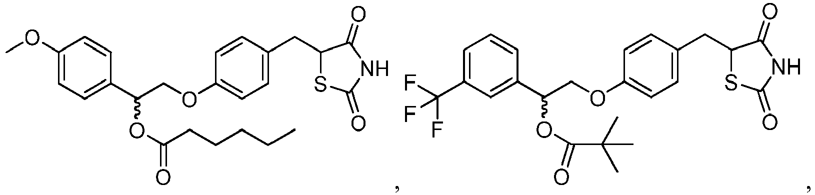 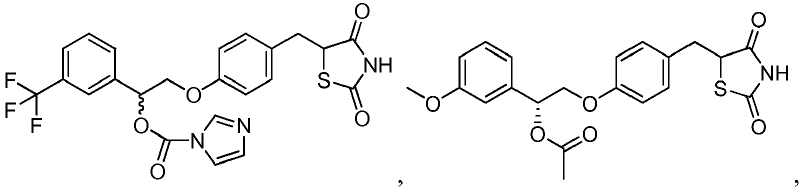 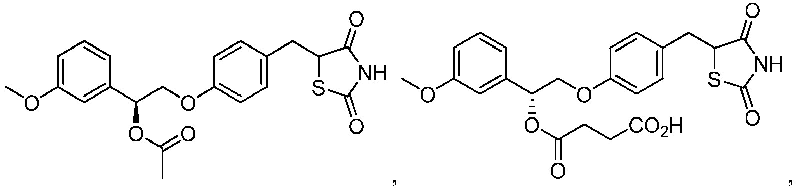 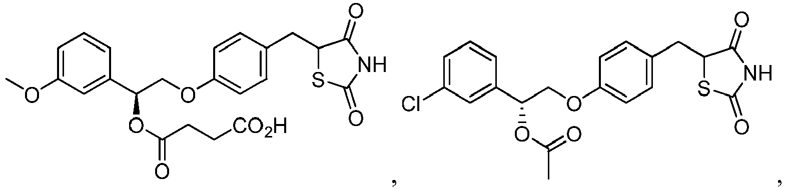 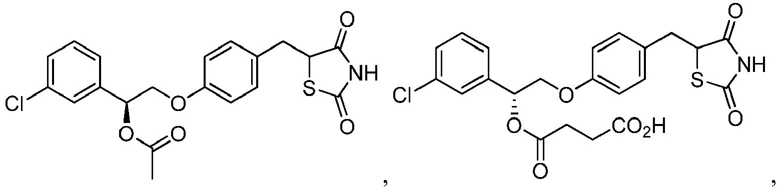 or

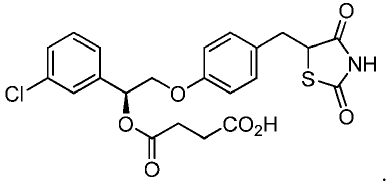

In some instances, the compound of Formula I is one selected from:

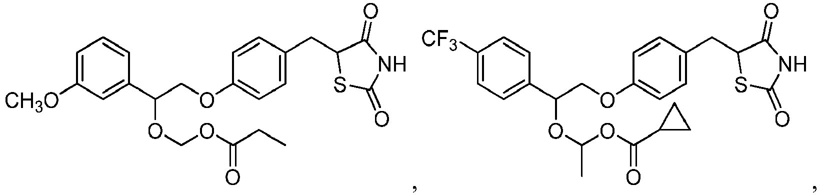

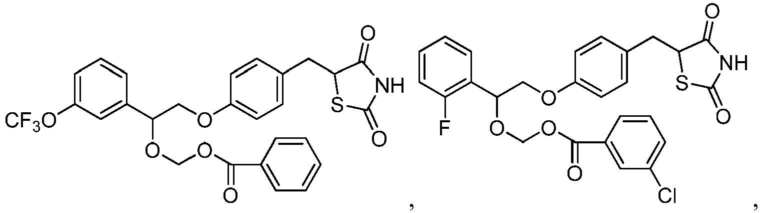

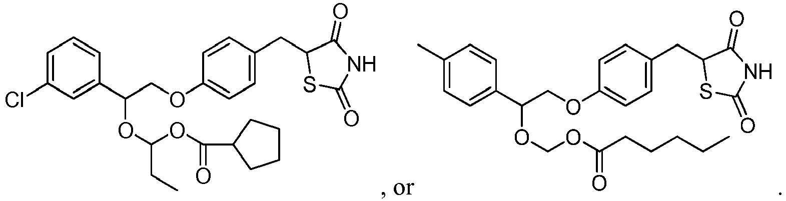

In other instances the compound of Formula I is one selected from:

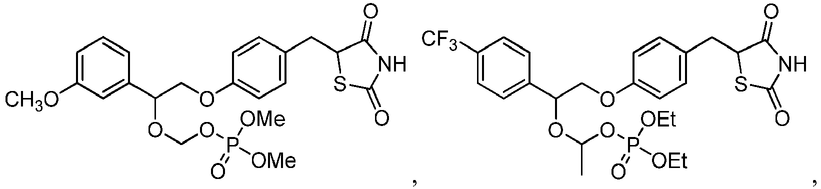

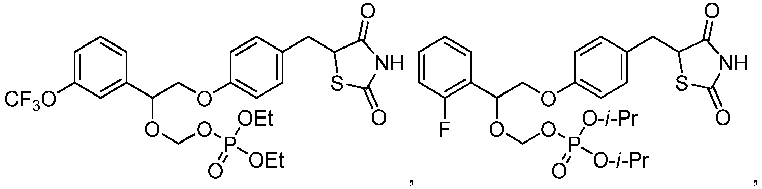

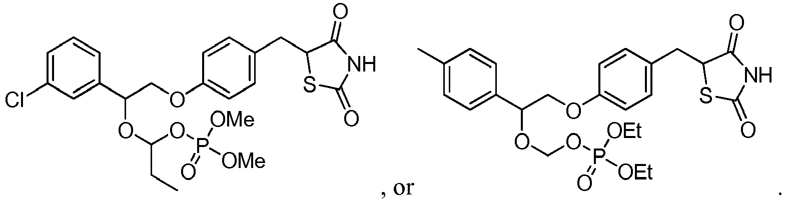

In some instances, the compound of Formula I is one selected from: 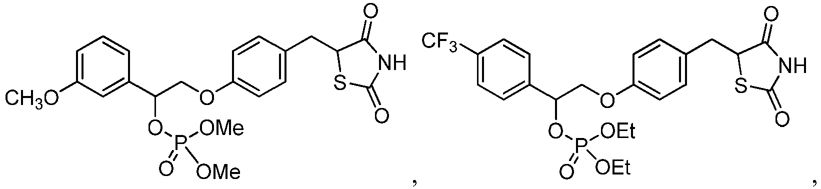 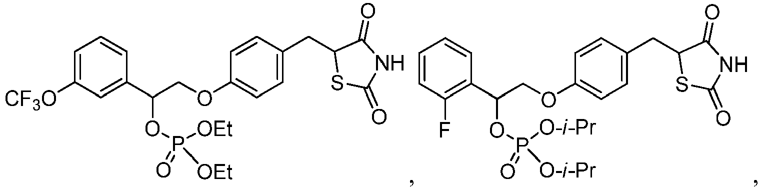 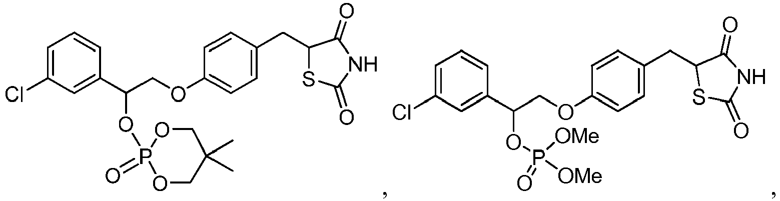 or

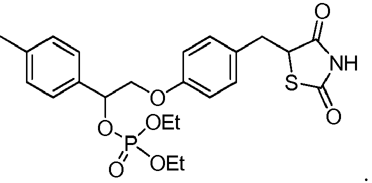

In some instances, the compound of Formula I is one selected from:

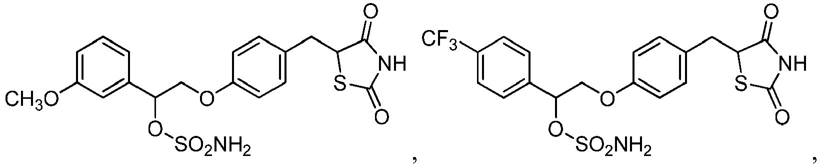

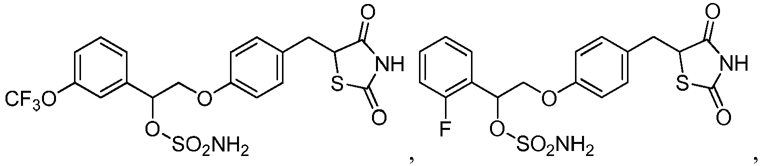

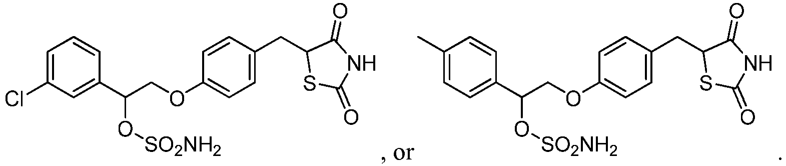

In some instances, the compound of Formula I is one selected from:

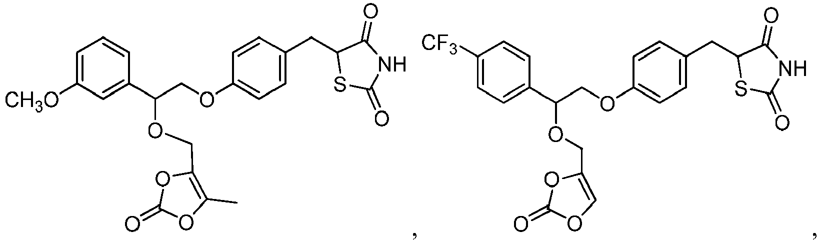

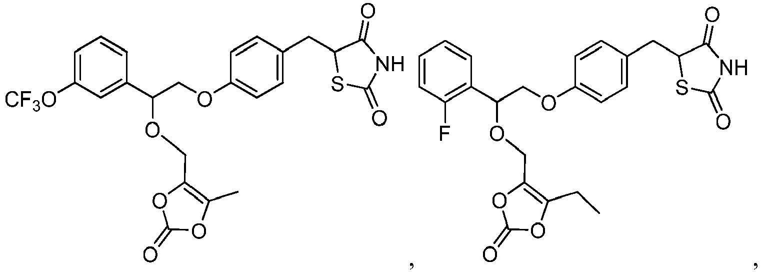

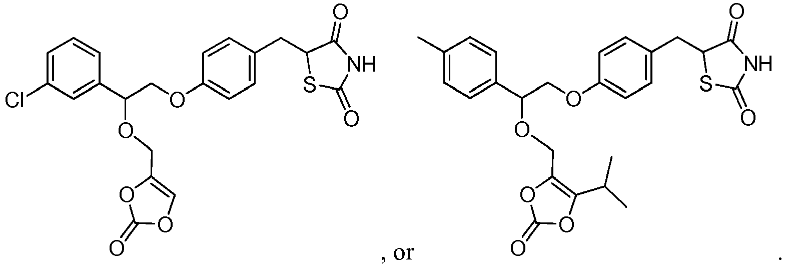

In some instances, the compound of Formula I is one selected from: 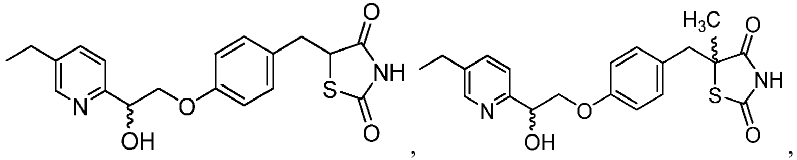 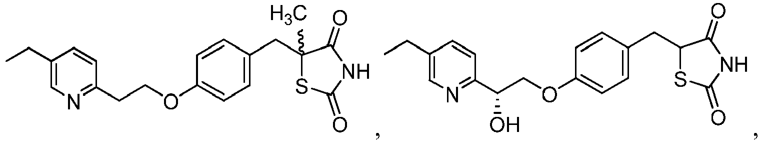 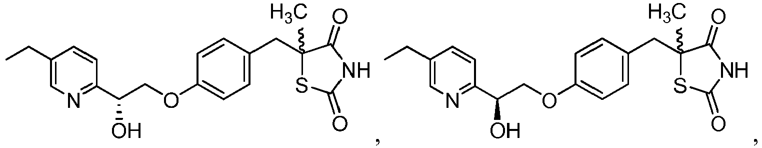 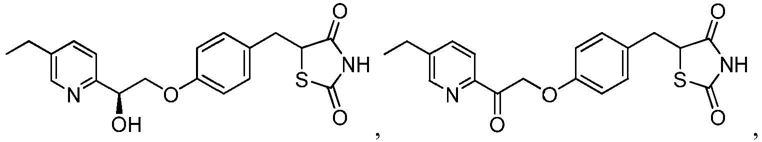 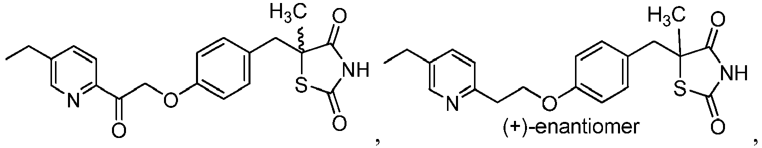 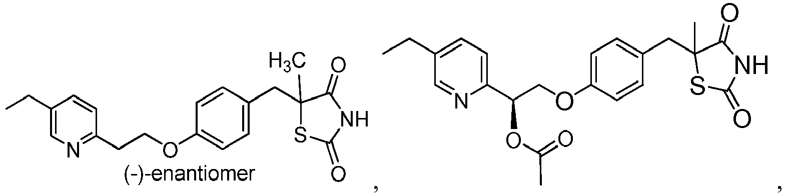 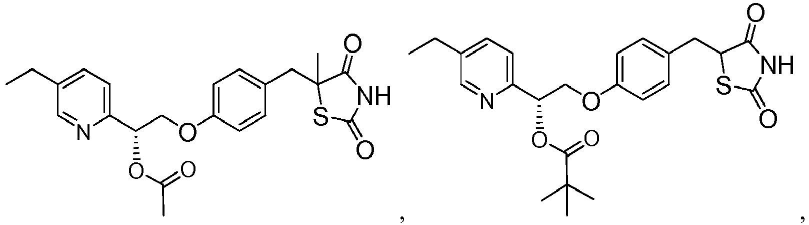 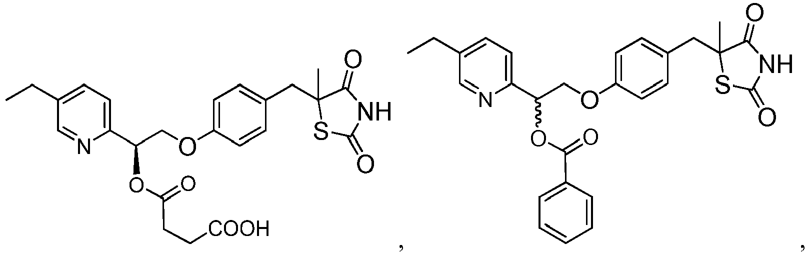 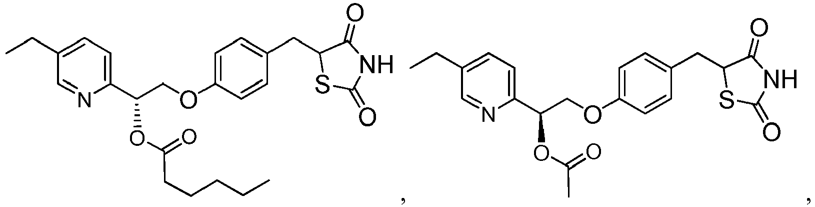 or

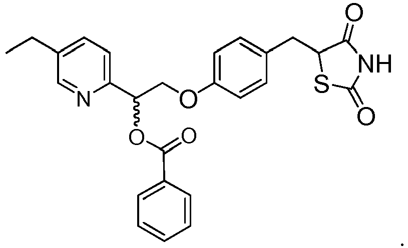

In some instances, the compound of Formula I is one selected from:

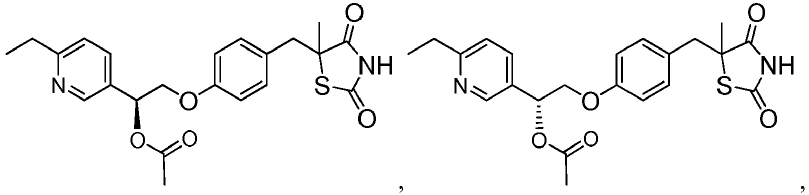

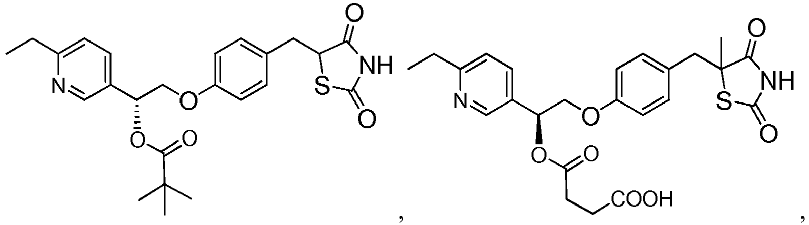

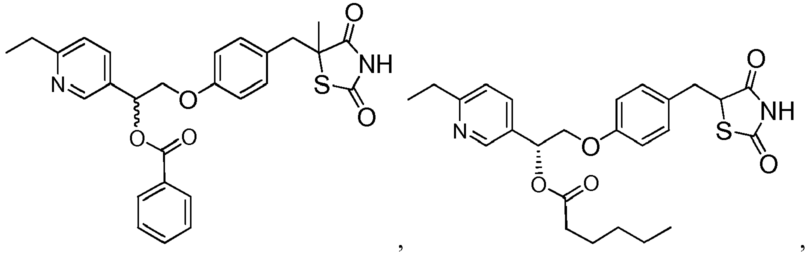

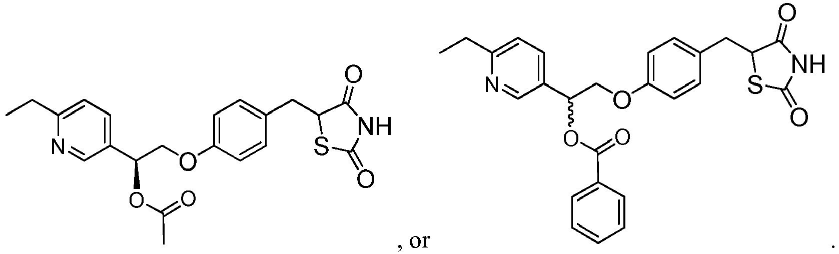

In some instances, the compound of Formula I is one selected from: 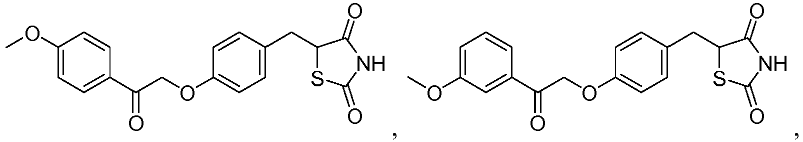 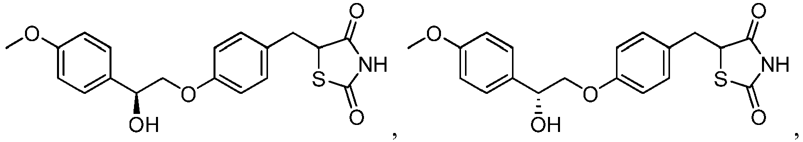 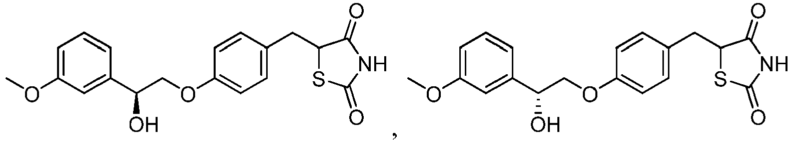 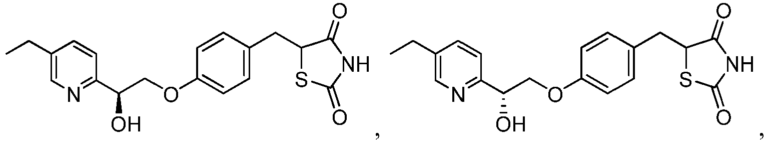 or

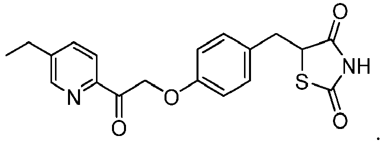

Some instances further comprise administering a phosphodiesterase inhibitor.

Some instances further comprise administering to the patient another pharmaceutical agent having an activity that increases cAMP in the patient.

In some instances, wherein the second pharmaceutical agent further comprises a beta-adrenergic agonist. For example, the beta-adrenergic agonist comprises a beta-1-adrenergic agonist, a beta-2-adrenergic agonist, a beta-3-adrenergic agonist, or any combination thereof. In other examples, the beta-adrenergic agonist comprises noradrenaline, isoprenaline, dobutamine, salbutamol, levosalbutamol, terbutaline, pirbuterol, procaterol, metaproterenol, fenoterol, bitolterol mesylate, salmeterol, formoterol, bambuterol, clenbuterol, indacaterol, L-796568, amibegron, solabegron, isoproterenol, albuterol, metaproterenol, arbutamine, befunolol, bromoacetylalprenololmenthane, broxaterol, cimaterol, cirazoline, denopamine, dopexamine, epinephrine, etilefrine, hexoprenaline, higenamine, isoetharine, isoxsuprine, mabuterol, methoxyphenamine, nylidrin, oxyfedrine, prenalterol, ractopamine, reproterol, rimiterol, ritodrine, tretoquinol, tulobuterol, xamoterol, zilpaterol, zinterol, or any combination thereof.

Also described herein is a method for treating Alzheimer's Disease comprising administering to a patient a compound of Formula I: 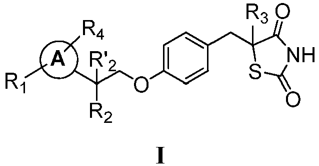 or a pharmaceutically acceptable salt thereof, wherein each of R₁ and R₄ is independently selected from H, halo, aliphatic, and alkoxy, wherein the aliphatic or alkoxy is optionally substituted with 1-3 of halo; R'₂ is H; R₂ is H, halo, hydroxy, or optionally substituted aliphatic, -O-acyl, -O-aroyl, -O-heteroaroyl, -O(SO₂)NH₂, -O-CH(Rₘ)OC(O)Rₙ, -O-CH(Rₘ)OP(O)(ORₙ)₂, -O-P(O)(ORₙ)₂, or 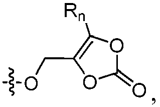 wherein each Rₘ is independently an optionally substituted C₁₋₆ alkyl, each Rₙ is independently C₁₋₁₂ alkyl, C₃₋₈ cycloalkyl, or phenyl, each of which is optionally substituted, or R₂ and R'₂ together form oxo; R₃ is H or optionally substituted C₁₋₃ alkyl; and Ring A is a phenyl, pyridin-2-yl, pyridin-3-yl, or pyridin-4-yl, each of which is substituted with an R₁ group and an R₄ group at any chemically feasible position on ring A.

In some instances, the compound of Formula I is selected from: 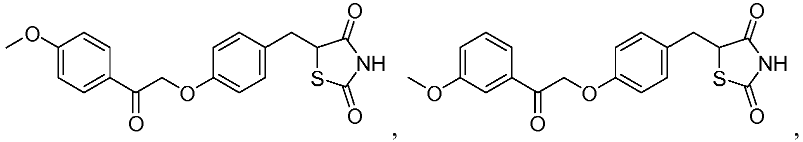 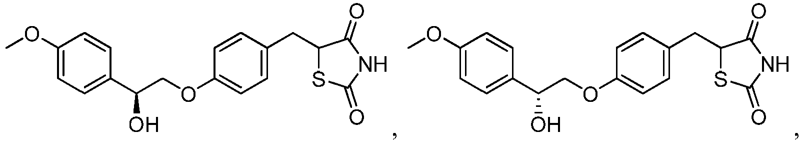 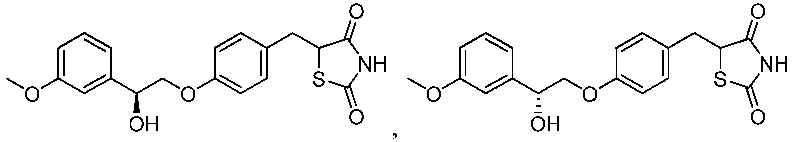 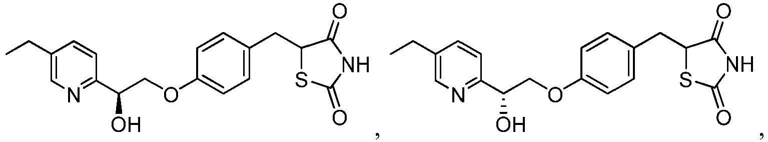 or

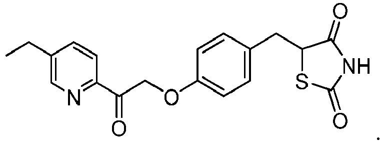

Some instances further comprise administering LDOPA to the patient.

Further described herein is a method of treating Alzheimer's Disease comprising adminstering to a patient an alkali earth metal salt of a compound of Formula I: 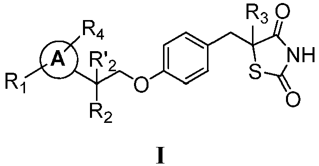 or a pharmaceutically acceptable salt thereof, wherein each of R₁ and R₄ is independently selected from H, halo, aliphatic, and alkoxy, wherein the aliphatic or alkoxy is optionally substituted with 1-3 of halo; R'₂ is H; R₂ is H, halo, hydroxy, or optionally substituted aliphatic, -O-acyl, -O-aroyl, -O-heteroaroyl, -O(SO₂)NH₂, -O-CH(Rₘ)OC(O)Rₙ, -O-CH(Rₘ)OP(O)(ORₙ)₂, -O-P(O)(ORₙ)₂, or 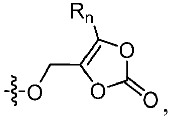 wherein each Rₘ is independently an optionally substituted C₁₋₆ alkyl, each Rₙ is independently C₁₋₁₂ alkyl, C₃₋₈ cycloalkyl, or phenyl, each of which is optionally substituted, or R₂ and R'₂ together form oxo; R₃ is H or optionally substituted C₁₋₃ alkyl; and ring A is a phenyl, pyridin-2-yl, pyridin-3-yl, or pyridin-4-yl, each of which is substituted with an R₁ group and an R₄ group at any chemically feasible position on ring A.

In some instances, the alkali earth metal is potassium or sodium.

In other instances, the compound of Formula I is selected from: 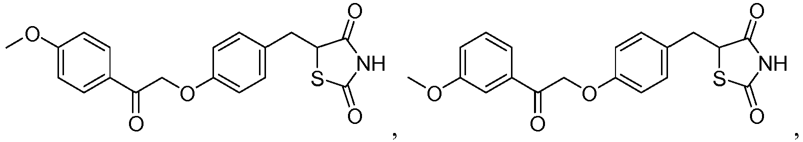 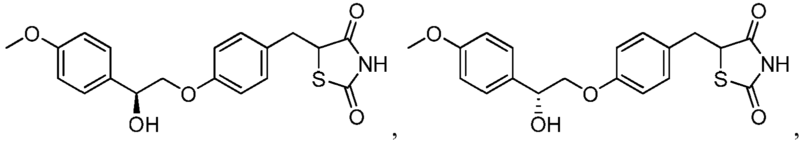 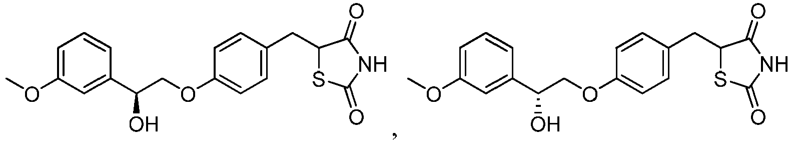 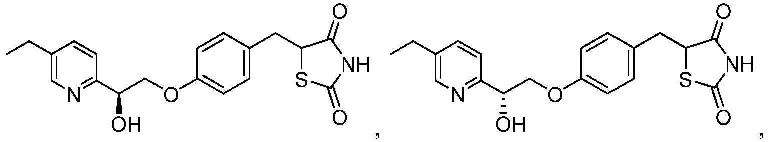 or

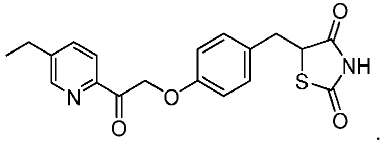

Some instances further comprise administering LDOPA to the patient.

### BRIEF DESCRIPTION OF THE DRAWINGS

The disclosure will now be described, by way of example, with reference to the accompanying drawings, in which:

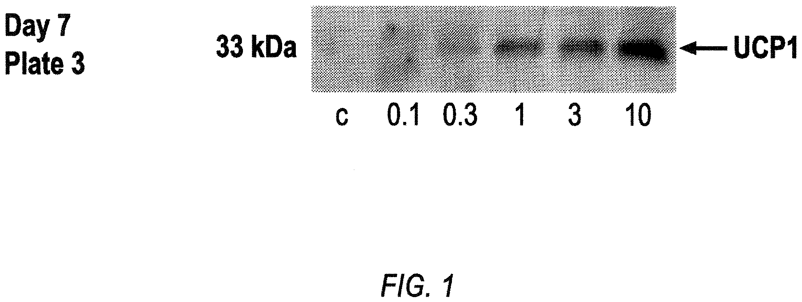

Figure 1 is a picture of a Western blot that assayed UCP1 protein in brown adipose tissue precursor cells treated with an exemplary compound of Formula I;

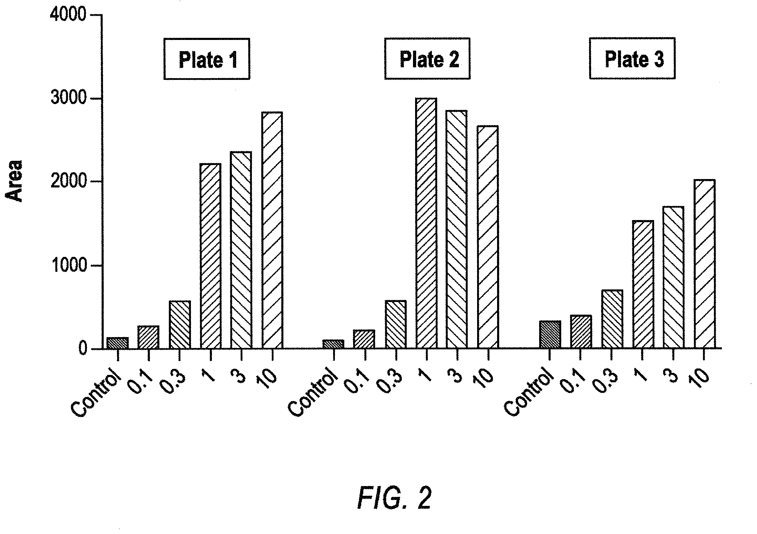

Figure 2 is a graphical representation of UCP1 protein in brown adipose tissue precursor cells treated with from 0 to 10 µM concentration of an exemplary compound of Formula I, as assayed by Western blot in triplicate;
Figure 3 is a graphical representation of the fold induction of PGC-1α in brown adipose tissue precursor cells after treatment with 3 µM of a compound of Formula I for two days followed by treatment with 1 µM norepinephrine for 2 hours;
Figure 4 is a ¹H NMR spectrum for 5-(4-(2-(5-ethylpyridin-2-yl)-2-oxoethoxy)benzyl)-1,3-thiazolidine-2,4-dione;
Figure 5 is a ¹H NMR spectrum for caffeine;
Figure 6 is a ¹H NMR spectrum for an exemplary co-crystal of 5-(4-(2-(5-ethylpyridin-2-yl)-2-oxoethoxy)benzyl)-1,3-thiazolidine-2,4-dione and caffeine;
Figure 7 provides graphical representations of plaque size and number in a mouse model that was administered an exemplary compound of the present invention; and
Figure 8 provides a graphical representation of GFAP astrocyte marker assay results in a mouse model that was administered an exemplary compound of the present invention.

### DETAILED DESCRIPTION OF THE INVENTION

The claimed sodium salt of Compound X 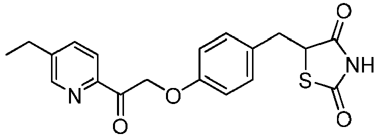 is useful in treating Alzheimer's Disease. This compound is referred to herein as compound of this invention and falls under the general Formula I.

Further described herein are methods of treating and/or preventing neurodegenerative disorders in a patient, and pharmaceutical compositions useful for treating and/or preventing neurodegenerative disorders in a patient.

### I. DEFINITIONS

As used herein, the following definitions shall apply unless otherwise indicated.

For purposes of this invention, the chemical elements are identified in accordance with the Periodic Table of the Elements, CAS version, Handbook of Chemistry and Physics, 75th Ed. Additionally, general principles of organic chemistry are described in "Organic Chemistry", Thomas Sorrell, University Science Books, Sausalito: 1999, and "March's Advanced Organic Chemistry", 5th Ed., Ed.: Smith, M.B. and March, J., John Wiley & Sons, New York: 2001.

As described herein, compounds described herein may optionally be substituted with one or more substituents, such as are illustrated generally above, or as exemplified by particular classes, subclasses, and species of the invention.

As used herein the term "aliphatic" encompasses the terms alkyl, alkenyl, alkynyl, each of which being optionally substituted as set forth below.

As used herein, an "alkyl" group refers to a saturated aliphatic hydrocarbon group containing 1-12 (e.g., 1-8, 1-6, or 1-4) carbon atoms. An alkyl group can be straight or branched. Examples of alkyl groups include, but are not limited to, methyl, ethyl, propyl, isopropyl, butyl, isobutyl, sec-butyl, tert-butyl, n-pentyl, n-heptyl, or 2-ethylhexyl. An alkyl group can be substituted (i.e., optionally substituted) with one or more substituents such as halo, phospho, cycloaliphatic [e.g., cycloalkyl or cycloalkenyl], heterocycloaliphatic [e.g., heterocycloalkyl or heterocycloalkenyl], aryl, heteroaryl, alkoxy, aroyl, heteroaroyl, acyl [e.g., (aliphatic)carbonyl, (cycloaliphatic)carbonyl, or (heterocycloaliphatic)carbonyl], nitro, cyano, amido [e.g., (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino alkylaminocarbonyl, cycloalkylaminocarbonyl, heterocycloalkylaminocarbonyl, arylaminocarbonyl, or heteroarylaminocarbonyl], amino [e.g., aliphaticamino, cycloaliphaticamino, or heterocycloaliphaticamino], sulfonyl [e.g., aliphatic-SO₂-], sulfinyl, sulfanyl, sulfoxy, urea, thiourea, sulfamoyl, sulfamide, oxo, carboxy, carbamoyl, cycloaliphaticoxy, heterocycloaliphaticoxy, aryloxy, heteroaryloxy, aralkyloxy, heteroarylalkoxy, alkoxycarbonyl, alkylcarbonyloxy, or hydroxy. Without limitation, some examples of substituted alkyls include carboxyalkyl (such as HOOC-alkyl, alkoxycarbonylalkyl, and alkylcarbonyloxyalkyl), cyanoalkyl, hydroxyalkyl, alkoxyalkyl, acylalkyl, aralkyl, (alkoxyaryl)alkyl, (sulfonylamino)alkyl (such as (alkyl-SO₂-amino)alkyl), aminoalkyl, amidoalkyl, (cycloaliphatic)alkyl, or haloalkyl.

As used herein, an "alkenyl" group refers to an aliphatic carbon group that contains 2-8 (e.g., 2-12, 2-6, or 2-4) carbon atoms and at least one double bond. Like an alkyl group, an alkenyl group can be straight or branched. Examples of an alkenyl group include, but are not limited to allyl, isoprenyl, 2-butenyl, and 2-hexenyl. An alkenyl group can be optionally substituted with one or more substituents such as halo, phospho, cycloaliphatic [e.g., cycloalkyl or cycloalkenyl], heterocycloaliphatic [e.g., heterocycloalkyl or heterocycloalkenyl], aryl, heteroaryl, alkoxy, aroyl, heteroaroyl, acyl [e.g., (aliphatic)carbonyl, (cycloaliphatic)carbonyl, or (heterocycloaliphatic)carbonyl], nitro, cyano, amido [e.g., (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino alkylaminocarbonyl, cycloalkylaminocarbonyl, heterocycloalkylaminocarbonyl, arylaminocarbonyl, or heteroarylaminocarbonyl], amino [e.g., aliphaticamino, cycloaliphaticamino, heterocycloaliphaticamino, or aliphaticsulfonylamino], sulfonyl [e.g., alkyl-SO₂-, cycloaliphatic-SO₂-, or aryl-SO₂-], sulfinyl, sulfanyl, sulfoxy, urea, thiourea, sulfamoyl, sulfamide, oxo, carboxy, carbamoyl, cycloaliphaticoxy, heterocycloaliphaticoxy, aryloxy, heteroaryloxy, aralkyloxy, heteroaralkoxy, alkoxycarbonyl, alkylcarbonyloxy, or hydroxy. Without limitation, some examples of substituted alkenyls include cyanoalkenyl, alkoxyalkenyl, acylalkenyl, hydroxyalkenyl, aralkenyl, (alkoxyaryl)alkenyl, (sulfonylamino)alkenyl (such as (alkyl-SO₂-amino)alkenyl), aminoalkenyl, amidoalkenyl, (cycloaliphatic)alkenyl, or haloalkenyl.

As used herein, an "alkynyl" group refers to an aliphatic carbon group that contains 2-8 (e.g., 2-12, 2-6, or 2-4) carbon atoms and has at least one triple bond. An alkynyl group can be straight or branched. Examples of an alkynyl group include, but are not limited to, propargyl and butynyl. An alkynyl group can be optionally substituted with one or more substituents such as aroyl, heteroaroyl, alkoxy, cycloalkyloxy, heterocycloalkyloxy, aryloxy, heteroaryloxy, aralkyloxy, nitro, carboxy, cyano, halo, hydroxy, sulfo, mercapto, sulfanyl [e.g., aliphaticsulfanyl or cycloaliphaticsulfanyl], sulfinyl [e.g., aliphaticsulfinyl or cycloaliphaticsulfinyl], sulfonyl [e.g., aliphatic-SO₂-, aliphaticamino-SO₂-, or cycloaliphatic-SO₂-], amido [e.g., aminocarbonyl, alkylaminocarbonyl, alkylcarbonylamino, cycloalkylaminocarbonyl, heterocycloalkylaminocarbonyl, cycloalkylcarbonylamino, arylaminocarbonyl, arylcarbonylamino, aralkylcarbonylamino, (heterocycloalkyl)carbonylamino, (cycloalkylalkyl)carbonylamino, heteroaralkylcarbonylamino, heteroarylcarbonylamino or heteroarylaminocarbonyl], urea, thiourea, sulfamoyl, sulfamide, alkoxycarbonyl, alkylcarbonyloxy, cycloaliphatic, heterocycloaliphatic, aryl, heteroaryl, acyl [e.g., (cycloaliphatic)carbonyl or (heterocycloaliphatic)carbonyl], amino [e.g., aliphaticamino], sulfoxy, oxo, carboxy, carbamoyl, (cycloaliphatic)oxy, (heterocycloaliphatic)oxy, or (heteroaryl)alkoxy.

As used herein, an "amido" encompasses both "aminocarbonyl" and "carbonylamino". These terms when used alone or in connection with another group refer to an amido group such as -N(R^{X})-C(O)-R^{Y} or -C(O)-N(R^{X})₂, when used terminally, and -C(O)-N(R^{X})- or -N(R^{X})-C(O)- when used internally, wherein R^{X} and R^{Y} can be aliphatic, cycloaliphatic, aryl, araliphatic, heterocycloaliphatic, heteroaryl or heteroaraliphatic.. Examples of amido groups include alkylamido (such as alkylcarbonylamino or alkylaminocarbonyl), (heterocycloaliphatic)amido, (heteroaralkyl)amido, (heteroaryl)amido, (heterocycloalkyl)alkylamido, arylamido, aralkylamido, (cycloalkyl)alkylamido, or cycloalkylamido.

As used herein, an "amino" group refers to -NR^{X}R^{Y} wherein each of R^{X} and R^{Y} is independently hydrogen, aliphatic, cycloaliphatic, (cycloaliphatic)aliphatic, aryl, araliphatic, heterocycloaliphatic, (heterocycloaliphatic)aliphatic, heteroaryl, carboxy, sulfanyl, sulfinyl, sulfonyl, (aliphatic)carbonyl, (cycloaliphatic)carbonyl, ((cycloaliphatic)aliphatic)carbonyl, arylcarbonyl, (araliphatic)carbonyl, (heterocycloaliphatic)carbonyl, ((heterocycloaliphatic)aliphatic)carbonyl, (heteroaryl)carbonyl, or (heteroaraliphatic)carbonyl, each of which being defined herein and being optionally substituted. Examples of amino groups include alkylamino, dialkylamino, or arylamino. When the term "amino" is not the terminal group (e.g., alkylcarbonylamino), it is represented by -NR^{X}-. R^{X} has the same meaning as defined above.

As used herein, an "aryl" group used alone or as part of a larger moiety as in "aralkyl", "aralkoxy", or "aryloxyalkyl" refers to monocyclic (e.g., phenyl); bicyclic (e.g., indenyl, naphthalenyl, tetrahydronaphthyl, tetrahydroindenyl); and tricyclic (e.g., fluorenyl tetrahydrofluorenyl, or tetrahydroanthracenyl, anthracenyl) ring systems in which the monocyclic ring system is aromatic or at least one of the rings in a bicyclic or tricyclic ring system is aromatic. The bicyclic and tricyclic groups include benzo fused 2-3 membered carbocyclic rings. For example, a benzofused group includes phenyl fused with two or more C₄₋₈ carbocyclic moieties. An aryl is optionally substituted with one or more substituents including aliphatic [e.g., alkyl, alkenyl, or alkynyl]; cycloaliphatic; (cycloaliphatic)aliphatic; heterocycloaliphatic; (heterocycloaliphatic)aliphatic; aryl; heteroaryl; alkoxy; (cycloaliphatic)oxy; (heterocycloaliphatic)oxy; aryloxy; heteroaryloxy; (araliphatic)oxy; (heteroaraliphatic)oxy; aroyl; heteroaroyl; amino; oxo (on a non-aromatic carbocyclic ring of a benzofused bicyclic or tricyclic aryl); nitro; carboxy; amido; acyl [e.g., (aliphatic)carbonyl; (cycloaliphatic)carbonyl; ((cycloaliphatic)aliphatic)carbonyl; (araliphatic)carbonyl; (heterocycloaliphatic)carbonyl; ((heterocycloaliphatic)aliphatic)carbonyl; or (heteroaraliphatic)carbonyl]; sulfonyl [e.g., aliphatic-SO₂- or amino-SO₂-]; sulfinyl [e.g., aliphatic-S(O)- or cycloaliphatic-S(O)-]; sulfanyl [e.g., aliphatic-S-]; cyano; halo; hydroxy; mercapto; sulfoxy; urea; thiourea; sulfamoyl; sulfamide; or carbamoyl. Alternatively, an aryl can be unsubstituted.

Non-limiting examples of substituted aryls include haloaryl [e.g., mono-, di (such as *p,m*-dihaloaryl), and (trihalo)aryl]; (carboxy)aryl [e.g., (alkoxycarbonyl)aryl, ((aralkyl)carbonyloxy)aryl, and (alkoxycarbonyl)aryl]; (amido)aryl [e.g., (aminocarbonyl)aryl, (((alkylamino)alkyl)aminocarbonyl)aryl, (alkylcarbonyl)aminoaryl, (arylaminocarbonyl)aryl, and (((heteroaryl)amino)carbonyl)aryl]; aminoaryl [e.g., ((alkylsulfonyl)amino)aryl or ((dialkyl)amino)aryl]; (cyanoalkyl)aryl; (alkoxy)aryl; (sulfamoyl)aryl [e.g., (aminosulfonyl)aryl]; (alkylsulfonyl)aryl; (cyano)aryl; (hydroxyalkyl)aryl; ((alkoxy)alkyl)aryl; (hydroxy)aryl, ((carboxy)alkyl)aryl; (((dialkyl)amino)alkyl)aryl; (nitroalkyl)aryl; (((alkylsulfonyl)amino)alkyl)aryl; ((heterocycloaliphatic)carbonyl)aryl; ((alkylsulfonyl)alkyl)aryl; (cyanoalkyl)aryl; (hydroxyalkyl)aryl; (alkylcarbonyl)aryl; alkylaryl; (trihaloalkyl)aryl; *p*-amino-*m-*alkoxycarbonylaryl; *p*-amino-*m*-cyanoaryl; *p*-halo-*m*-aminoaryl; or (*m*-(heterocycloaliphatic)-*o*-(alkyl))aryl.

As used herein, an "araliphatic" such as an "aralkyl" group refers to an aliphatic group (e.g., a C₁₋₄ alkyl group) that is substituted with an aryl group. "Aliphatic," "alkyl," and "aryl" are defined herein. An example of an araliphatic such as an aralkyl group is benzyl.

As used herein, an "aralkyl" group refers to an alkyl group (e.g., a C₁₋₄ alkyl group) that is substituted with an aryl group. Both "alkyl" and "aryl" have been defined above. An example of an aralkyl group is benzyl. An aralkyl is optionally substituted with one or more substituents such as aliphatic [e.g., alkyl, alkenyl, or alkynyl, including carboxyalkyl, hydroxyalkyl, or haloalkyl such as trifluoromethyl], cycloaliphatic [e.g., cycloalkyl or cycloalkenyl], (cycloalkyl)alkyl, heterocycloalkyl, (heterocycloalkyl)alkyl, aryl, heteroaryl, alkoxy, cycloalkyloxy, heterocycloalkyloxy, aryloxy, heteroaryloxy, aralkyloxy, heteroaralkyloxy, aroyl, heteroaroyl, nitro, carboxy, alkoxycarbonyl, alkylcarbonyloxy, amido [e.g., aminocarbonyl, alkylcarbonylamino, cycloalkylcarbonylamino, (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, or heteroaralkylcarbonylamino], cyano, halo, hydroxy, acyl, mercapto, alkylsulfanyl, sulfoxy, urea, thiourea, sulfamoyl, sulfamide, oxo, or carbamoyl.

As used herein, a "bicyclic ring system" includes 8-12 (e.g., 9, 10, or 11) membered structures that form two rings, wherein the two rings have at least one atom in common (e.g., 2 atoms in common). Bicyclic ring systems include bicycloaliphatics (e.g., bicycloalkyl or bicycloalkenyl), bicycloheteroaliphatics, bicyclic aryls, and bicyclic heteroaryls.

As used herein, a "cycloaliphatic" group encompasses a "cycloalkyl" group and a "cycloalkenyl" group, each of which being optionally substituted as set forth below.

As used herein, a "cycloalkyl" group refers to a saturated carbocyclic mono- or bicyclic (fused or bridged) ring of 3-10 (e.g., 5-10) carbon atoms. Examples of cycloalkyl groups include cyclopropyl, cyclobutyl, cyclopentyl, cyclohexyl, cycloheptyl, adamantyl, norbornyl, cubyl, octahydro-indenyl, decahydro-naphthyl, bicyclo[3.2.1]octyl, bicyclo[2.2.2]octyl, bicyclo[3.3.1]nonyl, bicyclo[3.3.2.]decyl, bicyclo[2.2.2]octyl, adamantyl, or ((aminocarbonyl)cycloalkyl)cycloalkyl.

A "cycloalkenyl" group, as used herein, refers to a non-aromatic carbocyclic ring of 3-10 (e.g., 4-8) carbon atoms having one or more double bonds. Examples of cycloalkenyl groups include cyclopentenyl, 1,4-cyclohexa-di-enyl, cycloheptenyl, cyclooctenyl, hexahydro-indenyl, octahydro-naphthyl, cyclohexenyl, cyclopentenyl, bicyclo[2.2.2]octenyl, or bicyclo[3.3.1]nonenyl.

A cycloalkyl or cycloalkenyl group can be optionally substituted with one or more substituents such as phosphor, aliphatic [e.g., alkyl, alkenyl, or alkynyl], cycloaliphatic, (cycloaliphatic) aliphatic, heterocycloaliphatic, (heterocycloaliphatic) aliphatic, aryl, heteroaryl, alkoxy, (cycloaliphatic)oxy, (heterocycloaliphatic)oxy, aryloxy, heteroaryloxy, (araliphatic)oxy, (heteroaraliphatic)oxy, aroyl, heteroaroyl, amino, amido [e.g., (aliphatic)carbonylamino, (cycloaliphatic)carbonylamino, ((cycloaliphatic)aliphatic)carbonylamino, (aryl)carbonylamino, (araliphatic)carbonylamino, (heterocycloaliphatic)carbonylamino, ((heterocycloaliphatic)aliphatic)carbonylamino, (heteroaryl)carbonylamino, or (heteroaraliphatic)carbonylamino], nitro, carboxy [e.g., HOOC-, alkoxycarbonyl, or alkylcarbonyloxy], acyl [e.g., (cycloaliphatic)carbonyl, ((cycloaliphatic) aliphatic)carbonyl, (araliphatic)carbonyl, (heterocycloaliphatic)carbonyl, ((heterocycloaliphatic)aliphatic)carbonyl, or (heteroaraliphatic)carbonyl], cyano, halo, hydroxy, mercapto, sulfonyl [e.g., alkyl-SO₂- and aryl-SO₂-], sulfinyl [e.g., alkyl-S(O)-], sulfanyl [e.g., alkyl-S-], sulfoxy, urea, thiourea, sulfamoyl, sulfamide, oxo, or carbamoyl.

As used herein, the term "heterocycloaliphatic" encompasses a heterocycloalkyl group and a heterocycloalkenyl group, each of which being optionally substituted as set forth below.

As used herein, a "heterocycloalkyl" group refers to a 3-10 membered mono- or bicylic (fused or bridged) (e.g., 5- to 10-membered mono- or bicyclic) saturated ring structure, in which one or more of the ring atoms is a heteroatom (e.g., N, O, S, or combinations thereof). Examples of a heterocycloalkyl group include piperidyl, piperazyl, tetrahydropyranyl, tetrahydrofuryl, 1,4-dioxolanyl, 1,4-dithianyl, 1,3-dioxolanyl, oxazolidyl, isoxazolidyl, morpholinyl, thiomorpholyl, octahydrobenzofuryl, octahydrochromenyl, octahydrothiochromenyl, octahydroindolyl, octahydropyrindinyl, decahydroquinolinyl, octahydrobenzo[*b*]thiopheneyl, 2-oxa-bicyclo[2.2.2]octyl, 1-aza-bicyclo[2.2.2]octyl, 3-aza-bicyclo[3.2.1]octyl, and 2,6-dioxa-tricyclo[3.3.1.0^{3,7}]nonyl. A monocyclic heterocycloalkyl group can be fused with a phenyl moiety to form structures, such as tetrahydroisoquinoline, which would be categorized as heteroaryls.

A "heterocycloalkenyl" group, as used herein, refers to a mono- or bicylic (e.g., 5- to 10-membered mono- or bicyclic) non-aromatic ring structure having one or more double bonds, and wherein one or more of the ring atoms is a heteroatom (e.g., N, O, or S). Monocyclic and bicyclic heterocycloaliphatics are numbered according to standard chemical nomenclature.

A heterocycloalkyl or heterocycloalkenyl group can be optionally substituted with one or more substituents such as phosphor, aliphatic [e.g., alkyl, alkenyl, or alkynyl], cycloaliphatic, (cycloaliphatic)aliphatic, heterocycloaliphatic, (heterocycloaliphatic)aliphatic, aryl, heteroaryl, alkoxy, (cycloaliphatic)oxy, (heterocycloaliphatic)oxy, aryloxy, heteroaryloxy, (araliphatic)oxy, (heteroaraliphatic)oxy, aroyl, heteroaroyl, amino, amido [e.g., (aliphatic)carbonylamino, (cycloaliphatic)carbonylamino, ((cycloaliphatic) aliphatic)carbonylamino, (aryl)carbonylamino, (araliphatic)carbonylamino, (heterocycloaliphatic)carbonylamino, ((heterocycloaliphatic) aliphatic)carbonylamino, (heteroaryl)carbonylamino, or (heteroaraliphatic)carbonylamino], nitro, carboxy [e.g., HOOC-, alkoxycarbonyl, or alkylcarbonyloxy], acyl [e.g., (cycloaliphatic)carbonyl, ((cycloaliphatic) aliphatic)carbonyl, (araliphatic)carbonyl, (heterocycloaliphatic)carbonyl, ((heterocycloaliphatic)aliphatic)carbonyl, or (heteroaraliphatic)carbonyl], nitro, cyano, halo, hydroxy, mercapto, sulfonyl [e.g., alkylsulfonyl or arylsulfonyl], sulfinyl [e.g., alkylsulfinyl], sulfanyl [e.g., alkylsulfanyl], sulfoxy, urea, thiourea, sulfamoyl, sulfamide, oxo, or carbamoyl.

A "heteroaryl" group, as used herein, refers to a monocyclic, bicyclic, or tricyclic ring system having 4 to 15 ring atoms wherein one or more of the ring atoms is a heteroatom (e.g., N, O, S, or combinations thereof) and in which the monocyclic ring system is aromatic or at least one of the rings in the bicyclic or tricyclic ring systems is aromatic. A heteroaryl group includes a benzofused ring system having 2 to 3 rings. For example, a benzofused group includes benzo fused with one or two 4 to 8 membered heterocycloaliphatic moieties (e.g., indolizyl, indolyl, isoindolyl, 3H-indolyl, indolinyl, benzo[*b*]furyl, benzo[*b*]thiophenyl, quinolinyl, or isoquinolinyl). Some examples of heteroaryl are azetidinyl, pyridyl, 1H-indazolyl, furyl, pyrrolyl, thienyl, thiazolyl, oxazolyl, imidazolyl, tetrazolyl, benzofuryl, isoquinolinyl, benzthiazolyl, xanthene, thioxanthene, phenothiazine, dihydroindole, benzo[1,3]dioxole, benzo[b]furyl, benzo[b]thiophenyl, indazolyl, benzimidazolyl, benzthiazolyl, puryl, cinnolyl, quinolyl, quinazolyl,cinnolyl, phthalazyl, quinazolyl, quinoxalyl, isoquinolyl, 4H-quinolizyl, benzo-1,2,5-thiadiazolyl, or 1,8-naphthyridyl.

Without limitation, monocyclic heteroaryls include furyl, thiophenyl, 2H-pyrrolyl, pyrrolyl, oxazolyl, thiazolyl, imidazolyl, pyrazolyl, isoxazolyl, isothiazolyl, 1,3,4-thiadiazolyl, 2H-pyranyl, 4-H-pranyl, pyridyl, pyridazyl, pyrimidyl, pyrazolyl, pyrazyl, or 1,3,5-triazyl. Monocyclic heteroaryls are numbered according to standard chemical nomenclature.

Without limitation, bicyclic heteroaryls include indolizyl, indolyl, isoindolyl, 3H-indolyl, indolinyl, benzo[*b*]furyl, benzo[*b*]thiophenyl, quinolinyl, isoquinolinyl, indolizyl, isoindolyl, indolyl, benzo[*b*]furyl, bexo[*b*]thiophenyl, indazolyl, benzimidazyl, benzthiazolyl, purinyl, 4H-quinolizyl, quinolyl, isoquinolyl, cinnolyl, phthalazyl, quinazolyl, quinoxalyl, 1,8-naphthyridyl, or pteridyl. Bicyclic heteroaryls are numbered according to standard chemical nomenclature.

A heteroaryl is optionally substituted with one or more substituents such as aliphatic [e.g., alkyl, alkenyl, or alkynyl]; cycloaliphatic; (cycloaliphatic)aliphatic; heterocycloaliphatic; (heterocycloaliphatic)aliphatic; aryl; heteroaryl; alkoxy; (cycloaliphatic)oxy; (heterocycloaliphatic)oxy; aryloxy; heteroaryloxy; (araliphatic)oxy; (heteroaraliphatic)oxy; aroyl; heteroaroyl; amino; oxo (on a non-aromatic carbocyclic or heterocyclic ring of a bicyclic or tricyclic heteroaryl); carboxy; amido; acyl [e.g., aliphaticcarbonyl; (cycloaliphatic)carbonyl; ((cycloaliphatic)aliphatic)carbonyl; (araliphatic)carbonyl; (heterocycloaliphatic)carbonyl; ((heterocycloaliphatic)aliphatic)carbonyl; or (heteroaraliphatic)carbonyl]; sulfonyl [e.g., aliphaticsulfonyl or aminosulfonyl]; sulfinyl [e.g., aliphaticsulfinyl]; sulfanyl [e.g., aliphaticsulfanyl]; nitro; cyano; halo; hydroxy; mercapto; sulfoxy; urea; thiourea; sulfamoyl; sulfamide; or carbamoyl. Alternatively, a heteroaryl can be unsubstituted.

Non-limiting examples of substituted heteroaryls include (halo)heteroaryl [e.g., mono- and di-(halo)heteroaryl]; (carboxy)heteroaryl [e.g., (alkoxycarbonyl)heteroaryl]; cyanoheteroaryl; aminoheteroaryl [e.g., ((alkylsulfonyl)amino)heteroaryl and ((dialkyl)amino)heteroaryl]; (amido)heteroaryl [e.g., aminocarbonylheteroaryl, ((alkylcarbonyl)amino)heteroaryl, ((((alkyl)amino)alkyl)aminocarbonyl)heteroaryl, (((heteroaryl)amino)carbonyl)heteroaryl, ((heterocycloaliphatic)carbonyl)heteroaryl, and ((alkylcarbonyl)amino)heteroaryl]; (cyanoalkyl)heteroaryl; (alkoxy)heteroaryl; (sulfamoyl)heteroaryl [e.g., (aminosulfonyl)heteroaryl]; (sulfonyl)heteroaryl [e.g., (alkylsulfonyl)heteroaryl]; (hydroxyalkyl)heteroaryl; (alkoxyalkyl)heteroaryl; (hydroxy)heteroaryl; ((carboxy)alkyl)heteroaryl; (((dialkyl)amino)alkyl]heteroaryl; (heterocycloaliphatic)heteroaryl; (cycloaliphatic)heteroaryl; (nitroalkyl)heteroaryl; (((alkylsulfonyl)amino)alkyl)heteroaryl; ((alkylsulfonyl)alkyl)heteroaryl; (cyanoalkyl)heteroaryl; (acyl)heteroaryl [e.g., (alkylcarbonyl)heteroaryl]; (alkyl)heteroaryl, and (haloalkyl)heteroaryl [e.g., trihaloalkylheteroaryl].

A "heteroaraliphatic (such as a heteroaralkyl group) as used herein, refers to an aliphatic group (e.g., a C₁₋₄ alkyl group) that is substituted with a heteroaryl group. "Aliphatic," "alkyl," and "heteroaryl" have been defined above.

A "heteroaralkyl" group, as used herein, refers to an alkyl group (e.g., a C₁₋₄ alkyl group) that is substituted with a heteroaryl group. Both "alkyl" and "heteroaryl" have been defined above. A heteroaralkyl is optionally substituted with one or more substituents such as alkyl (including carboxyalkyl, hydroxyalkyl, and haloalkyl such as trifluoromethyl), alkenyl, alkynyl, cycloalkyl, (cycloalkyl)alkyl, heterocycloalkyl, (heterocycloalkyl)alkyl, aryl, heteroaryl, alkoxy, cycloalkyloxy, heterocycloalkyloxy, aryloxy, heteroaryloxy, aralkyloxy, heteroaralkyloxy, aroyl, heteroaroyl, nitro, carboxy, alkoxycarbonyl, alkylcarbonyloxy, aminocarbonyl, alkylcarbonylamino, cycloalkylcarbonylamino, (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino, cyano, halo, hydroxy, acyl, mercapto, alkylsulfanyl, sulfoxy, urea, thiourea, sulfamoyl, sulfamide, oxo, or carbamoyl.

As used herein, "cyclic moiety" and "cyclic group" refer to mono-, bi-, and tri-cyclic ring systems including cycloaliphatic, heterocycloaliphatic, aryl, or heteroaryl, each of which has been previously defined.

As used herein, a "bridged bicyclic ring system" refers to a bicyclic heterocycloaliphatic ring system or bicyclic cycloaliphatic ring system in which the rings are bridged. Examples of bridged bicyclic ring systems include, but are not limited to, adamantanyl, norbornanyl, bicyclo[3.2.1]octyl, bicyclo[2.2.2]octyl, bicyclo[3.3.1]nonyl, bicyclo[3.3.2]decyl, 2-oxabicyclo[2.2.2]octyl, 1-azabicyclo[2.2.2]octyl, 3-azabicyclo[3.2.1]octyl, and 2,6-dioxa-tricyclo[3.3.1.0^{3,7}]nonyl. A bridged bicyclic ring system can be optionally substituted with one or more substituents such as alkyl (including carboxyalkyl, hydroxyalkyl, and haloalkyl such as trifluoromethyl), alkenyl, alkynyl, cycloalkyl, (cycloalkyl)alkyl, heterocycloalkyl, (heterocycloalkyl)alkyl, aryl, heteroaryl, alkoxy, cycloalkyloxy, heterocycloalkyloxy, aryloxy, heteroaryloxy, aralkyloxy, heteroaralkyloxy, aroyl, heteroaroyl, nitro, carboxy, alkoxycarbonyl, alkylcarbonyloxy, aminocarbonyl, alkylcarbonylamino, cycloalkylcarbonylamino, (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino, cyano, halo, hydroxy, acyl, mercapto, alkylsulfanyl, sulfoxy, urea, thiourea, sulfamoyl, sulfamide, oxo, or carbamoyl.

As used herein, an "acyl" group refers to a formyl group or R^{X}-C(O)- (such as alkyl-C(O)-, also referred to as "alkylcarbonyl") where R^{X} and "alkyl" have been defined previously. Acetyl and pivaloyl are examples of acyl groups.

As used herein, an "aroyl" or "heteroaroyl" refers to an aryl-C(O)- or a heteroaryl-C(O)-, respectively. The aryl and heteroaryl portion of the aroyl or heteroaroyl is optionally substituted as previously defined.

As used herein, an "alkoxy" group refers to an alkyl-O- group where "alkyl" has been defined previously.

As used herein, a "carbamoyl" group refers to a group having the structure -O-CO-NR^{X}R^{Y} or -NR^{X}-CO-O-R^{Z}, wherein R^{X} and R^{Y} have been defined above and R^{Z} can be aliphatic, aryl, araliphatic, heterocycloaliphatic, heteroaryl, or heteroaraliphatic.

As used herein, a "carboxy" group refers to -COOH, -COOR^{X}, -OC(O)H, -OC(O)R^{X}, when used as a terminal group; or -OC(O)- or -C(O)O- when used as an internal group.

As used herein, a "haloaliphatic" group refers to an aliphatic group substituted with 1-3 halogen. For instance, the term haloalkyl includes the group -CF₃.

As used herein, a "mercapto" group refers to -SH.

As used herein, a "sulfo" group refers to -SO₃H or -SO₃R^{X} when used terminally or -S(O)₃- when used internally.

As used herein, a "sulfamide" group refers to the structure -NR^{X}-S(O)₂-NR^{Y}R^{Z} when used terminally and -NR^{X}-S(O)₂-NR^{Y}- when used internally, wherein R^{X}, R^{Y}, and R^{Z} have been defined above.

As used herein, a "sulfamoyl" group refers to the structure -O-S(O)₂-NR^{Y}R^{Z} wherein R^{Y} and R^{Z} have been defined above.

As used herein, a "sulfonamide" group refers to the structure -S(O)₂-NR^{X}R^{Y} or -NR^{X}-S(O)₂-R^{Z} when used terminally; or -S(O)₂-NR^{X}- or -NR^{X} -S(O)₂- when used internally, wherein R^{X}, R^{Y}, and R^{Z} are defined above.

As used herein a "sulfanyl" group refers to -S-R^{X} when used terminally and -S-when used internally, wherein R^{X} has been defined above. Examples of sulfanyls include aliphatic-S-, cycloaliphatic-S-, aryl-S-, or the like.

As used herein a "sulfinyl" group refers to -S(O)-R^{x} when used terminally and - S(O)- when used internally, wherein R^{X} has been defined above. Exemplary sulfinyl groups include aliphatic-S(O)-, aryl-S(O)-, (cycloaliphatic(aliphatic))-S(O)-, cycloalkyl-S(O)-, heterocycloaliphatic-S(O)-, heteroaryl-S(O)-, or the like.

As used herein, a "sulfonyl" group refers to-S(O)₂-R^{X} when used terminally and -S(O)₂- when used internally, wherein R^{X} has been defined above. Exemplary sulfonyl groups include aliphatic-S(O)₂-, aryl-S(O)₂-, (cycloaliphatic(aliphatic))-S(O)₂-, cycloaliphatic-S(O)₂-, heterocycloaliphatic-S(O)₂-, heteroaryl-S(O)₂-, (cycloaliphatic(amido(aliphatic)))-S(O)₂-or the like.

As used herein, a "sulfoxy" group refers to -O-SO-R^{X} or -SO-O-R^{X}, when used terminally and -O-S(O)- or -S(O)-O- when used internally, where R^{X} has been defined above.

As used herein, a "halogen" or "halo" group refers to fluorine, chlorine, bromine or iodine.

As used herein, an "alkoxycarbonyl," which is encompassed by the term carboxy, used alone or in connection with another group refers to a group such as alkyl-O-C(O)-.

As used herein, an "alkoxyalkyl" refers to an alkyl group such as alkyl-O-alkyl-, wherein alkyl has been defined above.

As used herein, a "carbonyl" refer to -C(O)-.

As used herein, an "oxo" refers to =O.

As used herein, the term "phospho" refers to phosphinates and phosphonates. Examples of phosphinates and phosphonates include -P(O)(R^{P})₂, wherein R^{P} is aliphatic, alkoxy, aryloxy, heteroaryloxy, (cycloaliphatic)oxy, (heterocycloaliphatic)oxy aryl, heteroaryl, cycloaliphatic or amino.

As used herein, an "aminoalkyl" refers to the structure (R^{X})₂N-alkyl-.

As used herein, a "cyanoalkyl" refers to the structure (NC)-alkyl-.

As used herein, a "urea" group refers to the structure -NR^{X}-CO-NR^{Y}R^{Z} and a "thiourea" group refers to the structure -NR^{X}-CS-NR^{Y}R^{Z} when used terminally and -NR^{X}-CO-NR^{Y}- or -NR^{X}-CS-NR^{Y}- when used internally, wherein R^{X}, R^{Y}, and R^{Z} have been defined above.

As used herein, a "guanidine" group refers to the structure -N=C(N(R^{X}R^{Y}))N(R^{X}R^{Y}) or -NR^{X}-C(=NR^{X})NR^{X}R^{Y} wherein R^{X} and R^{Y} have been defined above.

As used herein, the term "amidino" group refers to the structure -C=(NR^{X})N(R^{X}R^{Y}) wherein R^{X} and R^{Y} have been defined above.

In general, the term "vicinal" refers to the placement of substituents on a group that includes two or more carbon atoms, wherein the substituents are attached to adjacent carbon atoms.

In general, the term "geminal" refers to the placement of substituents on a group that includes two or more carbon atoms, wherein the substituents are attached to the same carbon atom.

The terms "terminally" and "internally" refer to the location of a group within a substituent. A group is terminal when the group is present at the end of the substituent not further bonded to the rest of the chemical structure. Carboxyalkyl, i.e., R^{X}O(O)C-alkyl is an example of a carboxy group used terminally. A group is internal when the group is present in the middle of a substituent of the chemical structure. Alkylcarboxy (e.g., alkyl-C(O)O- or alkyl-OC(O)-) and alkylcarboxyaryl (e.g., alkyl-C(O)O-aryl- or alkyl-O(CO)-aryl-) are examples of carboxy groups used internally.

As used herein, an "aliphatic chain" refers to a branched or straight aliphatic group (e.g., alkyl groups, alkenyl groups, or alkynyl groups). A straight aliphatic chain has the structure -[CH₂]ᵥ-, where v is 1-12. A branched aliphatic chain is a straight aliphatic chain that is substituted with one or more aliphatic groups. A branched aliphatic chain has the structure -[CQQ]ᵥ- where Q is independently a hydrogen or an aliphatic group; however, Q shall be an aliphatic group in at least one instance. The term aliphatic chain includes alkyl chains, alkenyl chains, and alkynyl chains, where alkyl, alkenyl, and alkynyl are defined above.

The phrase "optionally substituted" is used interchangeably with the phrase "substituted or unsubstituted". As described herein, compounds described herein can optionally be substituted with one or more substituents, such as are illustrated generally above, or as exemplified by particular classes, subclasses, and species of the invention. As described herein, the variables R₁, R₂, R'₂, R₃, and R₄, and other variables contained in Formula I, described herein, encompass specific groups, such as alkyl and aryl. Unless otherwise noted, each of the specific groups for the variables R₁, R₂, R'₂, R₃, and R₄, and other variables contained therein can be optionally substituted with one or more substituents described herein. Each substituent of a specific group is further optionally substituted with one to three of halo, cyano, oxo, alkoxy, hydroxy, amino, nitro, aryl, cycloaliphatic, heterocycloaliphatic, heteroaryl, haloalkyl, and alkyl. For instance, an alkyl group can be substituted with alkylsulfanyl and the alkylsulfanyl can be optionally substituted with one to three of halo, cyano, oxo, alkoxy, hydroxy, amino, nitro, aryl, haloalkyl, and alkyl. As an additional example, the cycloalkyl portion of a (cycloalkyl)carbonylamino can be optionally substituted with one to three of halo, cyano, alkoxy, hydroxy, nitro, haloalkyl, and alkyl. When two alkoxy groups are bound to the same atom or adjacent atoms, the two alkoxy groups can form a ring together with the atom(s) to which they are bound.

In general, the term "substituted," whether preceded by the term "optionally" or not, refers to the replacement of hydrogen radicals in a given structure with the radical of a specified substituent. Specific substituents are described above in the definitions and below in the description of compounds and examples thereof. Unless otherwise indicated, an optionally substituted group can have a substituent at each substitutable position of the group, and when more than one position in any given structure can be substituted with more than one substituent selected from a specified group, the substituent can be either the same or different at every position. A ring substituent, such as a heterocycloalkyl, can be bound to another ring, such as a cycloalkyl, to form a spiro-bicyclic ring system, e.g., both rings share one common atom. As one of ordinary skill in the art will recognize, combinations of substituents envisioned by this invention are those combinations that result in the formation of stable or chemically feasible compounds.

The phrase "stable or chemically feasible", as used herein, refers to compounds that are not substantially altered when subjected to conditions to allow for their production, detection, and preferably their recovery, purification, and use for one or more of the purposes disclosed herein. In some instances, a stable compound or chemically feasible compound is one that is not substantially altered when kept at a temperature of 40 °C or less, in the absence of moisture or other chemically reactive conditions, for at least a week.

As used herein, an "effective amount" is defined as the amount required to confer a therapeutic effect on the treated patient, and is typically determined based on age, surface area, weight, and condition of the patient. The interrelationship of dosages for animals and humans (based on milligrams per meter squared of body surface) is described by Freireich et al., Cancer Chemother. Rep., 50: 219 (1966). Body surface area maybe approximately determined from height and weight of the patient. See, e.g., Scientific Tables, Geigy Pharmaceuticals, Ardsley, New York, 537 (1970). As used herein, "patient" refers to a mammal, including a human.

Unless otherwise stated, structures depicted herein are also meant to include all isomeric (e.g., enantiomeric, diastereomeric, and geometric (or conformational)) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the compound of the invention are within the scope of the invention. Unless otherwise stated, all tautomeric forms of the compound of the invention are within the scope of the invention. Additionally, unless otherwise stated, structures depicted herein are also meant to include compounds that differ only in the presence of one or more isotopically enriched atoms. For example, compounds having the present structures except for the replacement of hydrogen by deuterium or tritium, or the replacement of a carbon by a ¹³C- or ¹⁴C-enriched carbon are within the scope of this invention. Such compounds are useful, for example, as analytical tools or probes in biological assays, or as therapeutic agents.

As used herein, an "adrenergic agonist" refers to any compound having agonistic activity toward any adrenergic receptor (e.g., β₁, β₂, β₃). Note that the terms "beta-adrenergic" and "β-adrenergic" are used interchangeably. This usage also applies to subtypes of beta agonists, (e.g., 'beta-1-adrenergic agonist' is used interchangeable with ' β1-adrenergic agonist' and/or' β₁-adrenergic agonist').

As used herein, the term "LDOPA" refers to L-3,4-dihydroxyphenylalanine.

As used herein, the term "co-crystal" refers to a substantially crystalline material having two or more distinct molecular components (e.g., a compound of formula I or a salt thereof and a phosphodiesterase inhibitor) within the crystal lattice.

Chemical structures and nomenclature are derived from ChemDraw, version 11.0.1, Cambridge, MA.

### II. PHARMACEUTICAL COMPOSITIONS

Thiazolidinedione compounds described herein are uniquely effective in treating or preventing neurodegenerative diseases (e.g., Alzheimer's Disease, Parkinson's Disease, ALS, MS, and MCI) in a patient and possess a reduced interaction with PPARy. Accordingly, these compounds demonstrate reduced side effects related to PPARy interaction than PPARy activating compounds.

### A. Compounds of Formula I

Described herein are pharmaceutical compositions that are useful for treating or preventing a neurodegenerative disorder in a patient comprising a compound of Formula I: 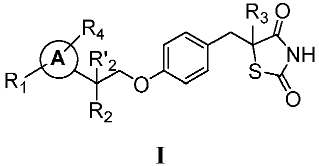 or a pharmaceutically acceptable salt thereof, wherein:
Each of R₁ and R₄ is independently selected from H, halo, aliphatic, and alkoxy, wherein the aliphatic or alkoxy is optionally substituted with 1-3 of halo;
R'₂ is H, and R₂ is H, halo, hydroxy, or optionally substituted aliphatic, -O-acyl, -O-aroyl,
   -O-heteroaroyl, -O(SO₂)NH₂, -O-CH(Rₘ)OC(O)Rₙ, -O-CH(Rₘ)OP(O)(ORₙ)₂ -OP(O)(ORₙ)₂, or 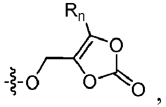 wherein each Rₘ is independently C₁₋₆ alkyl, each Rₙ is independently C₁₋₁₂ alkyl, C₃₋₈ cycloalkyl, or phenyl, each of which is optionally substituted; or R₂ and R'₂ together may form oxo;
R₃ is H or C₁₋₃ alkyl; and
Ring A is phenyl, pyridin-2-yl, pyridin-3-yl or pyridin-4-yl, each of which is optionally substituted.

In several instances, R₁ is H. In some instances, R₁ is halo, such as F or Cl. In some instances, R₁ is an aliphatic optionally substituted with 1-3 halo. For instance, R₁ is trifluoromethyl. In some instances, R₁ is alkoxy. For instance, R₁ is methoxy, ethoxy, or -O-isopropyl. In still other instances, R₁ is alkoxy substituted with 1-3 halo. For instance, R₁ is - OCHF₂ or -OCF₃. In each of the foregoing instances, R₁ can be substituted at the ortho, meta, or para position of ring A. In certain instances, R₁ is substituted at the para or meta position of ring A.

In several instances, R₄ is H. In some instances, R₄ is halo, such as F or Cl. In some instances, R₄ is an aliphatic optionally substituted with 1-3 halo. For instance, R₄ is trifluoromethyl. In some instances R₄ is alkoxy. For instance, R₄ is methoxy, ethoxy, or -O-isopropyl. In still other instances, R₄ is alkoxy substituted with 1-3 halo. For instance, R₄ is - OCHF₂ or -OCF₃. In each of the foregoing instances, R₄ can be substituted at the ortho, meta, or para position of ring A. In certain instances, R₄ is substituted at the para or meta position of ring A. In some instances, R₁ and R₄ are different substituents. In still other instances, R₁ and R₄ are the same substituent. In some instances when R₁ is aliphatic, R₄ is other than H.

In several instances, each of R₁ and R₄ is independently selected from H, halo, aliphatic, and alkoxy, wherein the aliphatic and alkoxy are optionally substituted with 1-3 of halo.

In several instances, each of R₁ and R₄ is independently selected from H, halo, aliphatic, and alkoxy, wherein the aliphatic and alkoxy are optionally substituted with 1-3 of halo.

In several instances, R₂ is halo, hydroxy, aliphatic, -O-acyl, -O-aroyl, -O-heteroaroyl, -O(SO₂)NH₂, -O-CH(Rₘ)OC(O)Rₙ, -O-CH(Rₘ)OP(O)(ORₙ)₂, -O-P(O)(ORₙ)₂, or 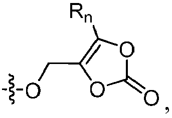 wherein each Rₘ is C₁₋₆ alkyl, Rₙ is C₁₋₁₂ alkyl, C₃₋₈ cycloalkyl, or phenyl and each substituent Rₘ or Rₙ is optionally substituted

In some instances, R₂ is H.

In some instances, R₂ is hydroxy.

In some instances, R₂ is an optionally substituted straight or branched C₁₋₆ alkyl, an optionally substituted straight or branched C₂₋₆ alkenyl, or an optionally substituted straight or branched C₂₋₆ alkynyl. In other instances, R₂ is a C₁₋₆ aliphatic optionally substituted with 1-2 hydroxy, carboxy or halo. In other instances, R₂ is a C₁₋₆ alkyl optionally substituted with hydroxy. In further instances, R₂ is a C₁₋₆ alkyl optionally substituted with -O-acyl, -O-aroyl, - O-heteroaroyl. In several other instances, R₂ is a methyl, ethyl, propyl, isopropyl, butyl, tert-butyl, pentyl, or hexyl, each of which is optionally substituted with hydroxy. In several additional instances, R₂ is methyl or ethyl, each of which is substituted with hydroxy.

In certain instances, R₂ is -O-acyl, -O-aroyl, or -O-heteroaryoyl.

In other instances, R₂ is -O-acetyl, -O-hexanoyl, -O-benzoyl, -O-pivaloyl, -O-imidazolyl, -O-succinoyl, -O-thiazoloyl or -O-pyridinoyl, each optionally substituted.

In some instances, R₂ is -O-C(O)-imidazol-1-yl.

In certain instances, R₂ is -O-CH(Rₘ)-O-C(O)-Rₙ.

In some instances, R₂ is -O-CH(Rₘ)OP(O)(ORₙ)₂.

In some instances, R₂ is -O-P(O)(ORₙ)₂.

In other instances, R₂ is -O-S(O₂)NH₂.

In some further instances, R₂ is a 1,3-dioxolan-2-one of the Formula 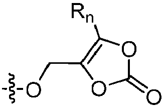 , wherein Rₘ and Rₙ are as previously described.

In several instances, R'₂ is H.

In some instances, R₂ and R'₂ together form oxo.

In some instances, R'₂ is H and R₂ has an R configuration.

In some instances, R'₂ is H and R₂ has an S configuration.

In some instances, R'₂ is H and R₂ is racemic.

In further instances, ring A is phenyl or pyridinyl.

In some instances, ring A is pyridin-2-yl.

In some instances, ring A is pyridin-3-yl.

In some instances, ring A is pyridin-4-yl.

In other instances, R₃ is H or optionally substituted C₁₋₃ alkyl.

In some instances, R₃ is H.

In some instances, R₃ is CH₃.

In several instances, the composition further comprises a pharmaceutically acceptable carrier.

Also described herein is a pharmaceutical composition to include a compound of Formula II, IIA, or IIB: 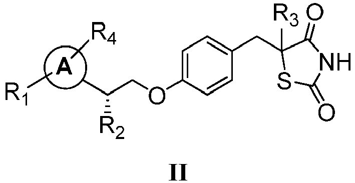 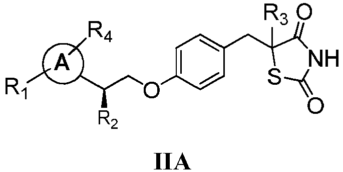 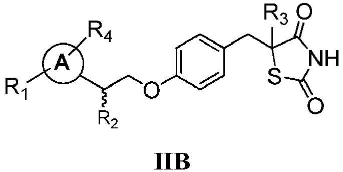 or a pharmaceutically acceptable salt thereof, wherein R'₂ is H; and R₁, R₃, R₄, and ring A are defined above in Formula I.

Exemplary compositions described herein include a single unit dosage form having about 1 mg to about 200 mg (e.g., about 10 mg to about 120 mg, about 10 mg to about 100 mg, or about 15 mg to about 60 mg) of a compound of Formula I, II, IIA, IIB, III, IIIA, IIIB, IVA or IVB.

Several exemplary compounds of Formula I, wherein R₂ and R'₂ together form oxo and Ring A is phenyl are shown in Table A, below.

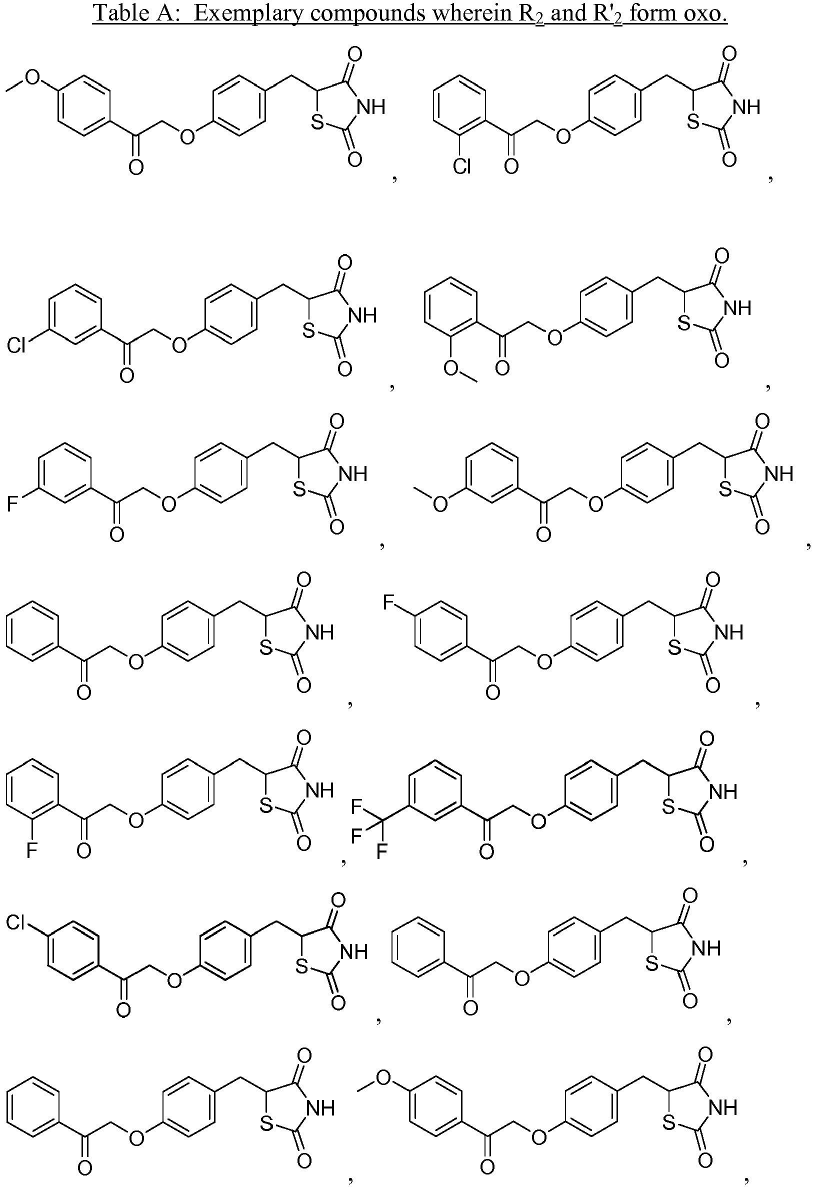

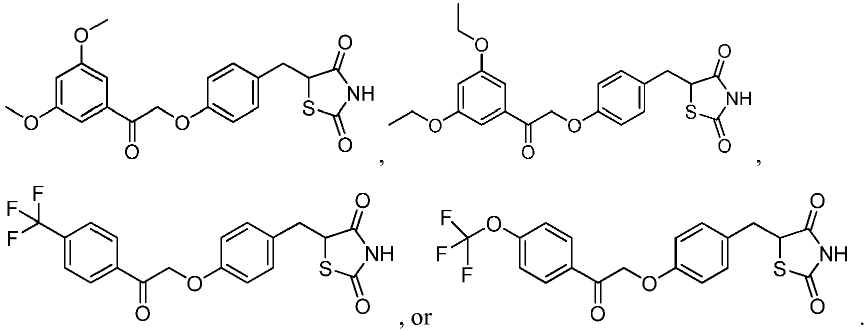

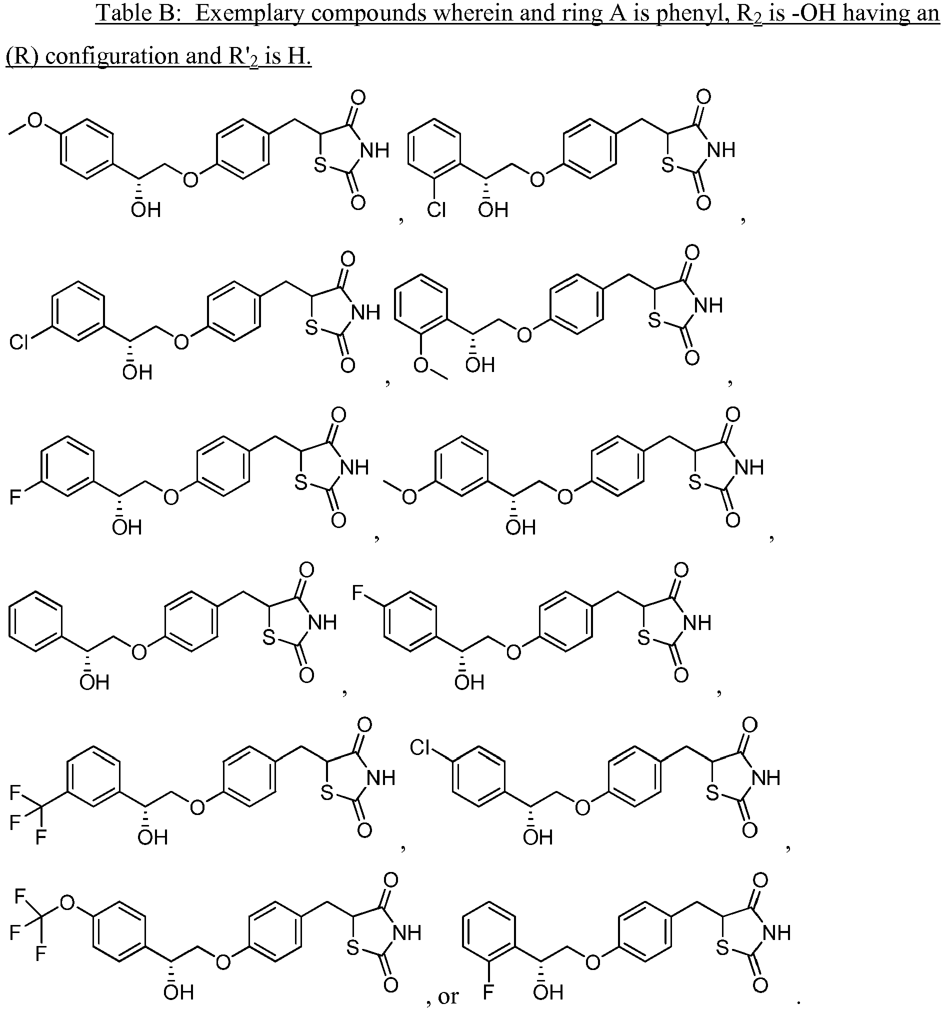

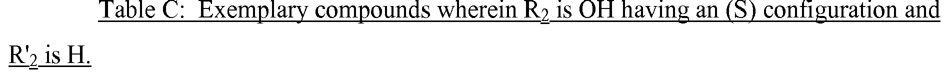

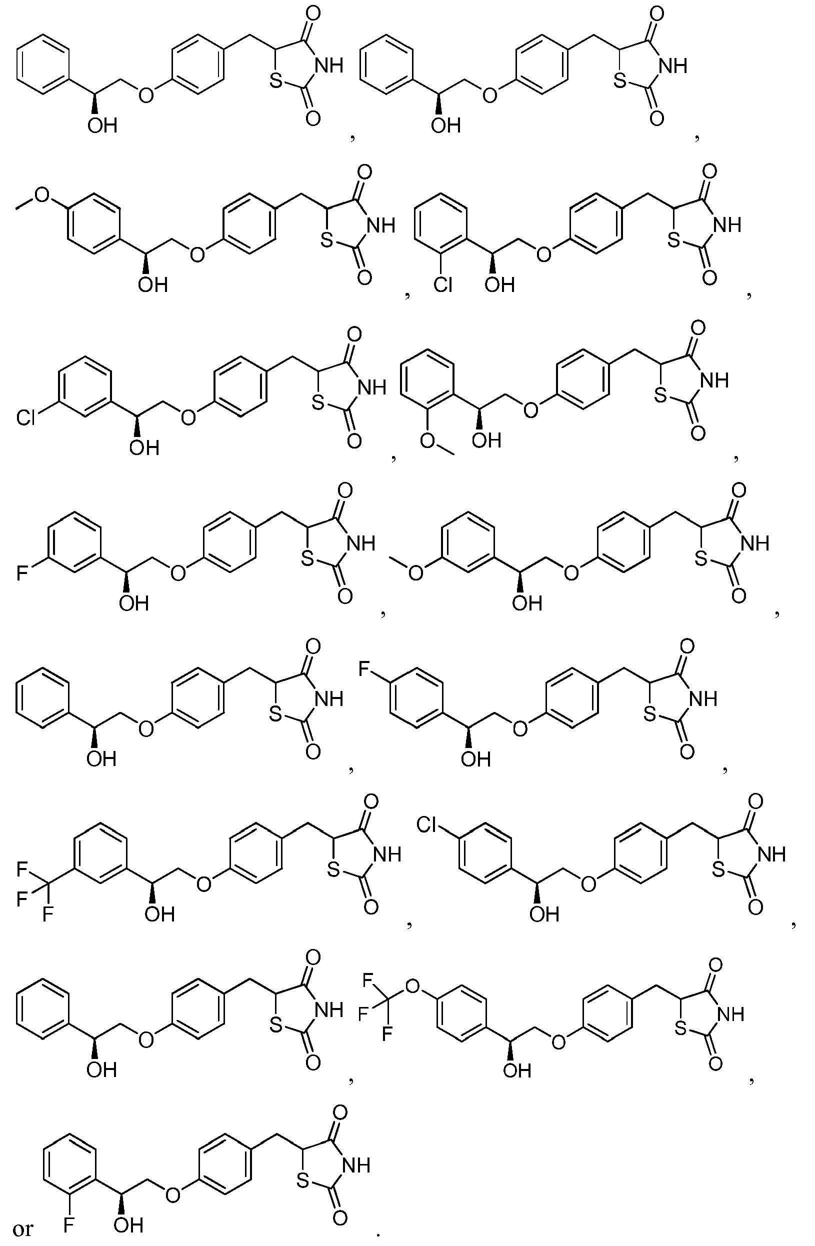

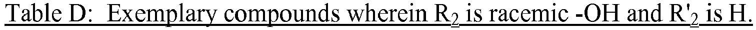

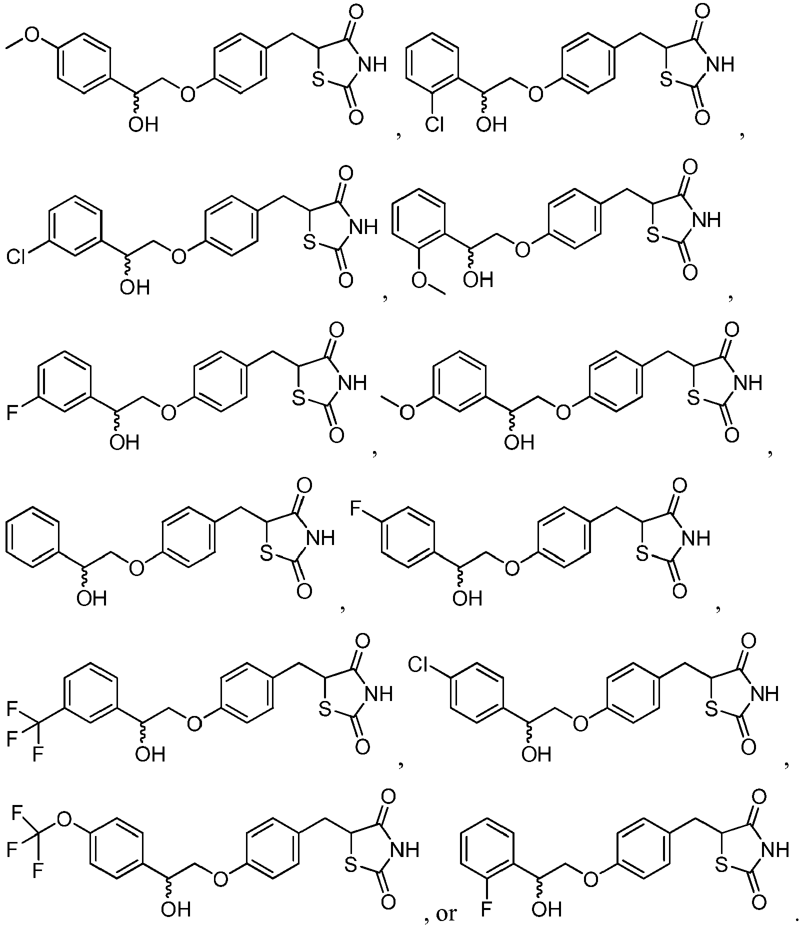

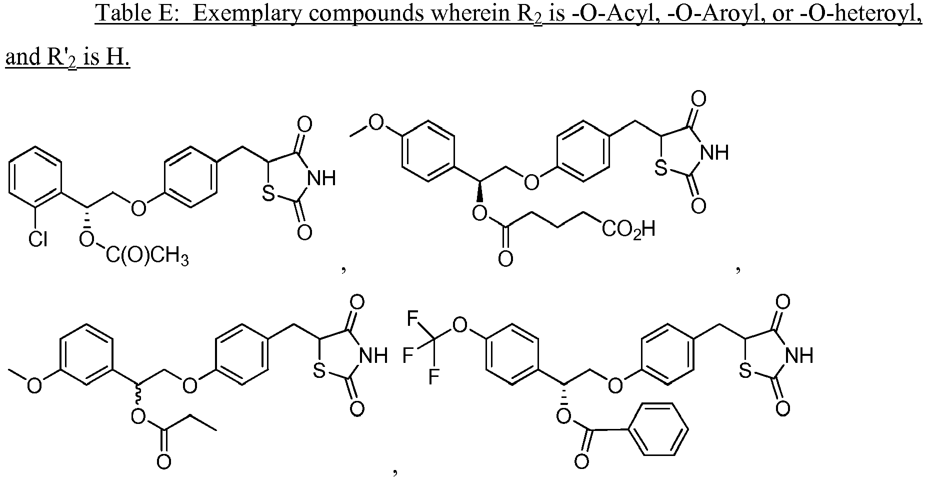

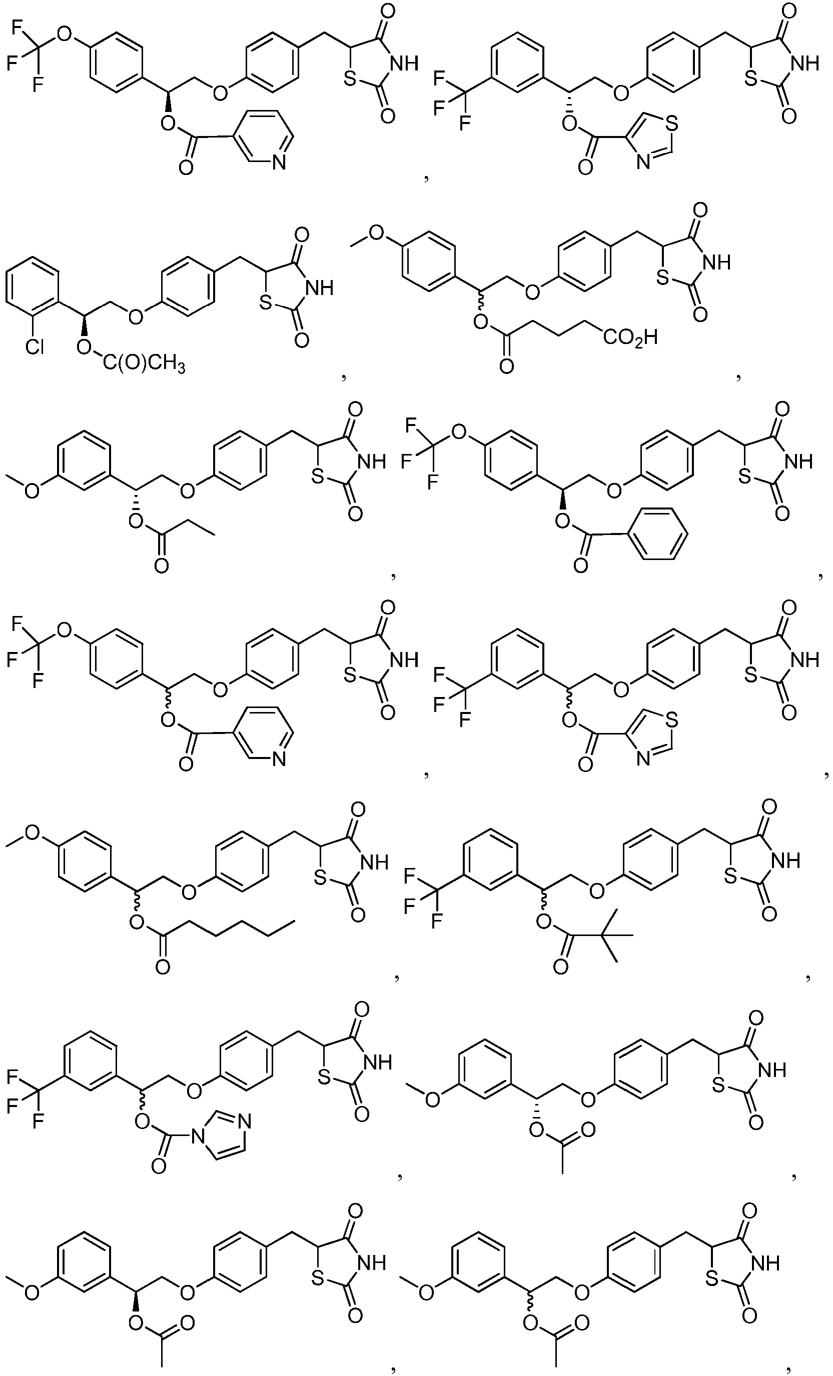

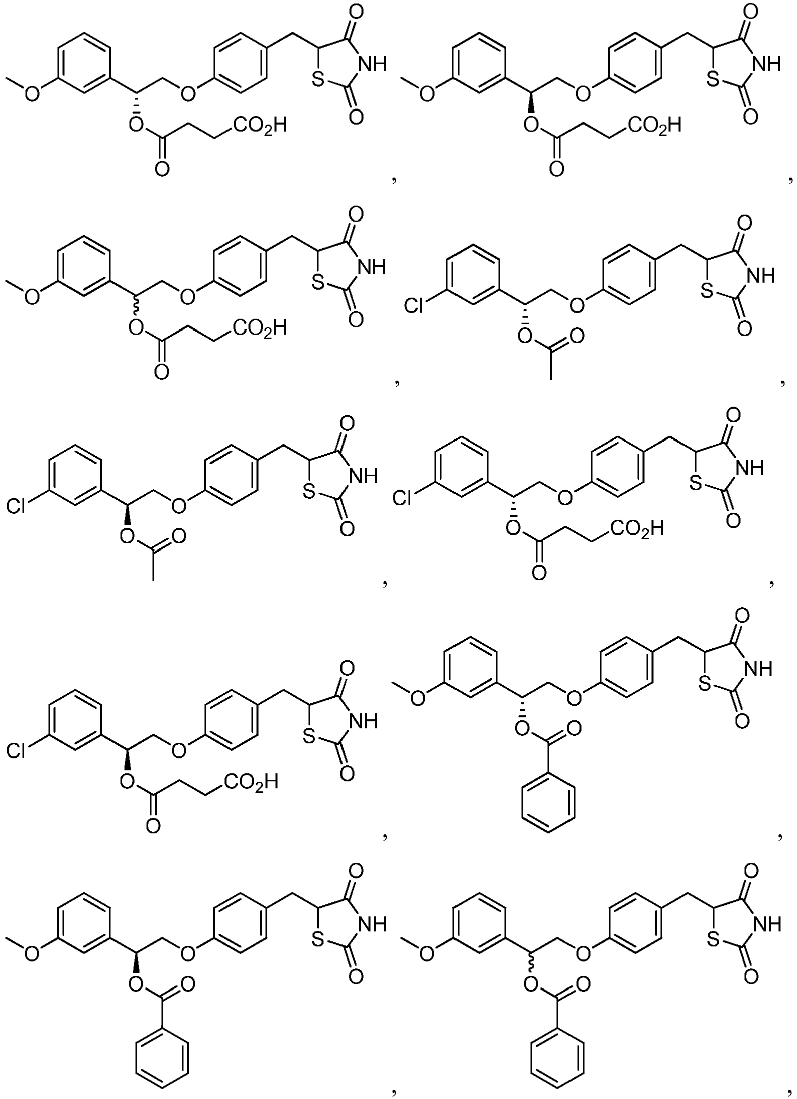

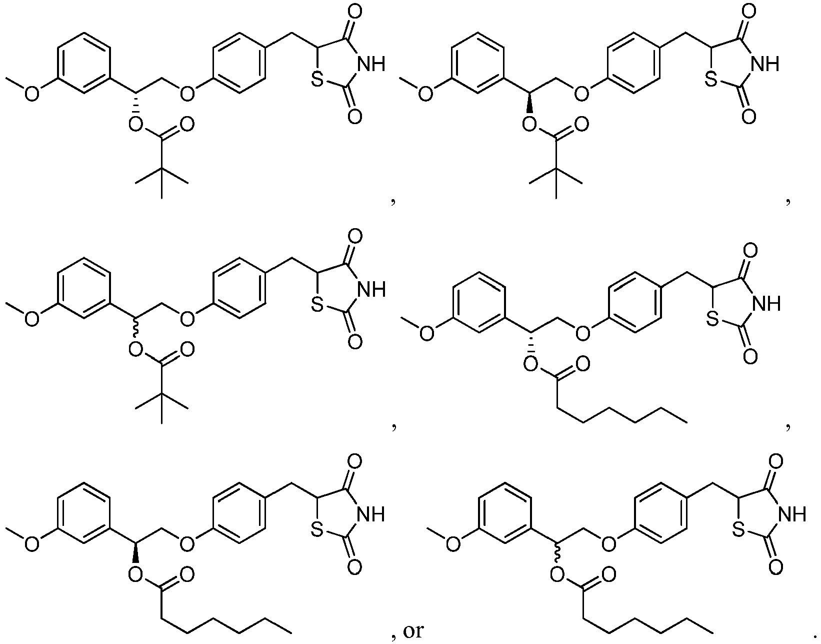

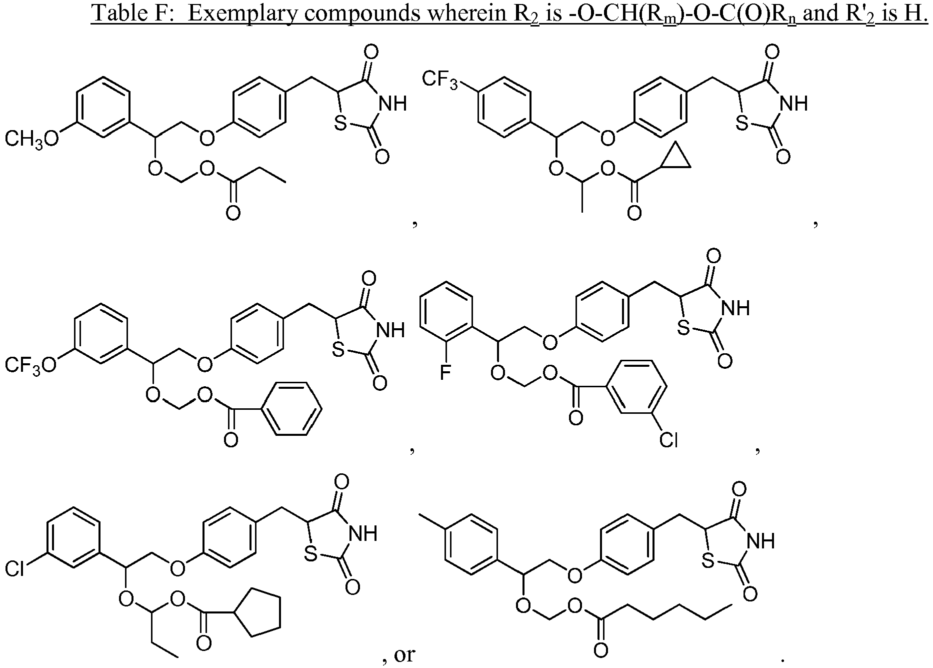

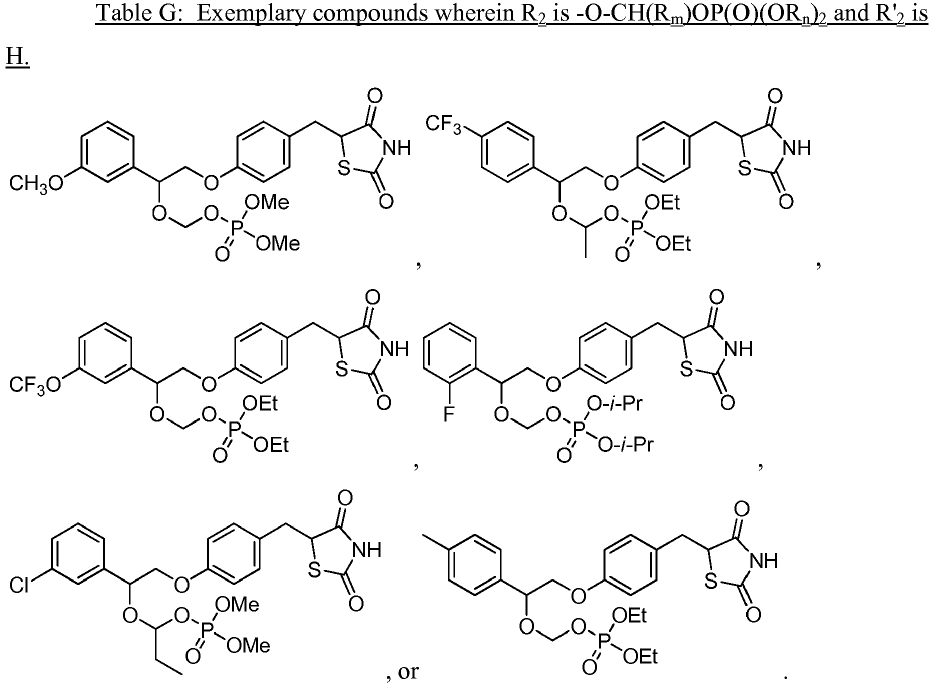

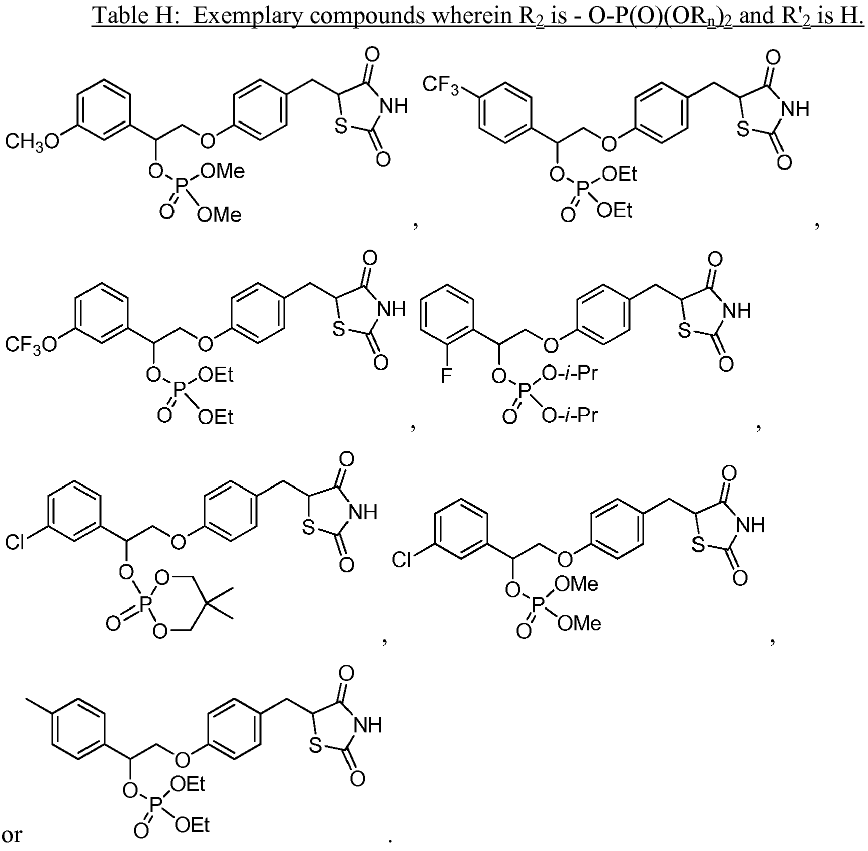

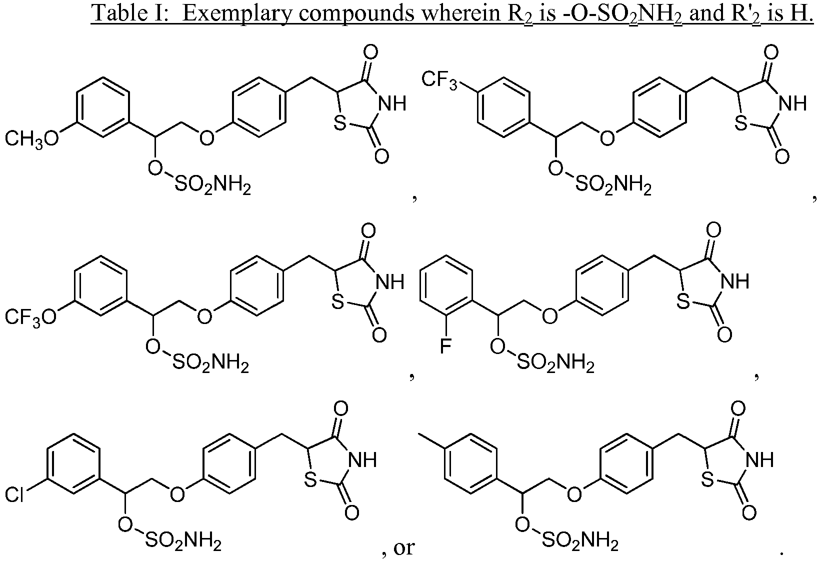

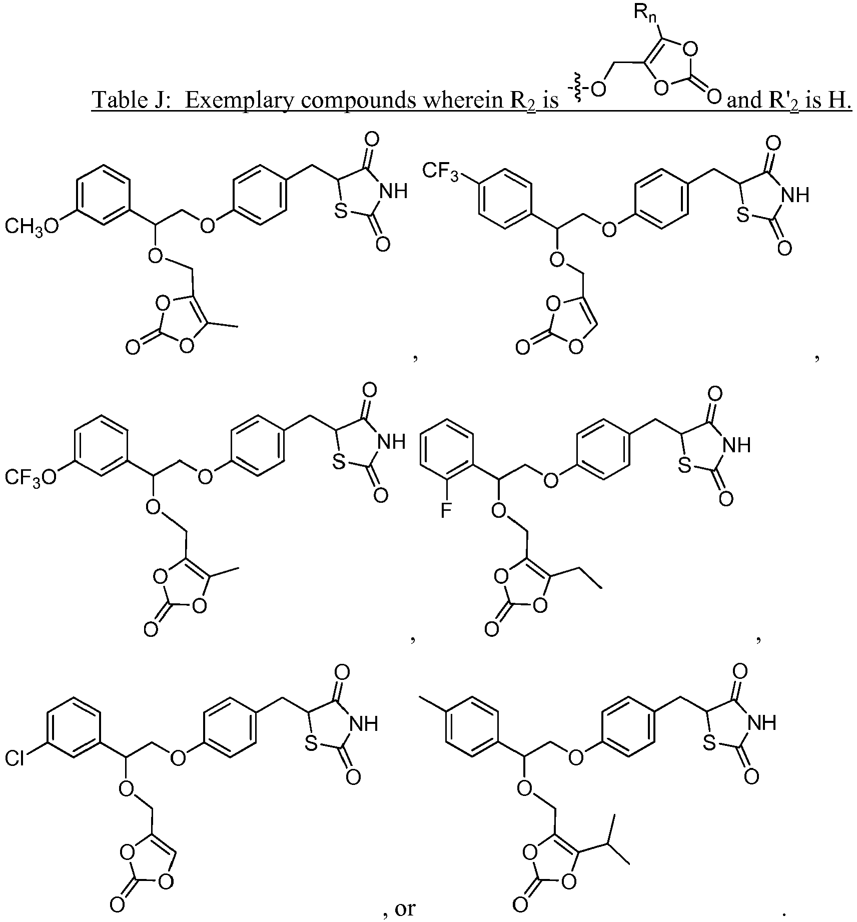

Further described herein are compounds of Formula III: 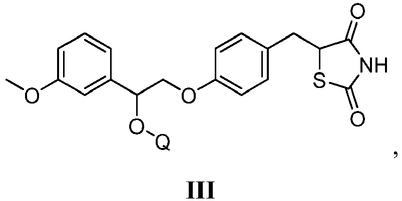 wherein Q is acyl, aroyl, heteroaroyl, -SO₂NH₂, -CH(Rₘ)OC(O)Rₙ, -CH(Rₘ)OP(O)(ORₙ)₂, -P(O)(ORₙ)₂, or 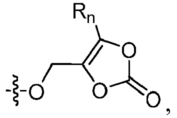 wherein each Rₘ is C₁₋₆ alkyl, Rₙ is C₁₋₁₂ alkyl, C₃₋₈ cycloalkyl, or phenyl, wherein each substituent is optionally substituted.

In some instances, Q in formula III is acyl.

In some instances, Q in formula III is -acetyl, -hexanoyl, -benzoyl, -pivaloyl, -succinoyl, each optionally substituted.

In certain instances, Q in formula III is acetyl.

In certain instances, Q in formula III is hexanoyl.

In certain instances, Q in formula III is benzoyl.

In certain instances, Q in formula III is pivaloyl.

In certain instances, Q in formula III is succinoyl.

In some instances, the compound of Formula I has is a compound of Formula IIIA or IIIB: 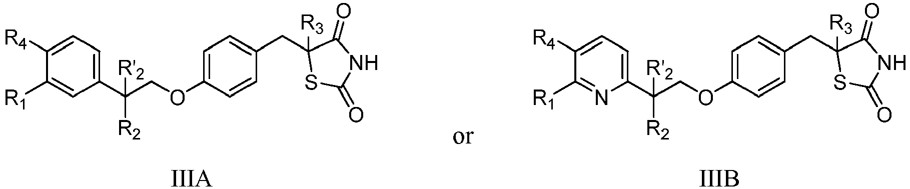 or a pharmaceutically acceptable salt thereof, wherein each of R₁, R₂, R'₂, R₃, and R₄ are defined above in Formula I.

In some instances, in the compound of Formula IIIA, one of R₁ and R₄ is an alkyl or alkoxy and the other is hydrogen. For instance, one of R₁ and R₄ is methyl, ethyl, or propyl, and the other is hydrogen. In other instances, one of R₁ and R₄ is methoxy or ethoxy.

In some instances, in the compound of Formula IIIB, one of R₁ and R₄ is an alkyl or alkoxy and the other is hydrogen. For instance, one of R₁ and R₄ is methyl, ethyl, or propyl, and the other is hydrogen. In other instances, one of R₁ and R₄ is methoxy or ethoxy.

Several exemplary compounds of Formula I, wherein R₂ and R'₂ together form oxo and Ring A is phenyl are shown in Table A, above.

Also described herein is a pharmaceutical composition which includes compounds of the Formula IVA or IVB: 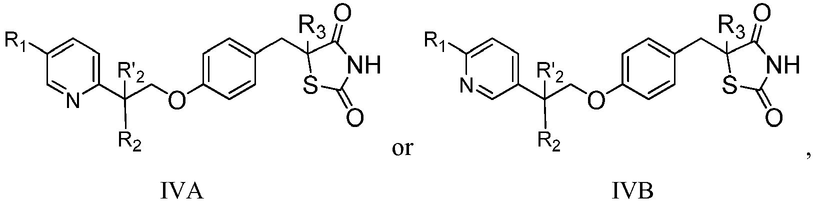 wherein R'₂ is H, R₁ and R₃ are as defined above for Formula **I**, ring A is pyridin-2-yl or pyridin-3-yl, and R₂ is H, -OH, -O-acyl, -O-aroyl or -O-heteroaryoyl; or R₂ and R'₂ together form oxo.

In further instances, Q in formula IVA or IVB is H, -O-acetyl, -O-hexanoyl, -O-benzoyl, -O-pivaloyl, -O-succinoyl, each optionally substituted.

In some instances, Q in formula IVA or IVB is H.

In certain instances, Q in formula IVA or IVB is -O-acetyl.

In certain instances, Q in formula IVA or IVB is -O-hexanoyl.

In certain instances, Q is in formula IVA or IVB -O-benzoyl.

In certain instances, Q is in formula IVA or IVB -O-pivaloyl.

In certain instances, Q is in formula IVA or IVB -O-succinoyl.

Several exemplary compounds of Formulae IVA and IVB are shown in Tables K and L below.

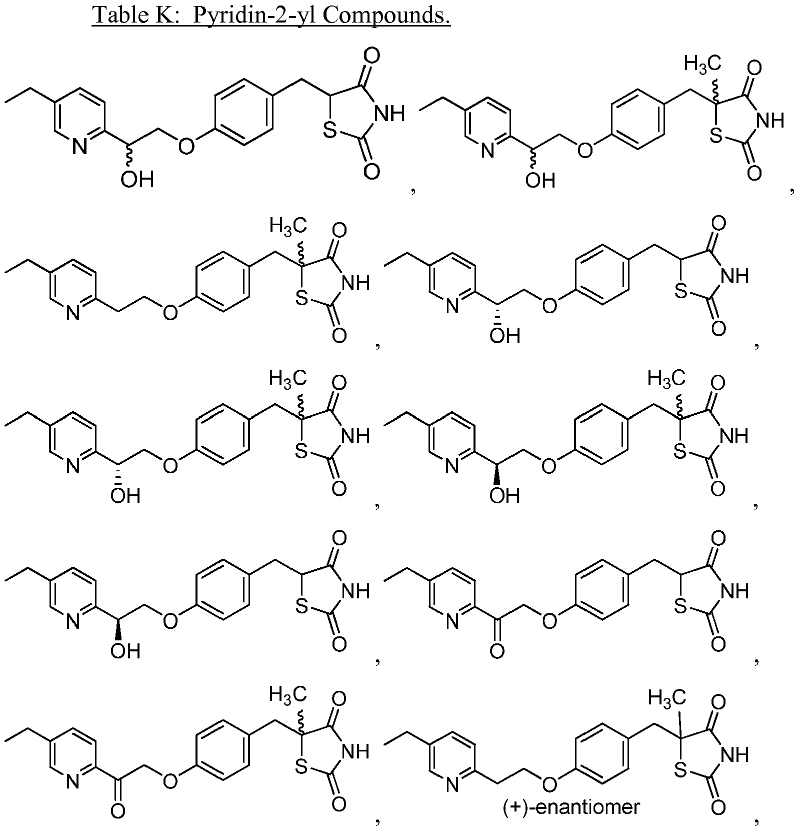

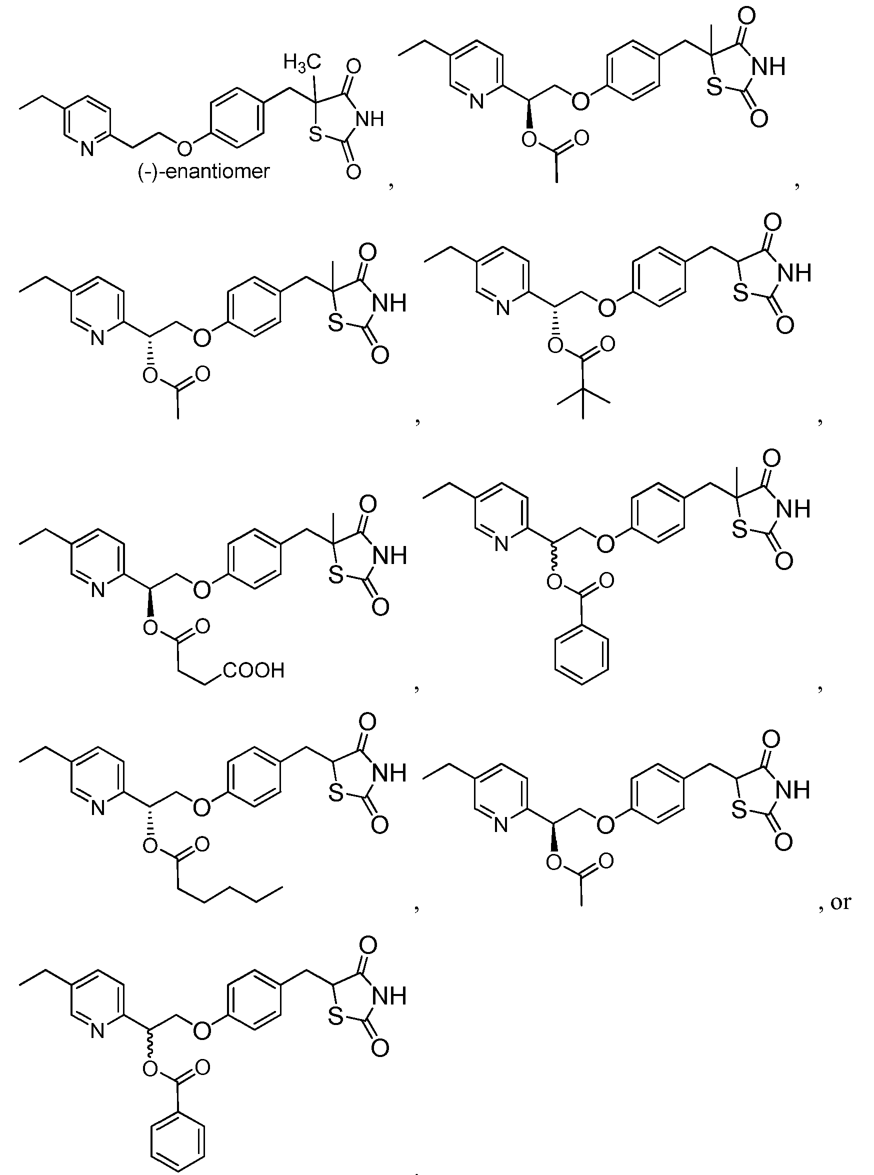

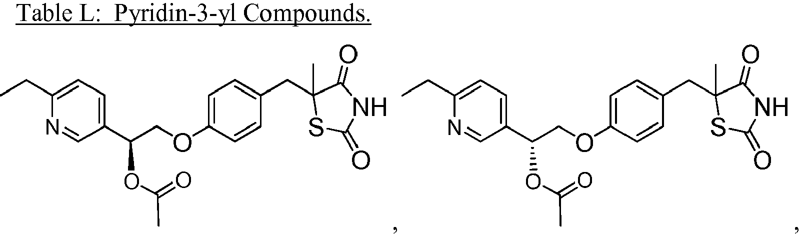

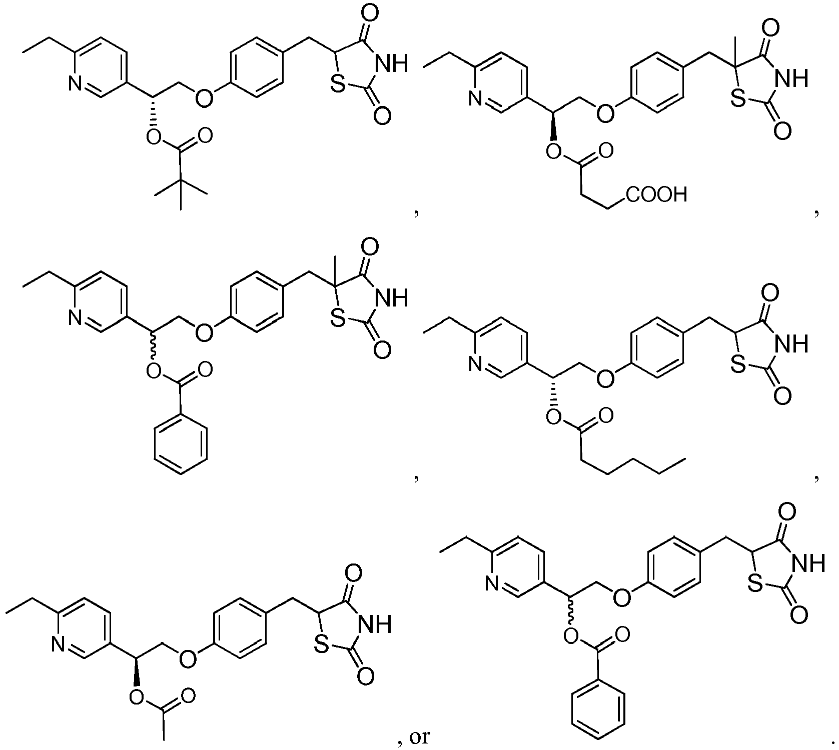

Further described herein is a compound selected from: 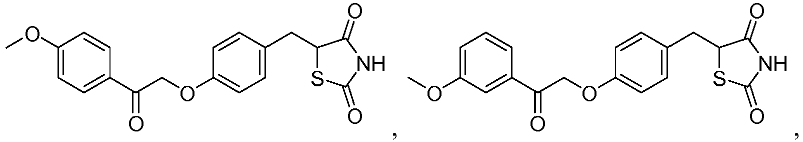 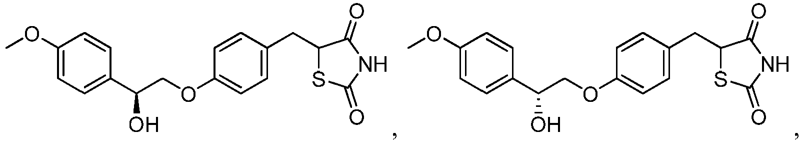 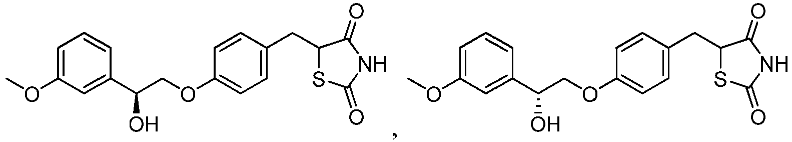 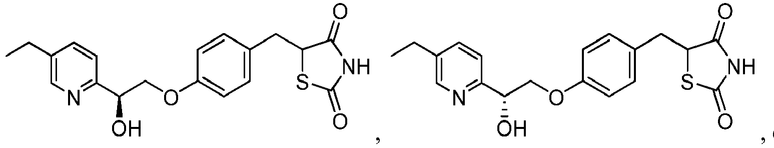 or 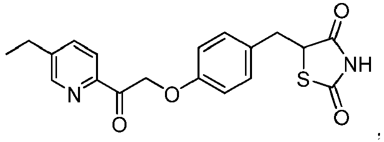 and a phosphodiesterase inhibitor (e.g., caffeine, IBMX, or any combination thereof).

Also described herein is a pharmaceutical composition comprising a compound of Formula I, II, IIA, IIB, III, IIIA, IIIB, IVA, or IVB and LDOPA. This composition is useful for the methods described below (e.g., treating Parkinson's Disease).

In some instances, the compound of Formula I is selected from: 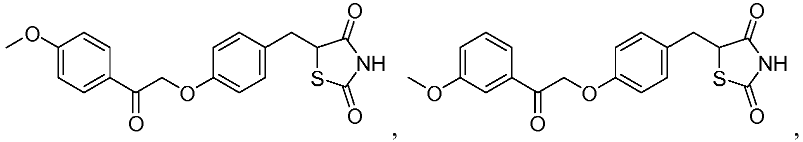 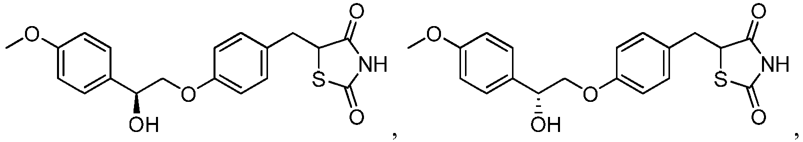 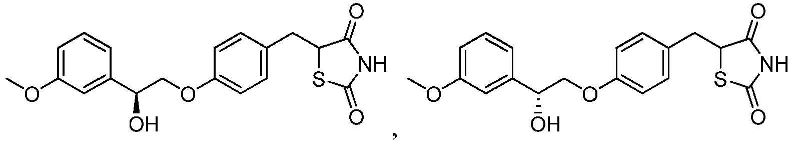 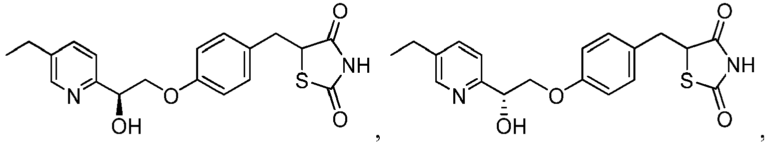 or

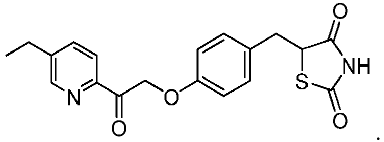

Another aspect described herein is a pharmaceutical composition comprising an alkali earth metal salt of a compound of Formula I, II, IIA, IIB, III, IIIA, IIIB, IVA, or IVB and LDOPA.

### B. Co-Crystals of a Compound of Formula I

Described herein is a method of treating or preventing a neurodegenerative disorder in a patient comprising administering to the patient a pharmaceutical composition comprising a co-crystal comprising a compound of Formula I or a pharmaceutically acceptable salt thereof, as described above, and a phosphodiesterase inhibitor. In several instances, the phosphodiesterase inhibitor is a selective inhibitor or a non-selective inhibitor.

For example, the phosphodiesterase inhibitor is a non-selective inhibitor. In several instances, the non-selective phosphodiesterase inhibitor includes caffeine (1,3,7-trimethylxanthine), theobromine (3,7-dimethyl-2,3,6,7-tetrahydro-1H-purine-2,6-dione), theophylline (1,3-dimethyl-7H-purine-2,6-dione), IBMX (3-isobutyl-1-methylxanthine), combinations thereof, or the like.

In another example, the phosphodiesterase inhibitor is a selective inhibitor. For instance, the selective phosphodiesterase inhibitor includes Milrinone (2-methyl-6-oxo-1,6-dihydro-3,4'-bipyridine-5-carbonitrile), Cilostazol (6-[4-(1-cyclohexyl-1H-tetrazol-5-yl)butoxy]-3,4-dihydro-2(1H)-quinolinone), Cilomilast (4-cyano-4-(3-cyclopentyloxy-4-methoxyphenyl)cyclohexane-1-carboxylic acid), Rolipram (4-(3-cyclopentyloxy-4-methoxyphenyl)pyrrolidin-2-one), Roflumilast (3-(cyclopropylmethoxy)-N-(3,5-dichloropyridin-4-yl)-4-(difluoromethoxy)benzamide), combinations thereof, or the like.

In some instances, the pharmaceutical composition comprises a co-crystal comprising an acid salt of a compound of Formula I, II, IIA, IIB, III, IIIA, IIIB, IVA, or IVB, and a phosphodiesterase inhibitor (e.g., caffeine, IBMX, or any combination thereof). For example, a co-crystal comprises an HCl salt of a compound of Formula I, II, IIA, IIB, III, IIIA, IIIB, IVA, or IVB, and a phosphodiesterase inhibitor. In another example, a co-crystal comprises an H₂SO₄ salt of a compound of Formula I, II, IIA, IIB, III, IIIA, IIIB, IVA, or IVB, and a phosphodiesterase inhibitor (e.g., caffeine, IBMX, or any combination thereof).

In several instances, the phosphodiesterase inhibitor is present in the co-crystal according to the ratio from about 1:1 to about 1:5 (e.g., 1:1, 1:2, 1:3, or 1:4) wherein the ratio represents the amount of phosphodiesterase inhibitor relative to the amount of compound of Formula I, i.e., amt of phosphodiesterase inhibitor : amt of compound of Formula I. Note that in some instances, the co-crystal also comprises method artifacts such as week acids that are used to facilitate crystal formation.

In one instance, the co-crystal comprises caffeine and a compound of Formula I, wherein the caffeine is present according to a ratio of from about 1:1.25 to about 1:1.75, wherein the ratio represents the amount of phosphodiesterase inhibitor relative to the amount of compound of Formula I. In one example, the co-crystal comprises caffeine and a compound of Formula I, wherein caffeine is present in according to the ratio 1:1.5, i.e., 40 %, relative to the compound of Formula I. In another example, the co-crystal comprises 5-(4-(2-(5-ethylpyridin-2-yl)-2-oxoethoxy)benzyl)-1,3-thiazolidine-2,4-dione and caffeine, wherein the caffeine is present according to the ratio from about 1:1.25 to about 1:1.75 (e.g., about 1:1.5) relative to 5-(4-(2-(5-ethylpyridin-2-yl)-2-oxoethoxy)benzyl)-1,3-thiazolidine-2,4-dione.

In other instances, the present invention provides a method of treating or preventing a neurodegenerative disorder in a patient comprising administering a pharmaceutical composition comprising a co-crystal comprising a compound of Formula II, IIA, IIB, III, IIIA, IIIB, IVA, or IVB, or a pharmaceutically acceptable salt thereof, and a phosphodiesterase inhibitor.

One instance of the present invention provides a method of treating or preventing a neurodegenerative disorder in a patient comprising administering to the patient a co-crystal comprising a compound selected from: 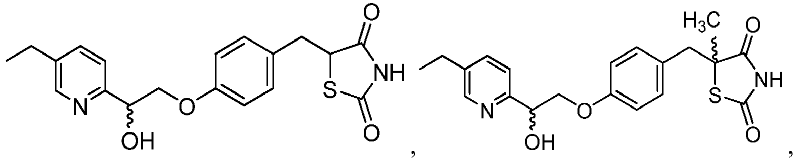 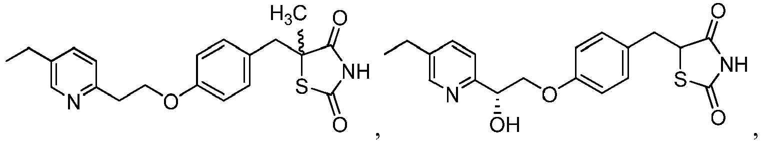 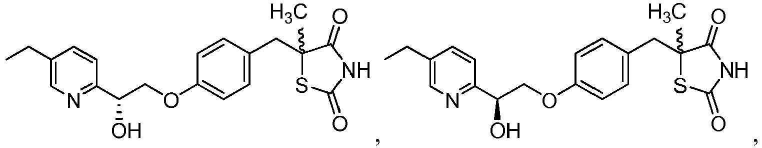 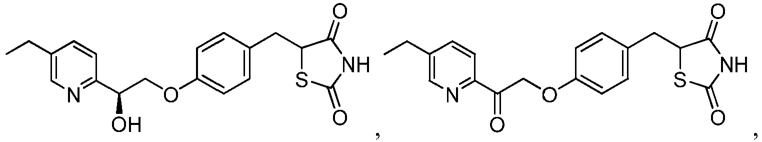 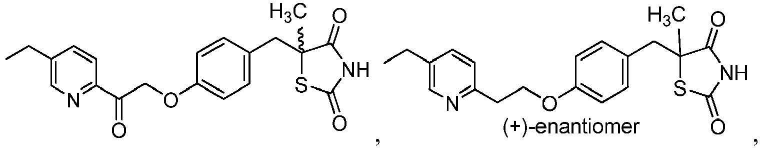 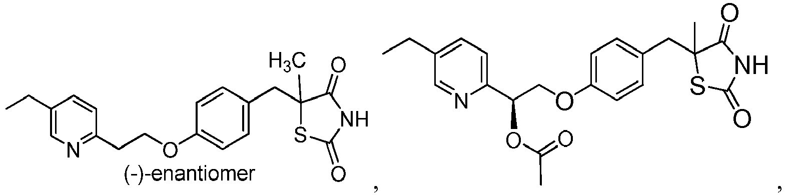 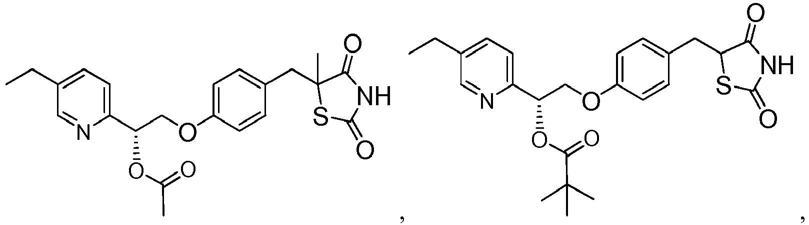 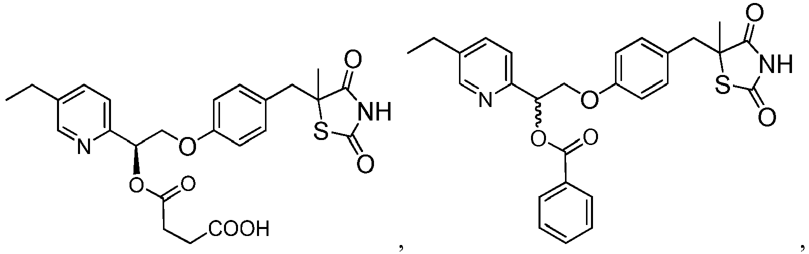 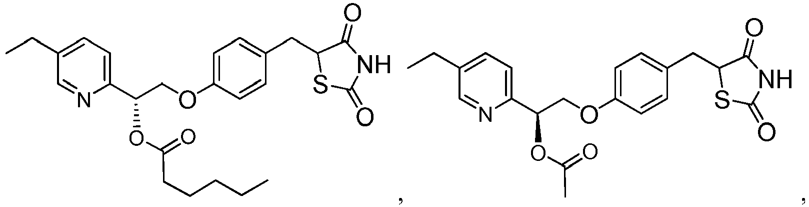 or 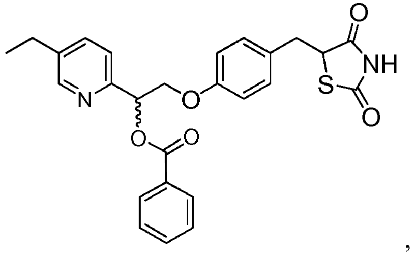 or a pharmaceutically acceptable salt thereof, and a phosphodiesterase inhibitor.

Also described herein is a method of treating or preventing a neurodegenerative disorder in a patient comprising administering to the patient a co-crystal comprising a compound selected from: 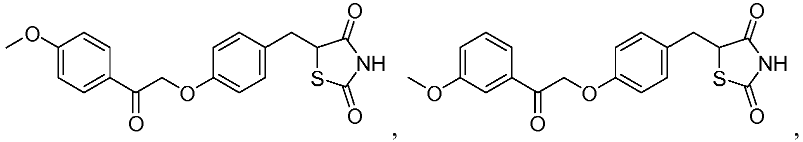 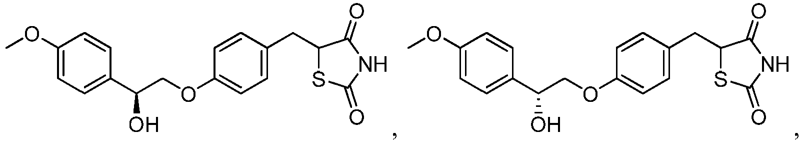 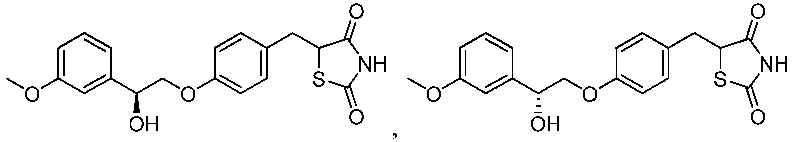 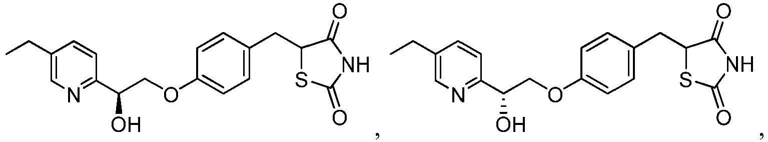 or 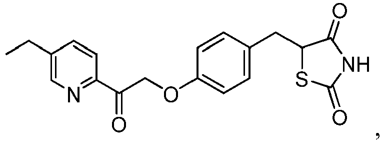 or a pharmaceutically acceptable salt thereof, and a phosphodiesterase inhibitor.

In several instances, the phosphodiesterase inhibitor is a selective inhibitor or a non-selective inhibitor.

For example, the phosphodiesterase inhibitor is a non-selective inhibitor. In several instances, the non-selective phosphodiesterase inhibitor includes caffeine (1,3,7-trimethylxanthine), theobromine (3,7-dimethyl-2,3,6,7-tetrahydro-1H-purine-2,6-dione), theophylline (1,3-dimethyl-7H-purine-2,6-dione), combinations thereof, and the like.

In another example, the phosphodiesterase inhibitor is a selective inhibitor. For instance, the selective phosphodiesterase inhibitor includes Milrinone (2-methyl-6-oxo-1,6-dihydro-3,4'-bipyridine-5-carbonitrile), Cilostazol (6-[4-(1-cyclohexyl-1H-tetrazol-5-yl)butoxy]-3,4-dihydro-2(1H)-quinolinone), Cilomilast (4-cyano-4-(3-cyclopentyloxy-4-methoxyphenyl)cyclohexane-1-carboxylic acid), Rolipram (4-(3-cyclopentyloxy-4-methoxyphenyl)pyrrolidin-2-one), Roflumilast (3-(cyclopropylmethoxy)-N-(3,5-dichloropyridin-4-yl)-4-(difluoromethoxy)benzamide), combinations thereof, and the like.

In other examples, the co-crystal comprises the compound 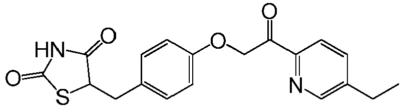 or a pharmaceutically acceptable salt thereof, and a phosphodiesterase inhibitor.

In other examples, the co-crystal comprises the compound 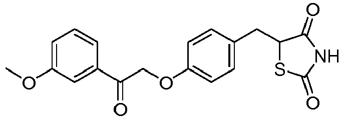 or a pharmaceutically acceptable salt thereof, and a phosphodiesterase inhibitor.

Further described herein is a pharmaceutical composition comprising a co-crystal, as described above, a second agent that increases the cyclic nucleotide in a patient, and a pharmaceutically acceptable carrier.

Agents that increase cAMP in a patient include, without limitation, β-adrenergic agonists, hormones (e.g., GLP1), any combination thereof, or the like.

Also described herein isa method of treating or preventing a neurodegenerative disorder in a patient comprising administering a pharmaceutical composition comprising a compound of Formula I, a salt thereof, or a co-crystal thereof, and a β-adrenergic agonist (e.g., a β1-adrenergic agonist, a β2-adrenergic agonist, a β3-adrenergic agonist, or any combination thereof). Non-limiting examples of β-adrenergic agonists include noradrenaline, isoprenaline, dobutamine, salbutamol, levosalbutamol, terbutaline, pirbuterol, procaterol, metaproterenol, fenoterol, bitolterol mesylate, salmeterol, formoterol, bambuterol, clenbuterol, indacaterol, L-796568, amibegron, solabegron, isoproterenol, albuterol, metaproterenol, arbutamine, befunolol, bromoacetylalprenololmenthane, broxaterol, cimaterol, cirazoline, denopamine, dopexamine, epinephrine, etilefrine, hexoprenaline, higenamine, isoetharine, isoxsuprine, mabuterol, methoxyphenamine, nylidrin, oxyfedrine, prenalterol, ractopamine, reproterol, rimiterol, ritodrine, tretoquinol, tulobuterol, xamoterol, zilpaterol, zinterol, or any combination thereof.

In other instances described herein, the method of treating or preventing a neurodegenerative disorder in a patient comprising administering to a patient a co-crystal comprising a compound of Formula I or a pharmaceutically acceptable salt thereof, and a phosphodiesterase inhibitor; and an agent that increases cAMP levels in a patient (e.g., β-adrenergic agonist or GLP1). For instance, the composition comprises a co-crystal comprising a compound of Formula II, IIA, IIB, III, IVA or IVB or a pharmaceutically acceptable salt thereof, and a phosphodiesterase inhibitor; and a β-adrenergic agonist. Any of the phosphodiesterase inhibitors or combinations thereof are suitable for use in co-crystals used to formulate pharmaceutical compositions described herein that also include one or more agents that increase cyclic nucleotide (e.g., cAMP) levels in a patient (e.g., a β-adrenergic agonist).

In one particular example, the pharmaceutical composition comprises the compound 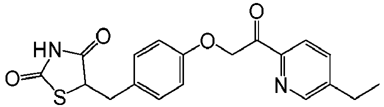 or a pharmaceutically acceptable salt thereof, and a phosphodiesterase inhibitor (e.g., caffeine and/or IBMX).

In another particular example, the pharmaceutical composition comprises the compound 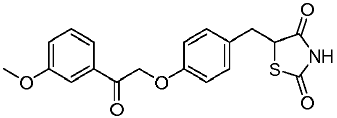 or a pharmaceutically acceptable salt thereof, and a phosphodiesterase inhibitor (e.g., caffeine and/or IBMX).

Some of these examples further comprise a β-adrenergic agonist, such as any of those described above.

### III. METHODS

Also described herein is a method of treating or preventing a neurodegenerative disorder in a patient comprising administering a pharmaceutical composition comprising a compound of Formula I, II, IIA, IIB, III, IIIA, IIIB, IVA, or IVB.

Several instances comprise the step of administering to a patient a compound of Formula I and a phosphodiesterase inhibitor. The administration of these ingredients can be sequential (e.g., the compound of Formula I is administered first in time, and the agent is administered second in time) or simultaneous, i.e., both ingredients are administered at substantially the same time.

Several instances comprise the step of administering to a patient a pharmaceutical composition comprising a co-crystal comprising a compound of Formula I or a pharmaceutically acceptable salt thereof, and a phosphodiesterase inhibitor. Some instances further comprise administering an agent that increases a cyclic nucleotide level in a patient (e.g., a β-adrenergic agonist).

Several methods comprise the step of administering to a patient a compound of Formula I and an agent that increases a cyclic nucleotide level in a patient.

Several methods comprise the step of administering to a patient a pharmaceutical composition comprising a co-crystal comprising a compound of Formula I or a pharmaceutically acceptable salt thereof, and a phosphodiesterase inhibitor; and an agent that increases a cyclic nucleotide level in a patient (e.g., a β-adrenergic agonist).

Further described herein is a method of treating and/or preventing a neurodegenerative disorder in a patient comprising administering a pharmaceutical composition comprising a compound of Formula I, II, IIA, IIB, III, IIIA, IIIB, IVA, or IVB wherein said compound has a purity of about 70 e.e.% or more. For example, the method treating or preventing a neurodegenerative comprises administering a pharmaceutical composition comprising a compound of Formula I and a phosphodiesterase inhibitor (e.g., caffeine and/or IBMX) wherein the compound of Formula I has a purity of about 80% e.e. or more (e.g., 90% e.e. or more, 95% e.e. or more, 97% e.e. or more, or 99% e.e. or more).

According to yet another instance, the present invention provides a method of treating or reducing the severity of Alzheimer's Disease, Parkinson's Disease, ALS, MS, MCI, or any combination thereof.

Also described herein is a method of treating or preventing a neurodegenerative disorder in a patient comprising administering to the patient a pharmaceutical composition comprising a compound of Formula I, II, IIA, IIB, III, IIIA, IIIB, IVA, or IVB, and a phosphodiesterase inhibitor (e.g., caffeine, IBMX, or any combination thereof).

Further described herein is a method of treating or preventing Alzheimer's Disease in a patient comprising administering to the patient a compound of Formula I, II, IIA, IIB, III, IIIA, IIIB, IVA, or IVB, or an alkali earth metal salt thereof. Some methods further comprise administering LDOPA to the patient. The LDOPA can be administered concurrently with the compound or compound salt, or the LDOPA can be administered before or after the administration of the compound or compound salt. In some instances, the patient is administered a pharmaceutical composition comprising a compound or compound salt of Formula I and LDOPA.

Described herein is also a method of treating or preventing a neurodegenerative disorder in a patient comprising administering to the patient a pharmaceutical composition comprising an salt of a compound of Formula I, II, IIA, IIB, III, IIIA, IIIB, IVA, or IVB, and a phosphodiesterase inhibitor (e.g., caffeine, IBMX, or any combination thereof). In some examples, the salt is a sodium salt of a compound of Formula I, II, IIA, IIB, III, IIIA, IIIB, IVA, or IVB, and in other examples, the salt is an potassium salt of a compound of a compound of Formula I, II, IIA, IIB, III, IIIA, IIIB, IVA, or IVB.

Further described herein is a method of treating or preventing a neurodegenerative disorder in a patient comprising administering to the patient a pharmaceutical composition comprising a compound of Formula I, II, IIA, IIB, III, IIIA, IIIB, IVA, or IVB, wherein the compound has a PPARγ activity of 50% or less relative to the activity of rosiglitazone when dosed to produce circulating levels greater than 3 µM or having a PPARγ activity of 10 times less than pioglitazone at the same dosage.

Also described herein is a method of treating or preventing a neurodegenerative disorder in a patient comprising administering to the patient a pharmaceutical composition comprising a compound of Formula I, a phosphodiesterase inhibitor, and a pharmaceutically acceptable carrier.

### IV. GENERAL SYNTHETIC SCHEMES

The compounds of Formula I, II, IIA, IIB, III, IIIA, IIIB, IVA, or IVB may be readily synthesized from commercially available or known starting materials by known methods. Exemplary synthetic routes to produce compounds of Formula I, II, IIA, IIB, III, IIIA, IIIB, IVA, or IVB are provided in Scheme 1 below.

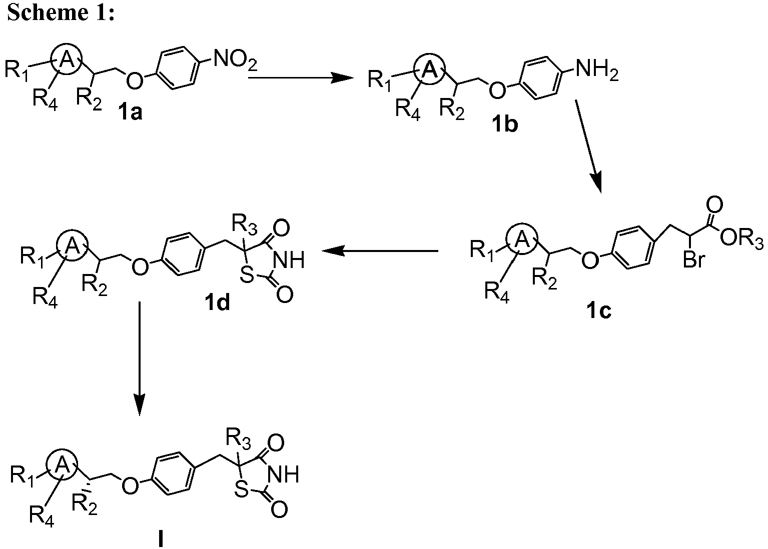

Referring to Scheme 1, the starting material **1a** is reduced to form the aniline **1b**. The aniline **1b** is diazotized in the presence of hydrobromic acid, acrylic acid ester, and a catalyst such as cuprous oxide to produce the alpha-bromo acid ester **1c**. The alpha-bromo acid ester **1c** is cyclized with thiourea to produce racemic thiazolidinedione **1d**. Compounds of Formula II can be separated from the racemic mixture using any suitable process such as HPLC.

In Scheme 2 below, R₂ and R'₂ form an oxo group or -O-Q and R₃ is hydrogen.

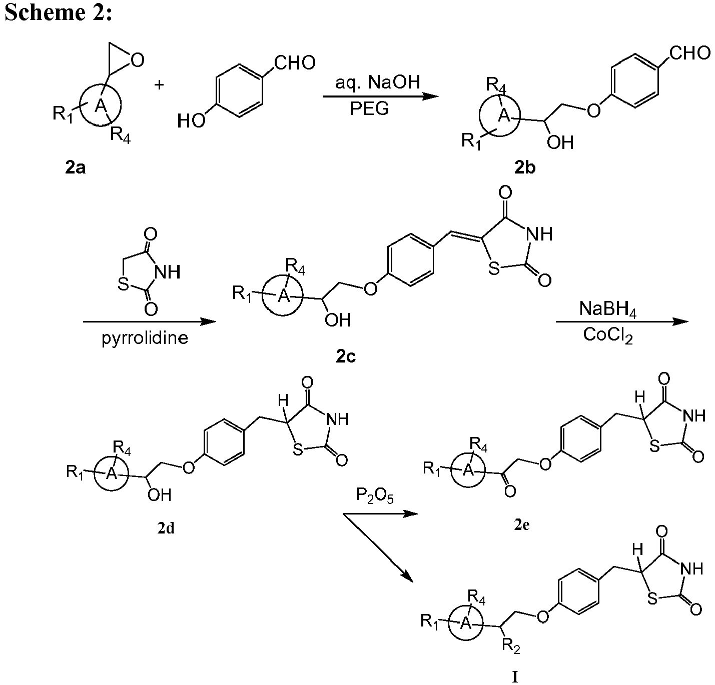

Referring to Scheme 2, the starting material **2a** is reacted with 4-hydroxybenzalde under basic conditions (e.g., aq. NaOH) to give a mixture of regioisomeric alcohols **2b** that were separated by chromatography. The regioisomeric alcohols **2b** is reacted with 2,4-thiazolidinedione using pyrrolidine as base to give compound **2c.** Cobalt catalyzed reduction with sodium borohydride affords compound **2d,** which is oxidized, for example, with phosphorus pentoxide in the presence of dimethyl sulfoxide, to give the ketone **2e**. Alternatively, compounds of Formula I wherein R₂ is -O-Q, may be prepared from the hydroxy compound **2d** using known methods of alkylation, acylation, sulfonation or phosphorylation.

### V. USES, FORMULATIONS, AND ADMINISTRATION

As discussed above, the present invention provides a sodium salt of Compound X 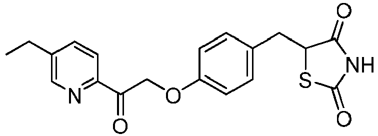 for use in treating Alzheimer's Disease. Also described herein are compounds that are useful as treatments for preventing or treating one or more neurodegenerative disorders in a patient.

Accordingly, described herein are pharmaceutically acceptable compositions, wherein these compositions comprise any of the compounds as described herein, and optionally comprise a pharmaceutically acceptable carrier, adjuvant or vehicle. In certain instances, these compositions optionally further comprise one or more additional therapeutic agents.

It will also be appreciated that certain of the compounds described herein can exist in free form for treatment, or where appropriate, as a pharmaceutically acceptable derivative or a prodrug thereof. According to the present invention, a pharmaceutically acceptable derivative or a prodrug includes, but is not limited to, pharmaceutically acceptable salts, esters, salts of such esters, or any other adduct or derivative which upon administration to a patient in need is capable of providing, directly or indirectly, a compound as otherwise described herein, or a metabolite or residue thereof.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. A "pharmaceutically acceptable salt" means any non-toxic salt or salt of an ester of the compound of this invention that, upon administration to a recipient, is capable of providing, either directly or indirectly, the compound of this invention or an inhibitorily active metabolite or residue thereof.

Pharmaceutically acceptable salts are well known in the art. For example, S. M. Berge, et al. describes pharmaceutically acceptable salts in detail in J. Pharmaceutical Sciences, 1977, 66, 1-19. Pharmaceutically acceptable salts of the compounds described hereininclude those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and N⁺(C₁₋₄alkyl)₄ salts. This invention also envisions the quaternization of any basic nitrogen-containing groups of the compounds disclosed herein. Water or oil-soluble or dispersible products may be obtained by such quaternization. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate.

As described above, the pharmaceutically acceptable compositions described herein additionally comprise a pharmaceutically acceptable carrier, adjuvant, or vehicle, which, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Sixteenth Edition, E. W. Martin (Mack Publishing Co., Easton, Pa., 1980) discloses various carriers used in formulating pharmaceutically acceptable compositions and known techniques for the preparation thereof. Except insofar as any conventional carrier medium is incompatible with the compounds described herein, such as by producing any undesirable biological effect or otherwise interacting in a deleterious manner with any other component(s) of the pharmaceutically acceptable composition, its use is contemplated to be within the scope of this invention. Some examples of materials which can serve as pharmaceutically acceptable carriers include, but are not limited to, ion exchangers, alumina, aluminum stearate, lecithin, serum proteins, such as human serum albumin, buffer substances such as phosphates, glycine, sorbic acid, or potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, polyacrylates, waxes, polyethylene-polyoxypropylene-block polymers, wool fat, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol or polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

According to the invention an "effective amount" of the compound or pharmaceutically acceptable composition is that amount effective for treating, preventing, or lessening the severity of metabolic diseases such as neurodegenerative disorders, e.g., Alzheimer's Disease, Parkinson's Disease, ALS, MS, MCI, any combination thereof, or the like.

The pharmaceutical compositions described herein may be administered using any amount and any route of administration effective for treating or lessening the severity of neurodegenerative disorders.

The exact amount required will vary from subject to subject, depending on the species, age, and general condition of the subject, the particular agent, its mode of administration, and the like. The compound of the invention is preferably formulated in dosage unit form for ease of administration and uniformity of dosage. The expression "dosage unit form" as used herein refers to a physically discrete unit of agent appropriate for the patient to be treated. It will be understood, however, that the total daily usage of the compound and compositions of the present invention will be decided by the attending physician within the scope of sound medical judgment. The specific effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed, and like factors known in the medical arts. The term "patient", as used herein, means an animal, for example, a mammal, and more specifically a human.

The pharmaceutically acceptable compositions described herein can be administered to humans and other animals orally, rectally, parenterally, intracisternally, intravaginally, intraperitoneally, topically (as by powders, ointments, or drops), bucally, as an oral or nasal spray, or the like, depending on the severity of the infection being treated. In certain instances, the compound of the invention may be administered orally or parenterally at dosage levels of about 0.01 mg/kg to about 50 mg/kg and preferably from about 1 mg/kg to about 25 mg/kg, of subject body weight per day, one or more times a day, to obtain the desired therapeutic effect. Alternatively, the compound of the invention may be administered orally or parenterally at dosage levels of between 10 mg/kg and about 120 mg/kg.

Liquid dosage forms for oral administration include, but are not limited to, pharmaceutically acceptable emulsions, microemulsions, solutions, suspensions, syrups and elixirs. In addition to the active compound, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of the compound of the present invention, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of the parenterally administered compound form is accomplished by dissolving or suspending the compound in an oil vehicle. Injectable depot forms are made by forming microencapsulated matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing the compound of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polyethylene glycols and the like.

The active compound can also be in microencapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of the compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, eardrops, and eye drops are also contemplated as being within the scope of this invention. Additionally, the present invention contemplates the use of transdermal patches, which have the added advantage of providing controlled delivery of the compound to the body. Such dosage forms are prepared by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

As described generally above, the compounds described herein are useful as treatments for metabolic diseases.

The activity, or more importantly, reduced PPARγ activity of a compound described herein as a treatment or prevention of neurodegenerative disorders may be assayed according to methods described generally in the art and in the examples provided herein.

It will also be appreciated that the compounds and pharmaceutically acceptable compositions described herein can be employed in combination therapies, that is, the compounds and pharmaceutically acceptable compositions can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, a compound described herein may be administered concurrently with another agent used to treat the same disorder), or they may achieve different effects (e.g., control of any adverse effects). As used herein, additional therapeutic agents that are normally administered to treat or prevent a particular disease, or condition, are known as "appropriate for the disease, or condition, being treated".

The amount of additional therapeutic agent present in the compositions described herein will be no more than the amount that would normally be administered in a composition comprising that therapeutic agent as the only active agent. Preferably the amount of additional therapeutic agent in the presently described compositions will range from about 50% to 100% of the amount normally present in a composition comprising that agent as the only therapeutically active agent.

The compounds described herein or pharmaceutically acceptable compositions thereof may also be incorporated into compositions for coating an implantable medical device, such as prostheses, artificial valves, vascular grafts, stents and catheters. Accordingly, described herein is a composition for coating an implantable device comprising the compound of the present invention as described generally above, and in classes and subclasses herein, and a carrier suitable for coating said implantable device. Also described herein is an implantable device coated with a composition comprising the compound of the present invention as described generally above, and in classes and subclasses herein, and a carrier suitable for coating said implantable device. Suitable coatings and the general preparation of coated implantable devices are described in US Patents 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccarides, polyethylene glycol, phospholipids or combinations thereof to impart controlled release characteristics in the composition.

Another aspect described herein relates to treating metabolic diseases in a biological sample or a patient (e.g., in vitro or in vivo), which method comprises administering to the patient, or contacting said biological sample with a pharmaceutical composition comprising a compound of Formula I, II, IIA, IIB, III, IIIA, IIIB, IVA or IVB. The term "biological sample", as used herein, includes, without limitation, cell cultures or extracts thereof; biopsied material obtained from a mammal or extracts thereof; and blood, saliva, urine, feces, semen, tears, or other body fluids or extracts thereof.

In order that the invention described herein may be more fully understood, the following examples are set forth. It should be understood that these examples are for illustrative purposes only and are not to be construed as limiting this invention in any manner.

### VI. EXAMPLES

In the text below, compounds marked with an asterisk (*) are comparative compounds.

### Example 1: 5-[4-(2-oxo-2-phenylethoxy)benzyl]-1,3-thiazolidine-2,4-dione (*).

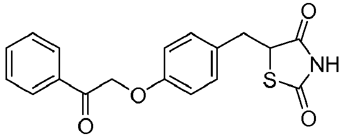

### Step 1: Preparation of 4-(2-hydroxy-2-phenylethoxy)benzaldehyde.

To 2-(4-fluorophenyl)oxirane (6.50 g, 54.0 mmol) was added toluene (85 mL), 4-hydroxybenzaldehyde (9.89 g, 81.0 mmol), PEG4000 (polyethylene glycol, 1.15 g) and 1M NaOH (85 mL) and the stirring mixture was heated at 78 °C overnight. After cooling to RT the reaction mixture was extracted with EtOAc, and the organic phase was washed with brine, dried (Na₂SO₄), filtered and evaporated in vacuo. The resulting yellow oil was chromatographed on a medium silica gel column eluting with 0-10% EtOAc/DCM. Fractions containing predominantly the higher R_{f} spot were combined and evaporated in vacuo to give 1.85g (14%) of the title compound as a yellow oil. Fractions containing predominantly the lower R_{f} spot were combined and evaporated in vacuo to give 0.64g of the regioisomer as a colorless, viscous oil. Mixed fractions were combined and rechromatographed eluting with 30% EtOAc/hexanes. Fractions containing the higher R_{f} material were combined and evaporated in vacuo to give an additional 2.64 g (20%) of the title compound as a colorless oil. Fractions containing the lower R_{f} material were combined and evaporated in vacuo to give an additional 1.82 g of the regioisomer as a colorless viscous oil.

### Step 2: Preparation of 5-[4-(2-hydroxy-2-phenylethoxy)benzylidene]-1,3-thiazolidine-2,4-dione.

To a stirring solution of 4-[(2S)-2-hydroxy-2-phenylethoxy]benzaldehyde (2.63 g, 10.8 mmol) in absolute EtOH (75 mL) was added 2,4-thiazolidinedione (1.27 g, 10.8 mmol) and piperidine (0.54 mL, 5.4 mmol), and the resulting solution was heated to reflux. The reaction was refluxed overnight. The reaction mixture was allowed to cool to RT. No precipitate formed. The pH of reaction mixture was ca. 5. Acetic acid (20 drops) was added, and the reaction was evaporated in vacuo. The material was adsorbed onto silica gel and chromatographed eluting with 30-40% EtOAc/hexanes. Fractions containing product were combined and evaporated in vacuo to give 3.18g (86%) of the title compound as a light yellow solid. MS (ESI-) for C₁₈H₁₅NO₄S m/z 340.1 (M-H)⁻.

### Step 3: Preparation of 5-[4-(2-hydroxy-2-phenylethoxy)benzyl]-1,3-thiazolidine-2,4-dione.

To a mixture of 5-[4-(2-hydroxy-2-phenylethoxy)benzylidene]-1,3-thiazolidine-2,4-dione (1.50 g, 4.39 mmol) in THF (20 mL) was added H₂O (20 mL), 1M NaOH (3 mL), cobalt (II) chloride hexahydrate (0.60 mg, 0.003 mmol) and dimethylglyoxime (15 mg, 0.13 mmol). A solution of sodium tetrahydroborate (240 mg, 6.33 mmol) in 0.2M NaOH (3.6 mL) was added. The reaction mixture immediately turned dark but very soon assumed a clear yellow appearance. Acetic acid was added dropwise until the solution turned dark (3 drops). After ca. one hour, the reaction lightened. Additional NaBH₄, CoCl₂ and HOAc were added to produce a deep blue-purple color. When that color faded, more NaBH₄ was added. When HPLC analysis indicated that the reaction was complete, it was partitioned between H₂O and EtOAc, and the organic phase was washed with brine, dried (Na₂SO₄), filtered and evaporated in vacuo. The resulting foamy solid was chromatographed, eluting with 50% EtOAc/hexanes. Fractions containing product were combined and evaporated in vacuo to give 1.15 g (76%) of the title compound as a white solid. MS (ESI-) for C₁₈H₁₇NO₄S m/z 342.1 (M-H)⁻.

### Step 4: Preparation of 5-[4-(2-oxo-2-phenylethoxy)benzyl]-1,3-thiazolidine-2,4-dione.

To a stirring solution of 5-[4-(2-hydroxy-2-phenylethoxy)benzyl]-1,3-thiazolidine-2,4-dione (1.00 g, 2.91 mmol) in DCM (35 mL) was added DMSO (2 mL) and the solution was cooled to 0 °C. Phosphorus pentoxide (0.83 g, 2.91 mmol) was added followed by triethylamine (1.8 mL, 13.1 mmol). The reaction was allowed to slowly warm to RT. After 2 hours, the reaction mixture was partitioned between DCM and water and the organic phase was washed with brine, dried (Na₂SO₄), filtered and evaporated in vacuo. The resulting yellow oil was chromatographed on silica gel eluting with 25-35% EtOAc/hexanes. Fractions containing product were combined and evaporated in vacuo to give 0.40 g (40%) of the title compound as a white solid. Trituration with ether afforded 245 mg of clean product. MS (ESI-) for C₁₈H₁₅NO₄S m/z 340.1 (M-H)⁻.

### Example 2: Preparation of 5-{4-[2-(4-fluorophenyl)-2-oxoethoxy]benzyl}-1,3-thiazolidine-2,4-dione (*).

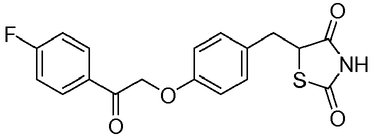

### Step 1: Preparation of 4-[2-(fluorophenyl)-2-hydroxyethoxy]benzaldehyde.

To a stirring solution of 2-(4-fluorophenyl)oxirane (5.60 g, 40.0 mmol) in toluene (65 mL) was added 4-hydroxybenzaldehyde (7.40 g, 61.0 mmol), 1M NaOH (65 mL) and PEG4000 (polyethylene glycol, 0.85 g) and the reaction was heated at 78 °C overnight. After cooling to RT, the reaction was extracted with EtOAc (2 x 150 mL) and the combined extracts were washed with brine, dried (Na₂SO₄), filtered and evaporated in vacuo. The resulting light brown oil was chromatographed on silica gel eluting with 30-40% EtOAc/hexanes. Fractions containing the higher R_{f} spot were combined and evaporated in vacuo to give 2.38 g of the regioisomer of the product as a white solid. Fractions containing the lower Rf spot were combined and evaporated in vacuo to give 1.54g (22%) of the title compound as a colorless viscous oil.

### Step 2: Preparation of 5-{4-[2-(4-fluorophenyl)-2-hydroxyethoxy]benzylidene}-1,3-thiazolidine-2,4-dione.

To a stirring solution of the aldehyde (2.36 g, 10.8 mmol) in absolute EtOH (75 mL) was added 2,4-thiazolidinedione (1.06 g, 9.07 mmol) and piperidine (0.45 mL, 4.50 mmol), and the resulting solution was heated to reflux. After refluxing overnight, the reaction was allowed to cool to RT, and then evaporated in vacuo. The residue was adsorbed onto silica gel and chromatographed, eluting with 30-40% EtOAc/hexanes. Fractions containing product were combined and evaporated in vacuo to give 0.88 g (27%) of the title compound as a yellow solid. MS (ESI-) for C₁₈H₁₄FNO₄S m/z 358.1 (M-H)⁻.

### Step 3: Preparation of 5-{4-[2-(4-fluorophenyl)-2-hydroxyethoxy]benzyl}-1,3-thiazolidine-2,4-dione.

To a stirring mixture of 5-{4-[2-(4-fluorophenyl)-2-hydroxyethoxy]benzylidene}-1,3-thiazolidine-2,4-dione (0.87 g, 2.40 mmol) in THF/H₂O (1:1, 20 mL) was added 1M NaOH (2 mL), cobalt (II) chloride hexahydrate (0.30 g, 0.001 mmol), dimethylglyoxime (8.4 mg, 0.073 mmol), and finally sodium tetrahydroborate (0.13 g, 3.53 mmol). The reaction turned a deep blue/purple color. After a short time, the dark color began to fade and HOAc was added dropwise to regenerate the darker color. When the color faded and addition of HOAc failed to regenerate it, NaBH₄ was added to regenerate the darker color. The reaction was left to stir at RT overnight. The reaction was partitioned between water and EtOAc. The organic phase was washed with brine, dried (Na₂SO₄), filtered and evaporated in vacuo. The resulting light brown oil was chromatographed, eluting with 35% EtOAc/hexanes. Fractions containing compound were combined and evaporated in vacuo to give 0.77 g (88%) of a light yellow solid. The yellow solid was dissolved in THF (8 mL) and H₂O (8 mL), and the resulting solution was treated with CoCl₂ (a small crystal), and 2,2'-dipyridyl (5 mg). Finally, NaBH₄ was added in small portions until the deep blue color persisted. The reaction mixture was partitioned between EtOAc and H₂O, and the aqueous phase was extracted with EtOAc. The combined organic phases were washed with brine, dried (Na₂SO₄), filtered and evaporated in vacuo. The resulting slightly tinted oil was chromatographed on a small silica gel column eluting with 25-35% EtOAc/hexanes. Fractions containing product were combined and evaporated in vacuo to afford 527 mg (60%) of the title compound as a white solid. MS (ESI-) for C₁₈H₁₆FNO₄S m/z 360.1 (M-H)⁻.

### Step 4: Preparation of 5-{4-[2-(4-fluorophenyl)-2-oxoethoxy]benzyl}-1,3-thiazolidine-2,4-dione.

To a stirring solution of 5-{4-[2-(4-fluorophenyl)-2-hydroxyethoxy]benzyl}-1,3-thiazolidine-2,4-dione (0.52 g, 1.40 mmol) in DCM (15 mL) was added DMSO (0.5 mL) and the solution was cooled to 0 °C. Phosphorus pentoxide (0.41g, 1.44 mmol) was added followed by triethylamine (0.90 mL, 6.48 mmol). The reaction was allowed to slowly warm to RT and then stirred for 5 hours. The reaction mixture was partitioned between DCM and H₂O, and the aqueous phase was extracted with DCM. The combined organic phases were washed with brine, dried (Na₂SO₄), filtered and evaporated in vacuo. The resulting white solid was chromatographed on a small silica gel column eluting with 10% EtOAc/DCM. Fractions containing product were combined and evaporated in vacuo to give 0.25 g (48%) of the title compound as a white solid. MS (ESI+) for C₁₈H₁₄FNO₄S m/z 359.9 (M+H)⁺. MS (ESI-) for C₁₈H₁₄FNO₄S m/z 358.0 (M-H)⁻.

### Example 3: Preparation of 5-{4-[2-(2-fluorophenyl)-2-oxoethoxy]benzyl}-1,3-thiazolidine-2,4-dione (*).

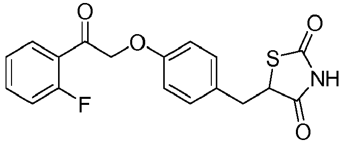

### Step 1: Preparation of 2-(2-fluorophenyl)oxirane.

To a solution of o-fluorostyrene (5.0 g, 41.0 mmol) and acetic acid (2.33 mL, 40.9 mmol) in dioxane (33 mL) and H₂O (78 mL) at 0 °C was added N-bromosuccinimide (8.02 g, 45.0 mol) in three portions. The reaction was allowed to warm to RT and stirred overnight. Sodium carbonate (8.68 g, 81.9 mmol) was added in portions and then 1M NaOH (ca. 10 mL) was added and the reaction was stirred at RT overnight. The reaction mixture was partitioned between water and EtOAc, and the aqueous phase was extracted with EtOAc. The combined organic phases washed with brine, dried (Na₂SO₄), filtered and evaporated in vacuo to give 5.31 g (94%) of the title compound as a slightly tinted oil which was used without further purification. MS (ESI+) for C₈H₇FO m/z 138.1 (M+H)⁺.

### Step 2: Preparation of 4-[2-(2-fluorophenyl)-2-hydroxyethoxy]benzaldehyde.

To a stirring solution of 2-(2-fluorophenyl)oxirane (5.30 g, 38.4 mmol) in toluene (65 mL) was added 4-hydroxybenzaldehyde (7.0 g, 58.0 mmol), 1M NaOH (65 mL) and PEG4000 (polyethylene glycol, 0.85 g) and the stirring mixture was heated at 78 °C overnight. The reaction was allowed to cool to RT and then extracted with EtOAc (2 x 150 mL). The combined extracts were washed with brine, dried (Na₂SO₄), filtered and evaporated in vacuo. The resulting light brown oil was adsorbed onto silica gel and chromatographed, eluting with 30-40% EtOAc/hexanes to give 2 major spots. Fractions containing the higher Rf spot were combined and evaporated in vacuo to give 1.10g (11%) of the title compound as a colorless oil. Fractions containing the lower R_{f} spot were combined and evaporated in vacuo to give 0.67g (7%) of the regioisomer as a colorless oil.

### Step 3: Preparation of 5-{4-[2-(2-fluorophenyl)- 2-hydroxyethoxy]benzylidene}-1,3-thiazolidine-2,4-dione.

To a stirring solution of the aldehyde (2.36 g, 10.8 mmol) in absolute EtOH (40 mL) was added 2,4-thiazolidinedione (0.495 g, 4.23 mmol) and piperidine (0.21 mL, 2.10 mmol), and the resulting solution was heated to reflux. After refluxing overnight, the reaction mixture was cooled to RT and then evaporated in vacuo. The residue was dissolved in EtOAc and this solution was washed with dilute aqueous HOAc, brine, dried (Na₂SO₄), filtered and evaporated in vacuo. The resulting yellow solid was washed with DCM and acetone and the filtrate was evaporated in vacuo. This material was adsorbed onto silica gel and chromatographed using 10-25% EtOAc/DCM. Fractions containing compound were combined and evaporated in vacuo to give 0.51g of the title compound as a yellow solid. MS (ESI-) for C₁₈H₁₄FNO₄S m/z 358.0 (M-H)⁻.

### Step 4: Preparation of 5-{4-[2-(2-fluorophenyl)- 2-hydroxyethoxy]benzyl}- 1,3-thiazolidine-2,4-dione.

To a stirring mixture of 5-{4-[2-(2-fluorophenyl)-2-hydroxyethoxy]benzylidene}-1,3-thiazolidine-2,4-dione (0.52 g, 1.40 mmol) in THF/H₂O (1:1, 16 mL) was added 1M NaOH (2 mL), cobalt (II) chloride hexahydrate (0.2 mg, 0.0009 mmol), 2,2'-bipyridine (50.8 mg, 0.33 mmol), and finally sodium tetrahydroborate (0.11 g, 2.90 mmol). The reaction turned a deep blue/purple color. After a short time, the dark color began to fade and HOAc was added dropwise to regenerate the darker color. When the color faded and addition of HOAc failed to regenerate it, NaBH₄ was added to regenerate the darker color. Added small portions of NaBH₄ and HOAc dropwise until deep blue color persisted. After repeating this several times, HPLC indicated that the reaction was complete despite the fact that the deep blue color has given way to a light brown solution. The reaction was partitioned between water and EtOAc. The organic phase was washed with brine, dried (Na₂SO₄), filtered and evaporated in vacuo. The resulting light brown oil was chromatographed, eluting with 35% EtOAc/hexanes. Fractions containing compound were combined and evaporated in vacuo to give 0.32 g of the title compound as a white solid. MS (ESI-) for C₁₈H₁₆FNO₄S m/z 360.1 (M-H)⁻.

### Step 5: Preparation of 5-{4-[2-(2-fluorophenyl)-2-oxoethoxy]benzyl}-1,3-thiazolidine-2,4-dione.

To a stirring solution of 5-{4-[2-(2-fluorophenyl)-2-hydroxyethoxy]benzyl}-1,3-thiazolidine-2,4-dione (0.29 g, 0.80 mmol) in DCM (15 mL) was added DMSO (0.5 mL) and the solution was cooled to 0 °C. Phosphorus pentoxide (0.23 g, 0.80 mmol) was added, followed by triethylamine (0.50 mL, 3.6 mmol). The reaction was allowed to slowly warm to RT. After 3 hours, water was added and the phases were separated. The pH of the aqueous phase was adjusted to ca. 7 and the aqueous phase was extracted with DCM. The combined organic phases were washed with brine, dried (Na₂SO₄), filtered and evaporated in vacuo. The resulting white solid was chromatographed on a small silica gel column eluting with 10% EtOAc/DCM. Fractions containing product were combined and evaporated in vacuo to give 0.19 g (66%) of the title compound as a white solid. MS (ESI-) for C₁₈H₁₄FNO₄S m/z 358.0 (M-H)⁻.

### Example 4: Preparation of 5-{4-[2-(3-fluorophenyl)-2-oxoethoxy]benzyl}-1,3-thiazolidine-2,4-dione (*).

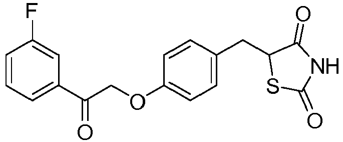

### Step 1: Preparation of 2-(3-fluorophenyl)oxirane.

To a solution of m-fluorostyrene (5.00 g, 41.0 mmol) and acetic acid (2.33 mL, 40.9 mmol) in dioxane (33 mL) and H₂O (78 mL) at 0 °C was added N-bromosuccinimide (8.02 g, 45.0 mmol) in three portions. The reaction was allowed to warm to RT. After 4 hours, 2N NaOH (60 mL) was added and the reaction was left to stir at RT overnight. The reaction mixture was partitioned between water and EtOAc, and the aqueous phase was extracted with EtOAc. The combined organic phases were washed with brine, dried (Na₂SO₄), filtered and evaporated in vacuo to give 6.30 g of the title compound as a slightly tinted oil which was used without further purification.

### Step 2: Preparation of 4-[2-(3-fluorophenyl)-2-hydroxyethoxy]benzaldehyde.

To a stirring solution of 2-(3-fluorophenyl)oxirane (5.60 g, 40.5 mmol) in toluene (65 mL) was added 4-hydroxybenzaldehyde (7.40 g, 61.0 mmol), 1M NaOH (65 mL) and PEG4000 (polyethylene glycol, 0.85 g) and the stirring mixture was heated at 78 °C overnight. The reaction mixture was allowed to cool to RT and then extracted with EtOAc (2 x 150 mL). The combined extracts were washed with brine, dried (Na₂SO₄), filtered and evaporated in vacuo. The resulting light brown oil was chromatographed eluting with 30-40% EtOAc/hexanes to give 2 major spots. Fractions containing the higher R_{f} spot were combined and evaporated in vacuo to give 1.78 g (17%) of the title compound as a white solid. Fractions containing the lower R_{f} spot were combined and evaporated in vacuo to give 0.90 g (9%) of the regioisomer as a nearly colorless oil.

### Step 3: Preparation of 5-{4-[2-(3-fluorophenyl)- 2-hydroxyethoxy]benzylidene}-1,3-thiazolidine-2,4-dione.

To a stirring solution of the aldehyde (2.36 g, 10.8 mmol) in absolute EtOH (40 mL) was added 2,4-thiazolidinedione (0.90 g, 7.69 mmol) and piperidine (0.76 mL, 7.7 mmol), and the resulting solution was heated to reflux. After 6 hours, the reaction mixture was allowed to cool to RT. The mixture was evaporated in vacuo and the residue was dissolved in EtOAc. This solution was washed with a dilute aqueous HOAc, brine, dried (Na₂SO₄), filtered and evaporated in vacuo. The resulting yellow solid was dissolved in MeOH/DCM adsorbed onto silica gel and chromatographed eluting with 30% EtOAc/DCM. Fractions containing compound were combined and evaporated in vacuo to afford 2.17 g (86%) of the title compound as a yellow solid. MS (ESI-) for C₁₈H₁₄FNO₄S m/z 358.1 (M-H)⁻.

### Step 4: Preparation of 5-{4-[2-(3-fluorophenyl)-2-hydroxyethoxy]benzyl}-1,3-thiazolidine-2,4-dione.

5-{4-[2-(3-fluorophenyl)-2-hydroxyethoxy]benzylidene}-1,3-thiazolidine-2,4-dione (1.00 g, 2.78 mmol) was suspended in THF (15 mL) and H₂O (10 mL). To this solution was added a small crystal of cobalt chloride followed by 2,2'-bipyridine (98 mg, 0.63 mmol). NaBH₄ was added in portions until blue color persisted. The color gradually faded and was regenerated repeatedly by small additions of boro hydride and HOAc. When HPLC analysis indicated that the reaction was complete, the reaction mixture was partitioned between EtOAc and H₂O. HOAc was added until the pH of the aqueous phase was ca. 6. The aqueous phase was extracted with EtOAc. The combined organic phases were washed with brine, dried (Na₂SO₄), filtered and evaporated in vacuo. The residue was chromatographed on a small silica gel column eluting with 20% EtOAc/DCM. Fractions containing product were combined and evaporated in vacuo to give 0.72 g (72%) of the title compound as a white solid. This material was rechromatographed on a small silica column eluting with 10-20% EtOAc/DCM. MS (ESI-) for C₁₈H₁₆FNO₄S m/z 360.1 (M-H)⁻.

### Step 5: Preparation of 5-{4-[2-(3-fluorophenyl)-2-oxoethoxy]benzyl}-1,3-thiazolidine-2,4-dione.

To a stirring solution of 5-{4-[2-(3-fluorophenyl)-2-hydroxyethoxy]benzyl}-1,3-thiazolidine-2,4-dione (0.62 g, 1.70 mmol) in DCM (15 mL) was added DMSO (0.5 mL) and the solution was cooled to 0 °C. Added phosphorus pentoxide (0.49 g, 1.72 mmol) followed by triethylamine (1.1 mL, 7.72 mmol). The reaction mixture was allowed to slowly warm to RT. After 2 hours, HPLC shows that the reaction was complete. Added water and separated phases. The pH of the aqueous phase was adjusted to ca. 7 with 2M NaOH and the aqueous phase was then extracted with EtOAc. The combined extracts were washed with brine, dried (Na₂SO₄), filtered and evaporated in vacuo. The resulting white solid was chromatographed on a small silica gel column eluting with 10% EtOAc/DCM. Fractions containing product were combined and evaporated in vacuo to give 0.25g (40%) of the title compound as a white solid. MS (ESI-) for C₁₈H₁₄FNO₄S m/z 358.0 (M-H)⁻.

### Example 5: Preparation of 5-{4-[2-(3-methoxyphenyl) -2-oxoethoxy]benzyl} -1,3 -thiazolidine-2,4-dione (*).

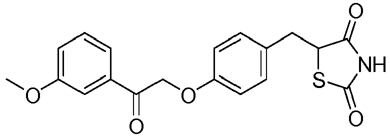

### Step 1: 2-(3-methoxyphenyl)oxirane.

To a solution of 3-vinylanisole (5.0 g, 37.0 mmol) and acetic acid (2.1 mL, 37.0 mmol) in dioxane (33 mL) and H₂O (78 mL) at 0 °C was added N-bromosuccinimide (7.30 g, 41.0 mmol) in three portions. The reaction was allowed to warm to RT and then 2M NaOH (50 mL) was added. The reaction was left to stir at RT overnight. The reaction mixture was then partitioned between water and EtOAc, and the aqueous phase was extracted with EtOAc. The combined organic phases washed with brine, dried (Na₂SO₄), filtered and evaporated in vacuo to give 5.60 g (100%) of the title compound as a slightly tinted oil.

### Step 2: 4-[2-hydroxy-2-(3-methoxyphenyl)ethoxy]benzaldehyde.

To a stirring solution of 2-(3-methoxyphenyl)oxirane (5.60 g, 37.0 mmol) in toluene (65 mL) was added 4-hydroxybenzaldehyde (6.80 g, 5.60 mmol), 1M NaOH (65 mL) and PEG4000 (polyethylene glycol, 0.85 g) and the stirring mixture was heated at 78 °C overnight. The reaction mixture was allowed to cool to RT and extracted with EtOAc (2 x 150 mL). The combined extracts were washed with brine, dried (Na₂SO₄), filtered and evaporated in vacuo. The resulting light brown oil was chromatographed, eluting with 30-40% EtOAc/hexanes. Fractions containing the higher R_{f} spot were combined and evaporated in vacuo to give 1.86 g (18%) of the title compound as a clear colorless oil. Fractions containing the lower R_{f} spot were combined and evaporated in vacuo to give 0.90 g (9%) the regioisomer as a nearly colorless oil.

### Step 3: 5-{4-[2-hydroxy-2-(3-methoxyphenyl)ethoxy]benzylidene}-1,3-thiazolidine-2,4-dione.

To a stirring solution of 4-[2-hydroxy-2-(3-methoxyphenyl)ethoxy]benzaldehyde (1.76 g, 6.46 mmol) in absolute EtOH (50 mL) was added 2,4-thiazolidinedione (0.83 g, 7.11 mmol) and piperidine (0.70 mL, 7.11 mmol), and the resulting solution was heated to reflux. The reaction was refluxed overnight and then evaporated in vacuo. The residue was dissolved in EtOAc and this solution was washed with water (pH adjusted to ca. 5-6 with HOAc), brine, dried (Na₂SO₄), filtered and adsorbed onto silica gel. After chromatography with 20-30% EtOAc/DCM, the fractions containing compound were combined and evaporated in vacuo to give 1.38 g (58%) of the title compound as a yellow solid. MS (ESI-) for C₁₉H₁₇NO₅S m/z 370.1 (M-H)⁻.

### Step 4: 5-{4-[2-hydroxy-2-(3-methoxyphenyl)ethoxy]benzyl} -1,3-thiazolidine-2,4-dione.

5-{4-[2-hydroxy-2-(3-methoxyphenyl)ethoxy]benzylidene}-1,3-thiazolidine-2,4-dione (1.15 g, 3.10 mmol) was dissolved in THF (15 mL). Added H₂O (15 mL) and sufficient THF to give a clear solution. A small crystal of cobalt chloride was added, followed by 2,2'-bipyridine (109 mg, 0.70 mmol). NaBH₄ was added in portions until the blue color persisted. The color gradually faded, but was regenerated repeatedly by small additions of borohydride and HOAc. When HPLC indicated that the reaction was complete the reaction mixture was partitioned between EtOAc and H₂O. HOAc was added until the pH of the aqueous phase was ca. 6, and then the aqueous phase was extracted with EtOAc. The combined organic phases were washed with brine, dried (Na₂SO₄), filtered and evaporated in vacuo. The residue was chromatographed on a small silica gel column eluting with 20% EtOAc/DCM. Fractions containing product were combined and evaporated in vacuo to give 0.82 g (74%) of the title compound as a white solid. MS (ESI-) for C₁₉H₁₉NO₅S m/z 372.0 (M-H)⁻.

### Step 5: Preparation of 5-{4-[2-(3-methoxyphenyl)-2-oxoethoxy]benzyl}-1,3-thiazolidine-2,4-dione.

To a stirring solution of 5-{4-[2-hydroxy-2-(3-methoxyphenyl)ethoxy]benzyl}-1,3-thiazolidine-2,4-dione (0.62 g, 1.7 mmol) in DCM (15 mL) was added DMSO (0.5 mL) and the solution was cooled to 0 °C. Added phosphorus pentoxide (0.52 g, 1.8 mmol) followed by triethylamine (1.2 mL, 8.3 mmol). The reaction was allowed to slowly warm to RT. After 2 hours water was added and the phases were separated. The pH of the aqueous phase was adjusted to ca. 7 with 2M NaOH. The aqueous phase was extracted with EtOAc. The combined extracts were washed with brine, dried (Na₂SO₄), filtered and evaporated in vacuo. The resulting white solid was chromatographed on a small silica gel column eluting with 10% EtOAc/DCM. Fractions containing product were combined and evaporated in vacuo to give 0.33 g (54%) of the title compound as a white solid. MS (ESI+) for C₁₉H₁₇NO₅S m/z 372.0 (M+H)⁺. MS (ESI-) for C₁₉H₁₇NO₅S m/z 370.1 (M-H)⁻.

### Example 6: Preparation of 5-{4-[2-(2-methoxyphenyl)-2-oxoethoxy]benzyl}-1,3-thiazolidine-2,4-dione (*).

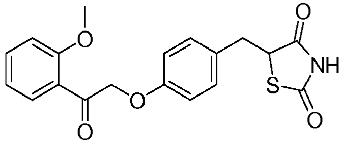

### Step 1: Preparation of 2-(2-methoxyphenyl)oxirane.

To a solution of 2-vinyl anisole (5.0 g, 0.037 mol) and acetic acid (2.1 mL, 37 mmol) in dioxane (33 mL) and H₂O (78 mL) at 0 °C was added N-bromosuccinimide (7.30 g, 40.1 mmol) in three portions. The reaction was allowed to warm to RT and after 1 hour, 2M NaOH (50 mL) was added. The reaction was left to stir at RT overnight. The reaction mixture was partitioned between water and EtOAc, and the aqueous phase was extracted with EtOAc. The combined organic phases were washed with brine, dried (Na₂SO₄), filtered and evaporated in vacuo to give 7.56 g slightly tinted oil. This was dissolved in dioxane, 2N NaOH was added and the reaction was stirred at RT overnight. Repeated aqueous work-up gave 5.60 g of the title compound as a nearly colorless oil.

### Step 2: Preparation of 4-[2-hydroxy-2-(2-methoxyphenyl)ethoxy]benzaldehyde.

To a stirring solution of 2-(2-methoxyphenyl)oxirane (5.60 g, 37.3 mmol) in toluene (65 mL) was added 4-hydroxybenzaldehyde (6.80 g, 56.0 mmol), 1M NaOH (65 mL) and PEG4000 (polyethylene glycol, 0.85 g) and the stirring mixture was heated at 78 °C overnight. The reaction was allowed to cool to RT and it was then extracted with EtOAc (2 x 150 mL). The combined extracts were washed with brine, dried (Na₂SO₄), filtered and evaporated in vacuo. The resulting light oil was adsorbed onto silica gel and chromatographed eluting with 30-40% EtOAc/hexanes to give 2 major spots. Fractions containing the higher Rf spot were combined and evaporated in vacuo to give 1.71 g (17%) the regioisomer as a brown oil. Fractions containing the lower R_{f} spot were combined and evaporated in vacuo to give 2.05 g (20%) of the title compound as a yellow solid.

### Step 3: Preparation of (5Z)-5-{4-[2-hydroxy-2-(2-methoxyphenyl)ethoxy] benzylidene}-1,3-thiazolidine-2,4-dione.

To a stirring solution of 4-[2-hydroxy-2-(2-methoxyphenyl)ethoxy]benzaldehyde (1.71 g, 6.28 mmol) in absolute EtOH (50 mL) was added 2,4-thiazolidinedione (0.81g, 6.91 mmol) and piperidine (0.68 mL, 6.9 mmol), and the resulting solution was heated to reflux. The reaction was refluxed overnight and then evaporated in vacuo. The residue was dissolved in EtOAc and this solution was washed with aqueous HOAc (pH 5-6), brine, dried (Na₂SO₄), filtered and evaporated in vacuo. The residue was adsorbed onto silica gel and chromatographed on silica gel eluting with 20-40% EtOAc/DCM. Fractions containing product were combined and evaporated in vacuo to give 1.87 g (80%) of the title compound as a light yellow solid. MS (ESI-) for C₁₉H₁₇NO₅S m/z 370.1 (M-H)⁻.

### Step 4: 5-{4-[2-hydroxy-2-(2-methoxyphenyl)ethoxy]benzyl}-1,3-thiazolidine-2,4-dione.

(5Z)-5-{4-[2-hydroxy-2-(2-methoxyphenyl)ethoxy]benzylidene}-1,3-thiazolidine-2,4-dione (1.00 g, 2.69 mmol) was dissolved in THF (20 mL). Water (20 mL) was added and then sufficient additional THF was added to give a clear solution. A small crystal of cobalt chloride was added followed by 2,2'-bipyridine (95 mg, 0.61 mmol). The reaction mixture was cooled to 0 °C. NaBH₄ was added in portions until the blue color persisted. The color gradually faded and was regenerated repeatedly by small additions of borohydride and HOAc. When HPLC indicated that the reaction was complete the reaction mixture was partitioned between EtOAc and H₂O. HOAc was added until the pH of the aqueous phase was ca. 6, and the aqueous phase was extracted with EtOAc. The combined organic phases were washed with brine, dried (Na₂SO₄), filtered and evaporated in vacuo. The residue was chromatographed on a small silica gel column eluting with 20% EtOAc/DCM. Fractions containing product were combined and evaporated in vacuo to give 0.63 g (63%) of the title compound as a white solid. MS (ESI-) for C₁₉H₁₉NO₅S m/z 372.1 (M-H)⁻.

### Step 5: Preparation of 5-{4-[2-(2-methoxyphenyl)-2-oxoethoxy]benzyl}-1,3-thiazolidine-2,4-dione.

To a stirring solution of phosphorus pentoxide (0.30 g, 1.10 mmol) in DCM (8 mL) at 0 °C was added a solution of 5-{4-[2-hydroxy-2-(2-methoxyphenyl)ethoxy]benzyl}-1,3-thiazolidine-2,4-dione (0.20 g, 0.54 mmol) in DCM (8 mL) followed by dimethyl sulfoxide (0.20 mL, 2.80 mmol). After stirring for 15 minutes, N,N-diiisopropylethylamine (0.28 mL, 1.60 mmol) was added. After 45 minutes, the reaction mixture was cast into cold saturated NaHCO₃ and extracted with EtOAc (x2). The combined extracts were washed with brine, dried (Na₂SO₄), filtered and evaporated in vacuo. The residue was chromatographed on a small silica gel column eluting with 0-10% EtOAc/DCM. Fractions containing product were combined and evaporated in vacuo to give 175 mg (88%) of the title compound as a light yellow solid. MS (ESI-) for C₁₉H₁₇NO₅S m/z 370.1 (M-H)⁻.

### Example 7: Preparation of 5-{4-[2-(3-chlorophenyl)-2-oxoethoxy]benzyl}-1,3-thiazolidine-2,4-dione (*).

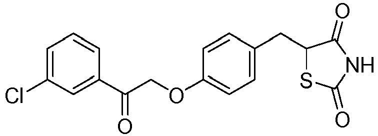

### Step 1: 2-(3-chlorophenyl)oxirane.

To a solution of m-chlorostyrene (5.70 g, 41.0 mmol) and acetic acid (2.33 mL, 40.9 mmol) in dioxane (33 mL) and H₂O (78 mL) at 0 °C was added N-bromosuccinimide (8.02 g, 45.0 mmol) in three portions. The reaction was allowed to warm to RT. After 4 hours, 2N NaOH (60 mL) was added and the reaction was allowed to stir at RT overnight. The reaction mixture was partitioned between water and EtOAc, and the aqueous phase was extracted with EtOAc. The combined organic phases were washed with brine, dried (Na₂SO₄), filtered and evaporated in vacuo to give 6.20 g of a slightly tinted oil which was used without further purification.

### Step 2: 4-[2-(3-chlorophenyl)-2-hydroxyethoxy]benzaldehyde.

To a stirring solution of 2-(3-chlorophenyl)oxirane (6.20 g, 40.0 mmol) in toluene (65 mL) was added 4-hydroxybenzaldehyde (7.30 g, 60.0 mmol), 1M NaOH (65 mL) and PEG4000 (polyethylene glycol, 0.85 g) and the stirring mixture was heated at 78 °C for three hours. The reaction was allowed to cool to RT and then extracted with EtOAc (2 x 150 mL). The combined extracts were washed with brine, dried (Na₂SO₄), filtered and evaporated in vacuo. The resulting light brown oil was adsorbed onto silica gel and chromatographed eluting with 25-40% EtOAc/hexanes. There are 2 major spots. Fractions containing the higher Rf spot were combined and evaporated in vacuo to give 1.08 g (10%) of the desired product as a colorless oil. Fractions containing the lower Rf spot were combined and evaporated in vacuo to give 0.95 g (8%) of the regioisomer as a colorless oil, 44B. Some starting epoxide (2.85 g) was also recovered.

### Step 3: 5-{4-[2-(3-chlorophenyl)-2-hydroxyethoxy]benzylidene}-1,3-thiazolidine-2,4-dione.

To a stirring solution of 4-[2-(3-chlorophenyl)-2-hydroxyethoxy]benzaldehyde (1.08 g, 3.90 mmol) in absolute EtOH (50 mL) was added 2,4-thiazolidinedione (0.50 g, 4.29 mmol) and piperidine (0.42 mL, 4.3 mmol), and the resulting solution was heated to reflux and then stirred overnight at room temperature. The reaction mixture was evaporated in vacuo and the residue was dissolved in EtOAc. This solution was washed with aqueous HOAc (pH 5-6), brine, dried (Na₂SO₄), filtered and evaporated in vacuo. The residue was adsorbed onto silica gel and chromatographed eluting with 10-20% EtOAc/DCM. Fractions containing product were combined and evaporated in vacuo to give 1.31 g (89%) of the product as a light yellow solid. MS (ESI+) for C₁₈H₁₄ClNO₄S m/z 375.0 (M+H)⁺. MS (ESI-) for C₁₈H₁₄ClNO₄S m/z 374.1 (M-H)⁻.

### Step 4: 5-{4-[2-(3-chlorophenyl)-2-hydroxyethoxy]benzyl}-1,3-thiazolidine-2,4-dione.

5- {4-[2-(3-chlorophenyl)-2-hydroxyethoxy]benzylidene} -1 ,3-thiazolidine-2,4-dione (0.74 g, 2.00 mmol) was dissolved in THF (20 mL). Water (20 mL) was added and then more THF was added until all solids dissolved. A small crystal of cobalt chloride was added, followed by 2,2'-bipyridine (69 mg, 0.44 mmol). The reaction mixture was cooled to 0 °C. NaBH₄ was added in portions until the blue color persisted. The color gradually faded and was regenerated repeatedly by small additions of borohydride and HOAc. When HPLC indicated that the reaction was complete, the reaction mixture was partitioned between EtOAc and H₂O. HOAc was added until the pH of the aqueous phase was ca. 6, and then the aqueous phase was extracted with EtOAc. The combined organic phases were washed with brine, dried (Na₂SO₄), filtered and evaporated in vacuo. The residue was chromatographed on a small silica gel column eluting with 0-10% EtOAc/DCM. Fractions containing product were combined and evaporated in vacuo to give 0.44 g (59%) of a sticky yellow solid. MS (ESI-) for C₁₈H₁₆ClNO₄S m/z 376.1 (M-H)⁻.

### Step 5: Preparation of 5-{4-[2-(3-chlorophenyl)-2-oxoethoxy]benzyl}-1,3-thiazolidine-2,4-dione.

To a stirring solution of phosphorus pentoxide (0.38 g, 1.30 mmol) in DCM (8 mL) at 0 °C was added a solution of 5-{4-[2-(3-chlorophenyl)-2-hydroxyethoxy]benzyl}-1,3-thiazolidine-2,4-dione (0.25 g, 0.66 mmol) in DCM (8 mL) followed by dimethyl sulfoxide (0.23 mL, 3.30 mL). After stirring for 15 minutes N,N-diiisopropylethylamine (0.34 mL, 2.00 mmol) was added. After 45 minutes the reaction was poured into cold saturated NaHCO₃ and the mixture was extracted with EtOAc (x2). The combined extracts were washed with brine, dried (Na₂SO₄), filtered and evaporated in vacuo. The residue was chromatographed on a small silica gel column eluting with 0-15% EtOAc/DCM. Fractions containing product were combined and evaporated in vacuo to give 117 mg (47%) of a white solid. MS (ESI-) for C₁₈H₁₄ClNO₄S m/z 374.1 (M-H)⁻.

### Example 8: Preparation of 5-{4-[2-(2-chlorophenyl)-2-oxoethoxy]benzyl}-1,3-thiazolidine-2,4-dione (*).

The title compound can be prepared as described in Example 7 using appropriate starting materials, such as 2-(2-chlorophenyl)oxirane.

### Example 9: Preparation of 5-{4-[2-(4-methoxyphenyl)-2-oxoethoxy]benzyl}-1,3-thiazolidine-2,4-dione (*).

The title compound was prepared as described in Examples 5 and 6 using appropriate starting materials, such as 2-(4-methoxyphenyl)oxirane. MS (ESI-) for C₁₉H₁₇NO₅S 370.2 m/z (M-1).

### Physical Data for Representative Compounds

### ¹H-NMR Data (400mHz)

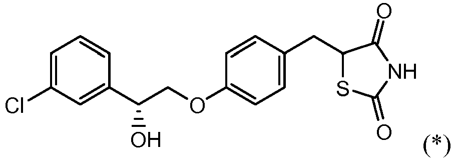

¹H-NMR (DMSO-*d*₆) δ: 12.00 (s, 1H), 7.50 (s, 1H), 7.42-7.32 (m, 3H), 7.13 (d, *J* = 8.5 Hz, 2H), 6.87 (d, *J* = 8.5 Hz, 2H), 5.77 (d, *J* = 5.0 Hz, 1H), 4.92 (d, *J* = 6.2 Hz, 1H), 4.86 (dd, *J* = 8.9, 4.3 Hz, 1H), 4.00 (m, 2H), 3.29 (dd, *J* = 14.3, 4.3 Hz, 1H), 3.05(dd, *J* = 14.2, 9.0 Hz, 1H).

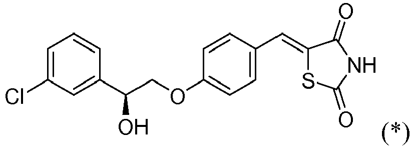

¹H-NMR (DMSO-*d*₆) δ: 12.52 (s, 1H), 7.75 (s, 1H), 7.54 (m, 3H), 7.44-7.33 (m, 3H), 7.11 (d, *J* = 8.91 Hz, 2H), 5.84 (d, *J* = 4.77 Hz, 1H), 4.97 (m, 1H), 4.12 (m, 2H).

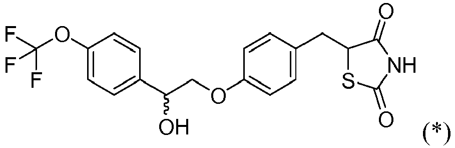

¹H-NMR (CDCl₃) δ: 8.32 (brs, 1H), 7.50 (d, *J* = 8.50 Hz, 2H), 7.26 (m, 2H), 7.17 (m, 2H), 6.88 (m, 2H), 5.15 (dd, *J* = 8.71, 3.11 Hz, 1H), 4.51 (dd, *J* = 9.23, 4.04 Hz, 1H), 4.09 (dd, *J* = 9.64, 3.21 Hz, 1H), 3.45 (dd, J = 14.1, 3.94 Hz, 1H), 3.13 (dd, J = 14.2, 9.23 Hz, 1H), 2.87 (brs, 1H).

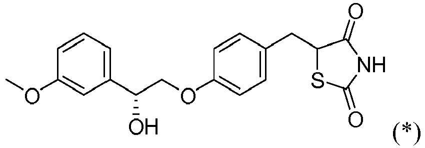

¹H-NMR (CDCl₃) δ: 8.35 (brs, 1H), 7.23 (t, *J* = 8.09, 1H), 7.07 (d, *J* = 8.71 Hz, 2H), 6.94 (m, 2H), 6.81 (m, 3H), 5.03 (dd, *J* = 8.60, 2.80 Hz, 1H), 4.42 (dd, *J* = 9.33, 3.94 Hz, 1H), 4.02 (m, 1H), 3.93 (t, *J* = 9.23 Hz, 1H), 3.76 (s, 3H), 3.36 (dd, *J* = 14.20, 3.84 Hz, 1H), 3.04 (dd, *J* = 14.10, 9.33 Hz, 1H), 2.75 (brs, 1H).

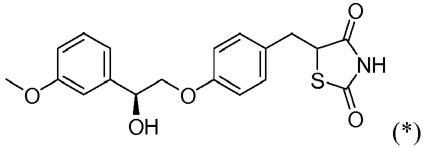

¹H-NMR (CDCl₃) δ: 8.42 (brs, 1H), 7.23 (t, *J* = 7.98 Hz, 1H), 7.07 (d, *J* = 8.71 Hz, 2H), 6.94 (m, 2H), 6.82-6.78 (m, 3H), 5.03 (dd, *J* = 8.71, 2.90 Hz, 1H), 4.41 (dd, *J* = 9.33, 3.94 Hz, 1H), 4.02 (m, 1H), 3.93 (t, *J* = 9.12 Hz, 1H), 3.76 (s, 3H), 3.36 (dd, *J* = 14.10, 3.94 Hz, 1H), 3.03 (dd, *J* = 14.31, 9.33 Hz, 1H), 2.77 (brs, 1H).

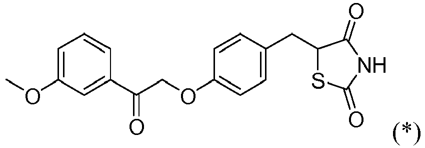

¹H-NMR (DMSO-*d*₆) δ: 12.03 (brs, 1H), 7.62 (d, *J* = 7.67 Hz, 1H), 7.49 (m, 2H), 7.27 (dd, *J* = 8.19, 2.38 Hz, 1H), 7.16 (d, *J* = 8.50 Hz, 2H), 6.91 (d, *J* = 8.50 Hz, 2H), 5.55 (s, 2H), 4.88 (dd, *J* = 9.12, 4.35 Hz, 1H), 3.84 (s, 3H), 3.33-3.29 (m, 1H), 3.05 (dd, *J* = 14.31, 9.12 Hz, 1H).

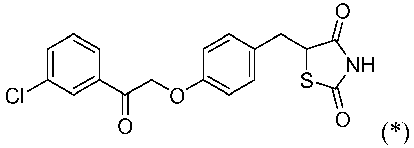

¹H-NMR (DMSO-*d*₆) δ: 12.02 (brs, 1H), 8.05 (t, *J* = 1.66 Hz, 1H), 7.96 (d, *J* = 7.88 Hz, 1H), 7.77 (m, 1H), 7.61 (t, *J* = 7.88 Hz, 1H), 7.16 (d, *J* = 8.71 Hz, 2H), 6.93 (d, *J* = 8.71 Hz, 2H), 5.57 (s, 2H), 4.88 (dd, *J* = 9.12, 4.35 Hz, 1H), 3.31 (m, 1H), 3.06 (dd, *J* = 14.20, 9.23 Hz, 1H).

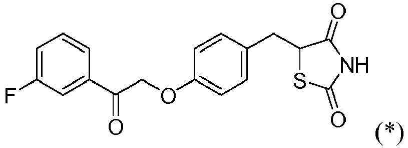

¹H-NMR (DMSO-*d*₆) δ: 12.02 (brs, 1H), 7.83 (m, 2H), 7.59 (m, 2H), 7.16 (d, *J* = 8.71 Hz, 2H), 6.93 (d, *J* = 8.71, 2H), 5.56 (s, 2H), 4.88 (dd, *J* = 9.12, 4.35 Hz, 1H), 3.33-3.29 (m, 1H), 3.06 (dd, *J* = 14.10, 9.12 Hz, 1H).

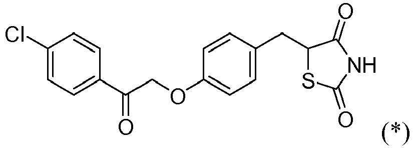

¹H-NMR (DMSO-*d*₆) δ: 12.02 (s, 1H), 8.03 (d, *J* = 8.71 Hz, 2H), 7.65 (d, *J* = 8.50 Hz, 2H), 7.15 (d, *J* = 8.50 Hz, 2H), 6.92 (d, *J* = 8.71 Hz, 2H), 5.54 (s, 2H), 4.88 (dd, *J* = 9.12, 4.35 Hz, 1H), 3.33-3.29 (m, 1H), 3.05 (dd, *J* = 14.10, 9.12 Hz, 1H).

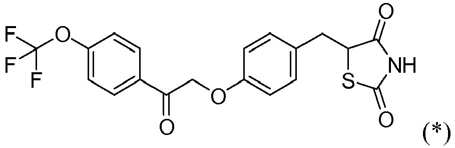

¹H-NMR (CDCl₃) δ: 8.08 (m, 3H), 7.34 (d, *J* = 8.09 Hz, 2H), 7.17 (d, *J* = 8.71 Hz, 2H), 6.90 (d, *J* = 8.71 Hz, 2H), 5.23 (s, 2H), 4.51 (dd, *J* = 9.43, 3.84 Hz, 1H), 3.46 (dd, *J* = 14.10, 3.94 Hz, 1H), 3.13 (dd, 14.20, 9.43 Hz, 1H), 1.60 (brs, 1H).

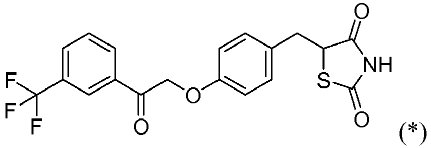

¹H-NMR (DMSO-*d*₆) δ: 12.20 (s, 1H), 8.30 (m, 2H), 8.07 (d, *J* = 7.88 Hz, 1H), 7.82 (t, *J* = 7.88 Hz, 1H), 7.16 (d, *J* = 8.71 Hz, 2H), 6.95 (d, *J* = 8.71 Hz, 2H), 5.64 (s, 2H), 4.88 (dd, *J* = 9.33, 4.35 Hz, 1H), 3.34-3.29 (m, 1H), 3.06 (dd, *J* = 14.10. 9.12 Hz, 1H).

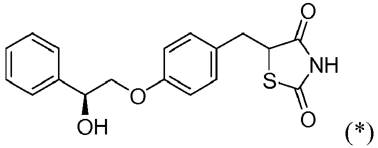

¹H-NMR (CDCl₃) δ: 8.42 (brs, 1H), 7.38 (m, 5H), 7.15 (d, *J* = 8.50 Hz, 2H), 6.88 (d, *J* = 8.50 Hz, 2H), 5.14 (dd, *J* = 8.81, 3.01Hz, 1H), 4.50 (dd, *J* = 9.33, 3.94 Hz, 1H), 4.11 (m, 1H), 4.01 (t, J = 9.23 Hz, 1H), 3.45 (dd, *J* = 14.20, 3.84 Hz, 1H), 3.12 (dd, J = 14.20, 9.43 Hz, 1H), 2.84 (brs, 1H).

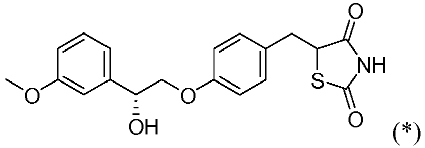

¹H-NMR (CDCl₃) δ: 8.35 (brs, 1H), 7.23 (t, *J* = 8.09, 1H), 7.07 (d, *J* = 8.71 Hz, 2H), 6.94 (m, 2H), 6.81 (m, 3H), 5.03 (dd, *J* = 8.60, 2.80 Hz, 1H), 4.42 (dd, *J* = 9.33, 3.94 Hz, 1H), 4.02 (m, 1H), 3.93 (t, *J* = 9.23 Hz, 1H), 3.76 (s, 3H), 3.36 (dd, *J* = 14.20, 3.84 Hz, 1H), 3.04 (dd, *J* = 14.10, 9.33 Hz, 1H), 2.75 (brs, 1H).

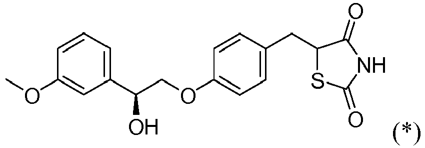

¹H-NMR (CDCl₃) δ: 8.42 (brs, 1H), 7.23 (t, *J* = 7.98 Hz, 1H), 7.07 (d, *J* = 8.71 Hz, 2H), 6.94 (m, 2H), 6.82-6.78 (m, 3H), 5.03 (dd, *J* = 8.71, 2.90 Hz, 1H), 4.41 (dd, *J* = 9.33, 3.94 Hz, 1H), 4.02 (m, 1H), 3.93 (t, *J* = 9.12 Hz, 1H), 3.76 (s, 3H), 3.36 (dd, *J* = 14.10, 3.94 Hz, 1H), 3.03 (dd, *J* = 14.31, 9.33 Hz, 1H), 2.77 (brs, 1H).

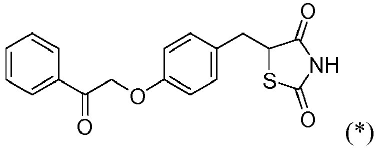

¹H-NMR (DMSO-*d*₆) δ: 12.03 (brs, 1H), 8.02 (m, 2H), 7.69 (t, *J* = 7.36 Hz, 1H), 7.57 (t, *J* = 7.67 Hz, 2H), 7.15 (d, *J* = 8.50 Hz, 2H), 6.91 (d, *J* = 8.50 Hz, 2H), 5.56 (s, 2H), 4.88 (dd, *J* = 9.23, 4.25 Hz, 1H), 3.31 (m, 2H), 3.05 (dd, *J* = 14.02, 9.23 Hz, 1H).

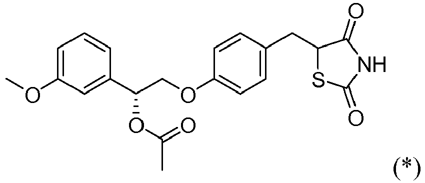

¹H-NMR (CDCl₃): δ = 8.57(brs, 1H), 7.28(m, 1H), 7.16(m, 1H), 6.99(m, 2H), 6.87(m, 3H), 6.12(dd, J=7.8, 3.6Hz, 1H), 4.49(dd, J=9.3, 3.9Hz, 1H), 4.25(m, 1H), 4.13(dd, J=10.5, 3.6Hz, 1H), 3.83(s, 3H), 3.45(dd, J=14.2, 3.8Hz, 1H), 3.10(dd, J=14.0, 9.6Hz, 1H), 2.14(s, 3H).

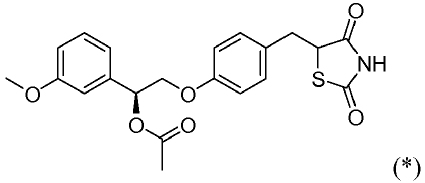

¹H-NMR (CDCl₃): δ = 8.31(brs, 1H), 7.29(m, 1H), 7.17(m, 1H), 6.99(m, 2H), 6.88(m, 3H), 6.12(dd, J=7.8, 3.4Hz, 1H), 4.50(dd, J=9.4, 3.8Hz, 1H), 4.25(m, 1H), 4.13(dd, J=10.4, 3.7Hz, 1H), 3.83(s, 3H), 3.45(dd, J=14.2, 3.8Hz, 1H), 3.11(dd, J=14.1, 9.3Hz, 1H), 2.14(s, 3H).

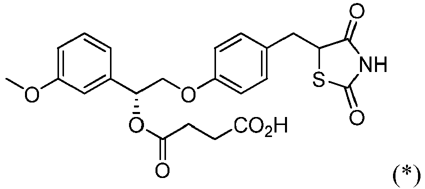

¹H-NMR (CDCl₃): δ = 8.65(m, 1H), 7.29(m, 1H), 7.13(m, 1H), 6.97(m, 2H), 6.86(m, 3H), 6.13(m, 1H), 4.49(dd, J=9.1, 3.9Hz, 1H), 4.24(m, 1H), 4.14(m, 1H), 3.82(s, 3H), 3.40(m, 1H), 3.12(dd, J=14.2, 9.0Hz, 1H), 2.69(m, 4H).

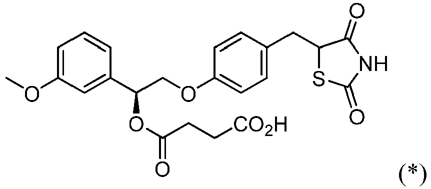

¹H-NMR (CDCl₃): δ = 8.78(brs, 1H), 7.29(m, 1H), 7.13(m, 1H), 6.97(m, 2H), 6.85(m, 3H), 6.12(m, 1H), 4.47(dd, J=8.8, 3.8Hz, 1H), 4.20(m, 2H), 3.81(s, 3H), 3.36(m, 1H), 3.13(m, 1H), 2.68(m, 4H).

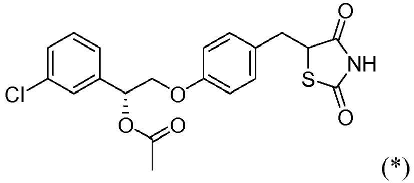

¹H-NMR (CDCl₃): δ = 8.74(brs, 1H), 7.42(s, 1H), 7.31(m, 2H), 7.15(d, J-8.7Hz, 2H), 6.85(d, J=8.7Hz, 2H), 6.10((dd, J=7.4, 4.0Hz, 1H), 4.50(dd, J=9.3, 3.9Hz, 1H), 4.24(M, 1H), 4.13(dd, J=10.4, 4.2Hz, 1H), 3.45(dd, J=14.1, 3.7Hz, 1H), 3.10(dd, J=14.0, 9.4Hz, 1H), 2.15(s, 3H).

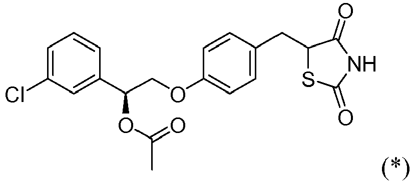

¹H-NMR (CDCl₃): δ = 8.67(brs, 1H), 7.42(s, 1H), 7.30(m, 2H), 7.15(d, J=7.2Hz, 2H), 6.85(d, J=8.5Hz, 2H), 6.10(dd, J=7.4, 4.0Hz, 1H), 4.50(dd, J=9.3, 3.9Hz, 1H), 4.24(m, 1H), 4.13(dd, J=10.4, 4.2Hz, 1H), 3.45(dd, J=14.2, 3.8Hz, 1H), 3.11(dd, J=14.2, 9.4Hz, 1H), 2.15(s, 3H).

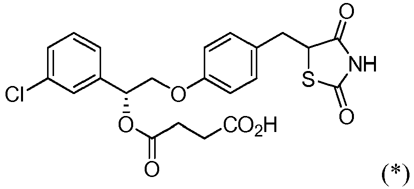

¹H-NMR (CDCl₃): δ = 8.94,(d, J=4.8Hz, 1H), 7.40(s, 1H), 7.30(m, 3H), 7.14(d, J=8.5Hz, 2H), 6.84(d, J=8.5Hz, 2H), 6.11(m, 1H), 4.49(dd, J=9.0, 3.8Hz, 1H), 4.23(m, 1H), 4.13(m, 1H), 3.40(dd, J=14.1, 3.5Hz, 1H), 3.13(dd, J=14.1, 9.1Hz, 1H), 2.71(m, 4H).

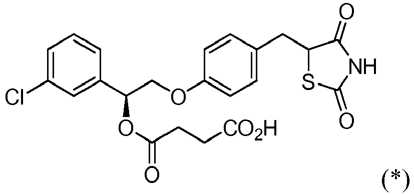

H-NMR (CDCl₃): δ = 8.88(d, J=6.4Hz, 1H), 7.40(s, 1H), 7.30(m, 3H), 7.14(d, J=8.5Hz, 2H), 6.84(d, J=7.7Hz, 2H), 6.11(m, 1H), 4.49(dd, J=9.1, 3.9Hz, 1H), 4.24(m, 1H), 4.14(m, 1H), 3.40(dd, J=14.3, 3.7Hz, 1H), 3.13(dd, J=14.2, 9.0Hz, 1H), 2.70(m, 4H).

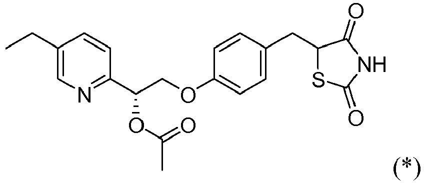

¹H-NMR (CDCl₃): □ = 9.34(brs, 1H), 8.46, s, 1H), 7.56(dd, J=8.0, 2.0Hz, 1H), 7.36(d, J=8.0, 1H), 7.13(d, J=7.1Hz, 2H), 6.86(dd, J=8.6, 1.8Hz, 2H), 6.18(dd, J=6.4, 4.1Hz, 1H), 4.48(m, 1H), 4.41(m, 1H), 3.44(m, 1H), 3.09(m, 1H), 2.67(q, J=7.6Hz, 2H), 2.15(s, 3H), 1.26(t, J=7.6Hz, 3H).

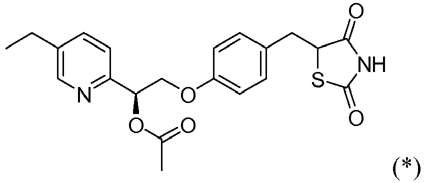

¹H-NMR (CDCl₃): δ = 8.85(brs, 1H), 8.46(d, J=1.7Hz, 1H), 7.56(dd, J=8.0, 2.0Hz, 1H), 7.37(d, J=8.1Hz, 1H), 7.13(d, J=8.7Hz, 2H), 6.86(d, J=7.1Hz, 2H), 6.19(dd, J=6.4, 4.2Hz, 1H), 4.49(dd, J=9.1, 3.5Hz, 1H), 4.41(m, 2H), 3.44(m, 1H), 3.10(m, 1H), 2.67(q, J=7.5Hz, 2H), 2.16(s, 3H)., 1.26(t, 3H).

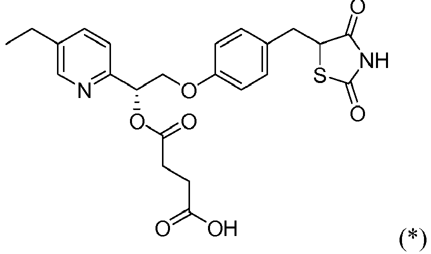

¹H-NMR (CDCl₃): δ = 8.63(brs, 1H), 8.45(s, 1H), 7.77(t, J=7.6Hz, 1H), 7.56(dd, J=7.9, 1.9Hz, 1H), 7.10(d, J=8.3Hz, 2H), 6.83(d, J=8.5Hz, 2H), 6.19(t, J=5.1Hz, 1H), 4.46(dd, J=9.0, 3.8Hz, 1H), 4.39(m, 2H), 3.38(dd, J=14.2, 3.8Hz, 1H), 3.10(dd, J=14.2, 9.2Hz, 1H), 2.68(m, 6H), 1.24(t, J=7.6Hz, 3H).

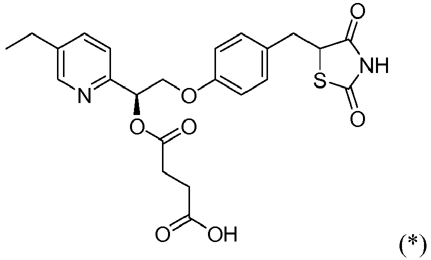

¹H-NMR (CDCl₃): δ = 9.20(brs, 1H), 8.48(s, 1H), 7.60(d, J=1.7Hz, 1H), 7.40(d, J=8.1Hz, 1H), 7.12(dd, J=8.5, 1.7Hz, 2H0, 6.84(dd, J=8.7, 2.7Hz, 2H), 6.20(m, 1H), 4.49(dd, J=8.3, 4.2Hz, 1H), 4.40(m, 2H), 3.33(m, 1H), 3.18(m, 1H), 2.71(m, 6H), 1.25(t, J=7.6Hz), 3H).

### Mass Spectra

| **Structure** | **Calc. MW** | **Found MW** |
|---|---|---|
| 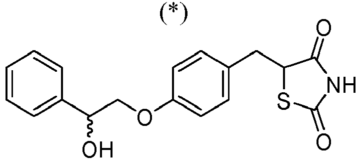 | 343.4 | ES+ 366.0 (M+Na) |
| | | ES-342.1 |
| | | (M-1) |
| 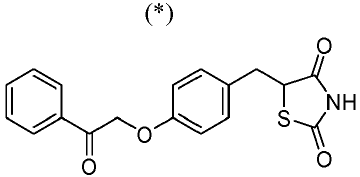 | 341.38 | ES+ 363.9 (M+Na) |
| | | ES-340.0 (M-1) |
| 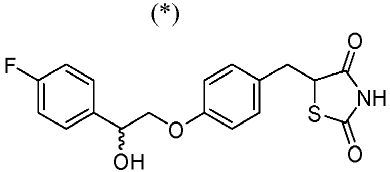 | 361.39 | ES-360.1 (M-1) |
| 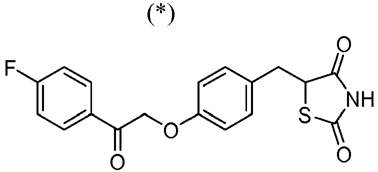 | 359.37 | ES+ 360.2 (M+1) |
| | | ES-358.2 (M-1) |
| 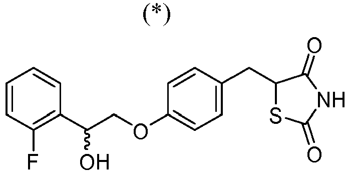 | 361.39 | ES-360.1 (M-1) |
| 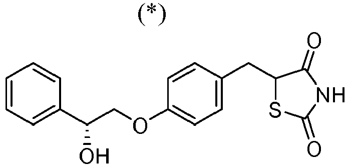 | 343.4 | ES-342.2 (M-1) |
| 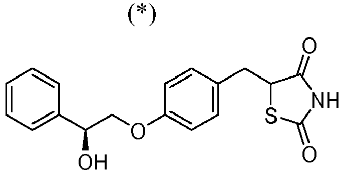 | 343.4 | ES-342.1 (M-1) |
| 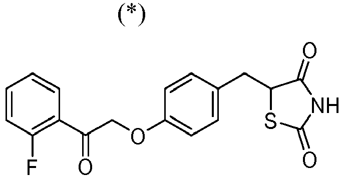 | 359.37 | ES-358.0 (M-1) |
| 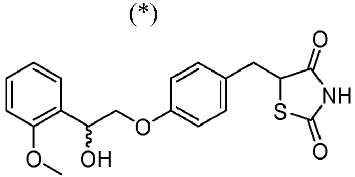 | 373.42 | ES-372.1 (M-1) |
| 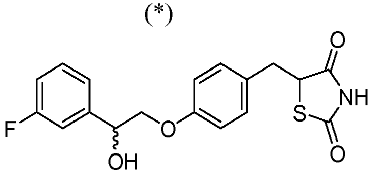 | 361.39 | ES+ 384.0 (M+Na) |
| | | ES-360.1 (M-1) |
| 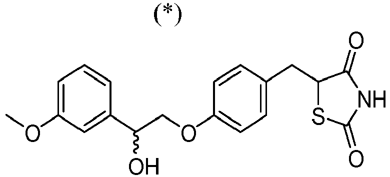 | 373.42 | ES-372.0 (M-1) |
| 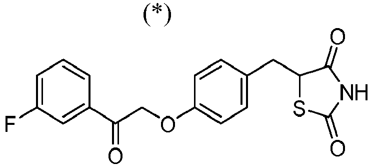 | 359.37 | ES-358.2 (M-1) |
| 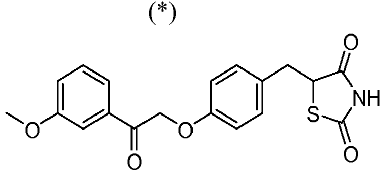 | 371.41 | ES+ 372.0 (M+1) |
| | | ES-370.1 (M-1) |
| 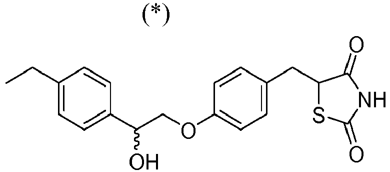 | 371.45 | ES-370.2 (M-1) |
| 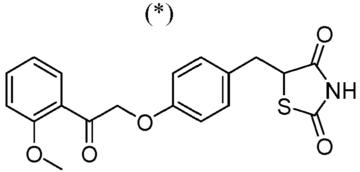 | 371.41 | ES-370.1 (M-1) |
| 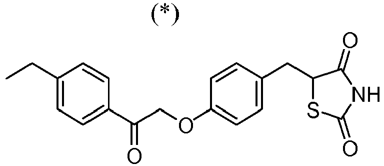 | 369.43 | ES+ 370.0 (M+1) |
| | | ES-368.1 (M-1) |
| 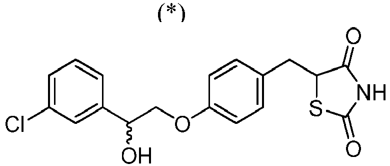 | 377.84 | ES-376.0 (M-1) |
| 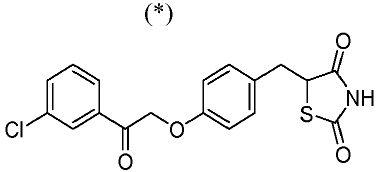 | 375.83 | ES-374.0 (M-1) |
| 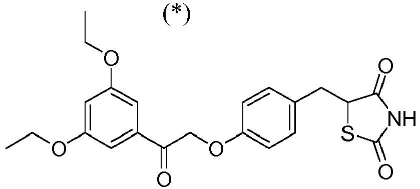 | 429.49 | ES+ 430.1 (M+1) |
| | | ES-428.2 (M-1) |
| 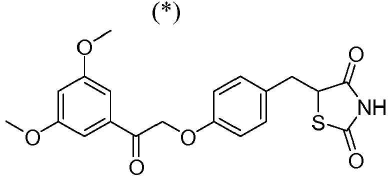 | 401.43 | ES+ 402.1 (M+1) |
| | | ES-400.2 (M-1) |
| 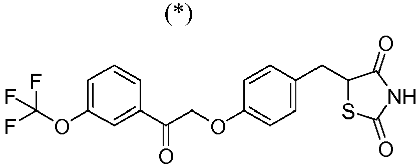 | 425.38 | ES+ 426.0 (M+1) |
| | | ES-424.1 (M-1) |
| 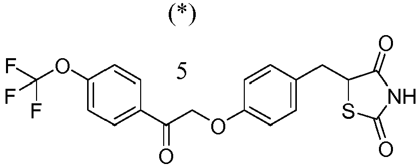 | 425.38 | ES+ 425.9 (M+1) |
| | | ES-424.2 (M-1) |
| 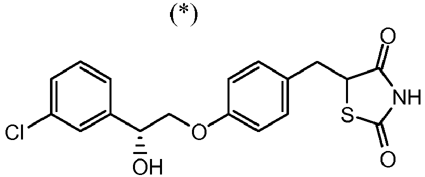 | 377.84 | ES-376.2 (M+1) |
| 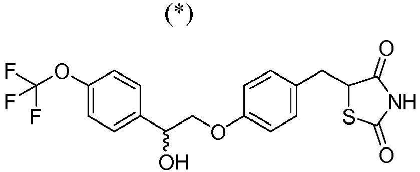 | 427.39 | ES-426.3 (M+) |
| 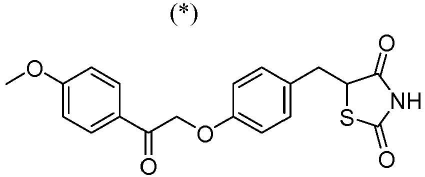 | 371.41 | ES-370.2 (M-1) |
| 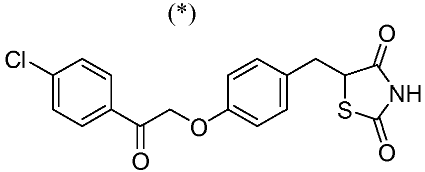 | 375.83 | ES+ 376.2 (M+1) |
| 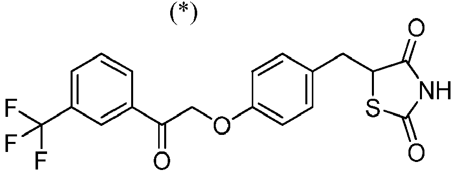 | 409.38 | ES-408.3 (M-1) |
| 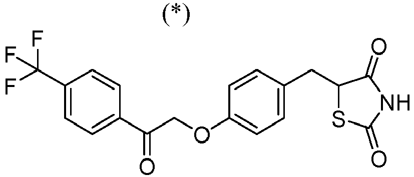 | 409.38 | ES-408.1 (M-1) |
| 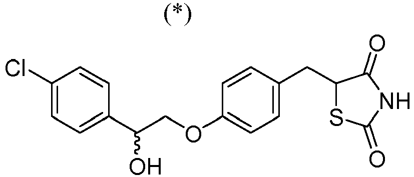 | 377.84 | ES-376.1 (M-1) |
| 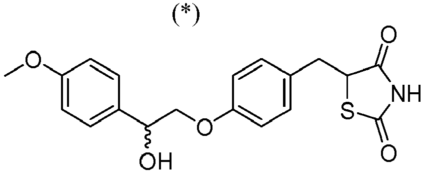 | 373.42 | ES-372.1 (M-1) |
| 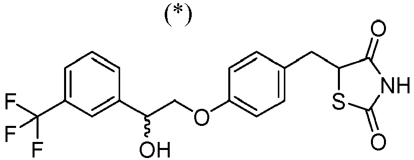 | 411.39 | ES-410.2 (M-1) |
| 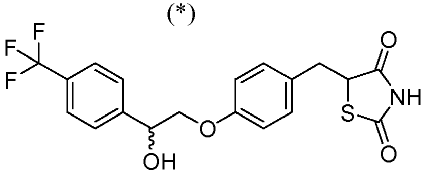 | 411.39 | ES-410.2 (M-1) |
| 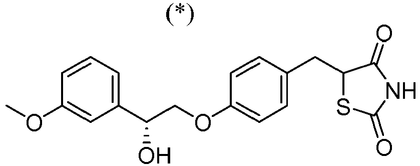 | 373.42 | ES-372.1 (M-1) |
| 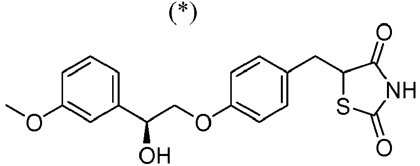 | 373.42 | ES-372.1 (M-1) |
| 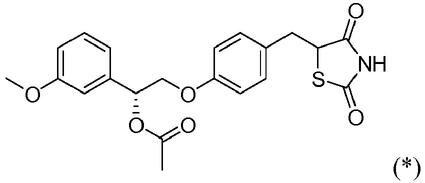 | 415.46 | ES-414.10 (M-1) |
| 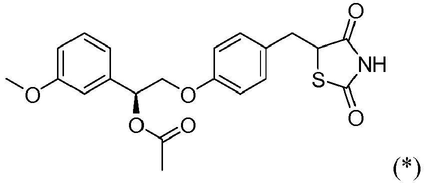 | 415.46 | ES-414.1 m/z (M-1) |
| 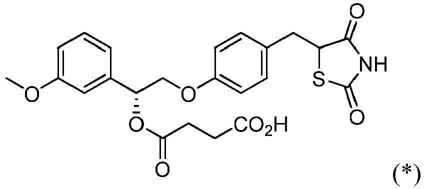 | 473.5 | ES-472.0 m/z (M-1) |
| 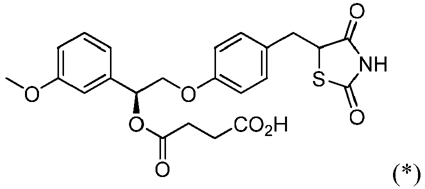 | 473.5 | ES-472.0 m/z (M-1) |
| 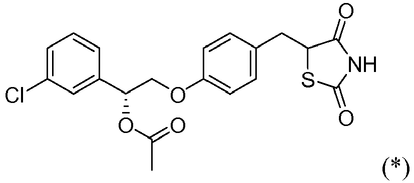 | 419.88 | ES-418.0 m/z (M-1) |
| 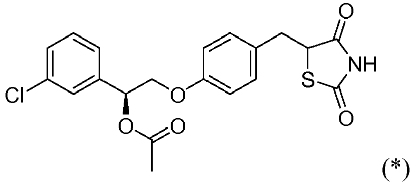 | 419.88 | ES- 418 m/z (M-1) |
| 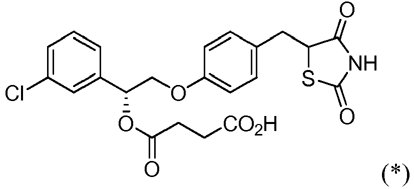 | 477.19 | ES-476.0 m/z (M-1) |
| 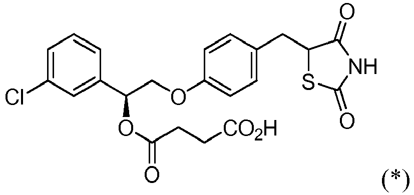 | 477.19 | ES-476.0 m/z (M-1) |
| 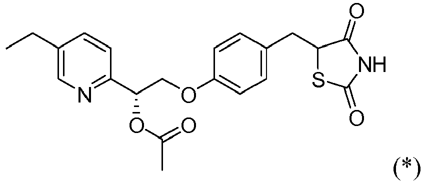 | 414.47 | ES+ 415.0 m/z (M+1); ES-413.0 m/z (M-1) |
| 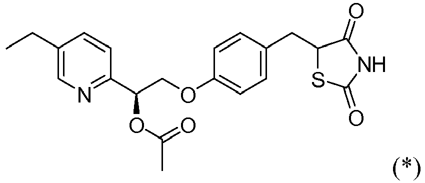 | 414.47 | ES+ 415.0 m/z (M-1); ES-413.0 m/z (M-1) |
| 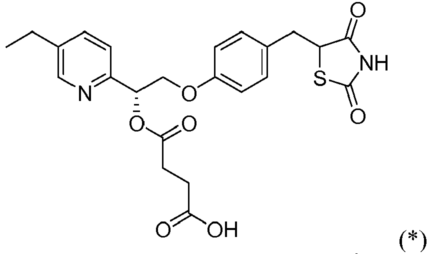 | 472.51 | ES+ 473.0 m/z (M+1); ES-471.0 m/z (M-1) |
| 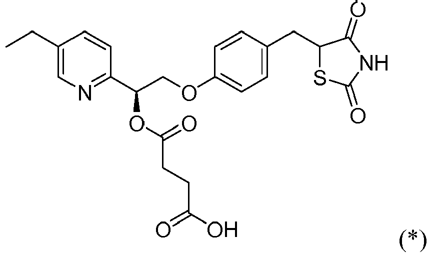 | 472.51 | ES+ 472.9 m/z (M+1) |
| | | ES-471.0 m/z (M-1) |
| 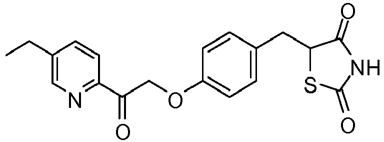 | 370.42 | ES +371.1 m/z (M+1) |
| | | ES - 369.1 (M-1) |
| 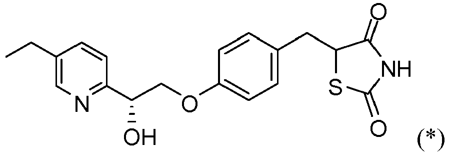 | 372.11 | ES +373.1 m/z (M+1) |
| | | ES - 371.1 (M-1) |
| 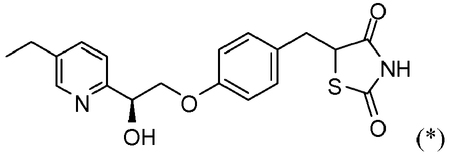 | 372.11 | ES +373.0 m/z (M+1) |
| | | ES - 371.1 (M-1) |
| 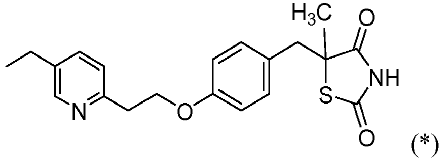 | 370.47 | ES+ 371.2 m/z (M+1) |
| | | ES-369.2 (M-1) |
| 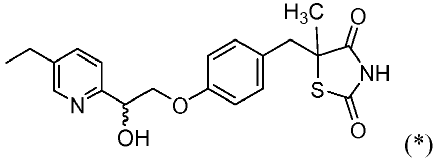 | 386.46 | ES +387.3 m/z (M+1) |
| | | ES - 385.3 (M-1) |
| 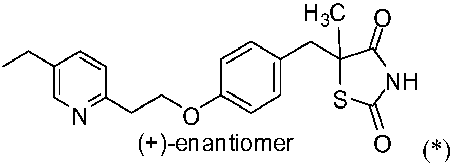 | 370.47 | ES +371.2 m/z (M+1) |
| | | ES - 369.2 (M-1) |
| 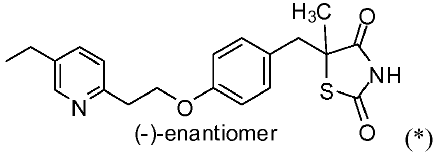 | 370.47 | ES +371.2 m/z (M+1) |
| | | ES - 369.2 (M-1) |
| 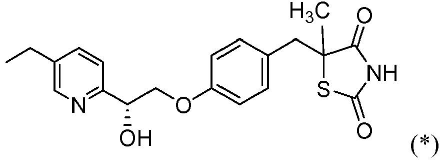 | 386.46 | ES +387.3 m/z (M+1) |
| | | ES - 385.3 (M-1) |
| 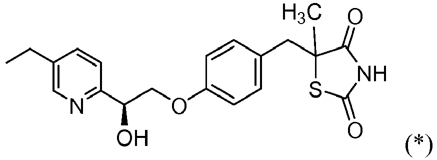 | 386.46 | ES +387.2 m/z (M+1) |
| | | ES - 385.2 (M-1) |
| 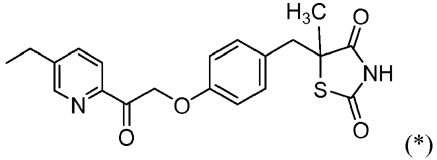 | 384.45 | ES +385.1 m/z (M+1) |
| | | ES - 383.1 (M-1) |
| 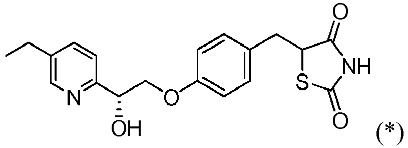 | 386.46 | ES+ 373.2 (M+1) ES-371.2 (M-1) |

### Example 10: Synergy between PPAR-sparing compounds and norepinephrine on the expression of PGC-1α.

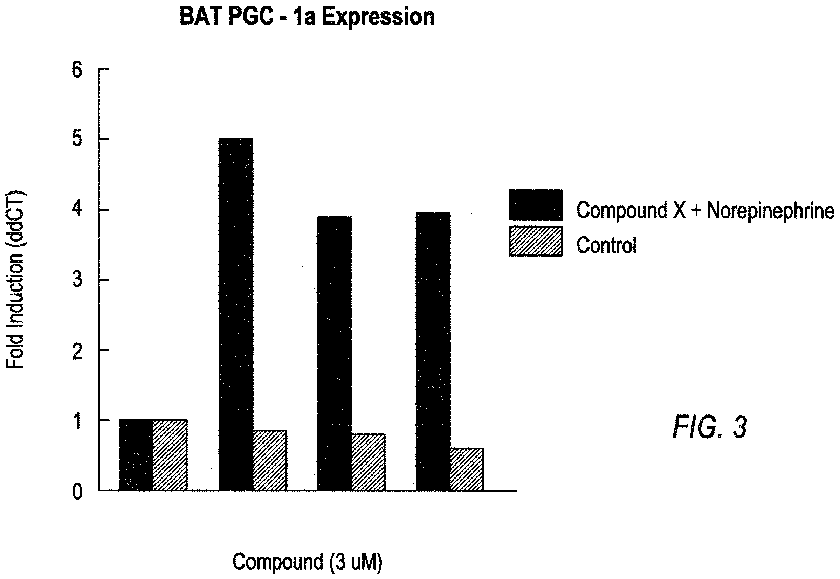

Another example of the ability of augmented signaling between cyclic nucleotides and compounds of Formula I is shown by the effect on expression of PGC-1α, a known regulator of mitochondrial biogenesis. Increased numbers of mitochondria are predictive of utility for the reduction of body weight. Figure 3 shows that three compounds of Formula I augment the ability of norepinephrine to increase the expression of PGC-1α.

Precursor BAT cells were isolated as described above and treated with or without 3 µM compounds: 1.] Compound X: 5-(4-(2-(5-ethylpyridin-2-yl)-2-oxoethoxy)benzyl)-1,3-thiazolidine-2,4-dione; 2.] Compound Y(*): 5-(4-(2R)- 2-(5-ethylpyridin-2-yl)-2-hydroxyethoxyy)benzyl)-1,3-thiazolidine-2,4-dione; or 3.] Compound Z(*): 5-(4-(2-(3-methoxyphenyl)- 2-oxoethoxy)benzyl)-1,3-thiazolidine-2,4-dione for seven days followed by treatment with 1 µM norepinephrine for 2 hours. Total RNA was isolated from the cells and the RNA message (mRNA) for PGC-1α was measured by quantitative polymerase chain reactions. In the absence of compound (control), norepinephrine alone did not produce an increase in the PGC-1 α mRNA; however, in the presence of Compounds X, Y(*), or Z(*), an increase in PGC-1 α message was observed in the presence of norepinephrine (solid bars) supporting the utility of compounds of Formula I, salts of compounds of formula I, co-crystals of compounds of Formula I, or combinations thereof.

### Example 11: Preparation of Acid Salts.

A compound of Formula I may be converted to a salt by dissolving the compound in a solvent in which the acid salt of the organic compound is insoluble or is only sparingly soluble; adding one or more molar equivalents of an acid, such as HCl, HBr, acetic acid, trifluroacetic acid, or H₂SO₄, methane sulfonic acid, *p*-toluene sulfonic acid, trifluoromethanesulfonic acid, or the like, to the solvent containing the dissolved compound of Formula I to form a precipitate of the organic compound salt; and collecting the precipitate using filtration, decanting or some similar method to produce the salt of the organic compound of Formula I in a pure form.

Alternatively, a compound of Formula I may be converted to a salt by dissolving the compound in a solvent in which the salt of the organic compound is also soluble; adding one or more molar equivalents of an acid with a relatively low boiling point, such as HCl, H₂SO₄, acetic acid, trifluroacetic acid, or the like, to the solvent containing the dissolved compound of Formula I; an then evaporating the solvent and any excess acid contained in the solution to produce the salt of the organic compound in a pure form.

### Example 12: Preparation of Co-Crystals.

### Co-Crystal A:

To caffeine (0.194g, 1mmol) and 5-(4-(2-(5-ethylpyridin-2-yl)-2-oxoethoxy)benzyl)-1,3-thiazolidine-2,4-dione (0.370g, 1mmol) was added acetonitrile (20mL). The mixtures was warmed in a 75 °C oil bath until the solids dissolved. Warming was continued for about 10 minutes, then the solution was filtered and allowed to cool to room temperature. The solvent was allowed to evaporate until crystallization was complete. Co-crystalline solid was isolated by filtration and was dried in vacuo. The melting point of the resulting crystalline material was measure to be from about 123 °C to about 131 °C. Note that melting point for pure caffeine is reported to be from about 234 °C to about 236 °C, and the melting point for pure 5-(4-(2-(5-ethylpyridin-2-yl)-2-oxoethoxy)benzyl)-1,3-thiazolidine-2,4-dione was measured to be from about 140 °C to about 142 °C.

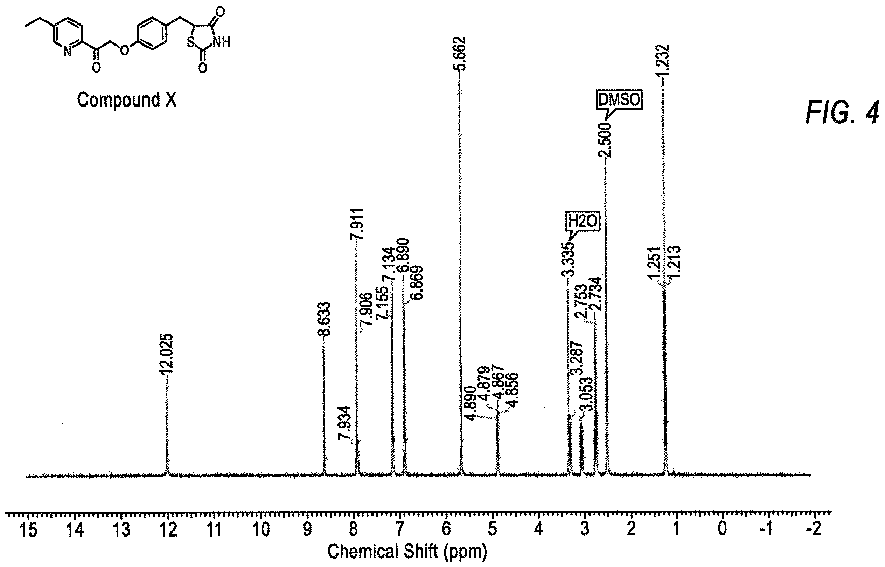

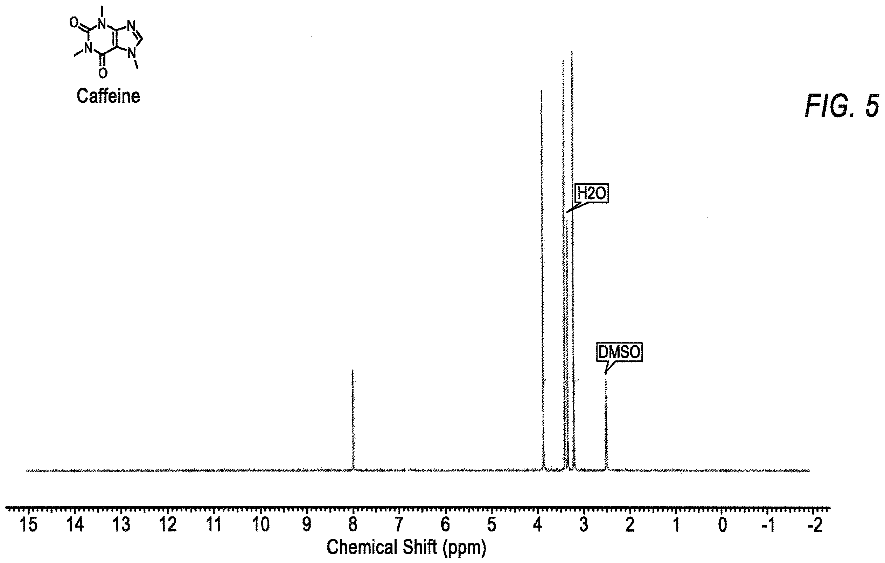

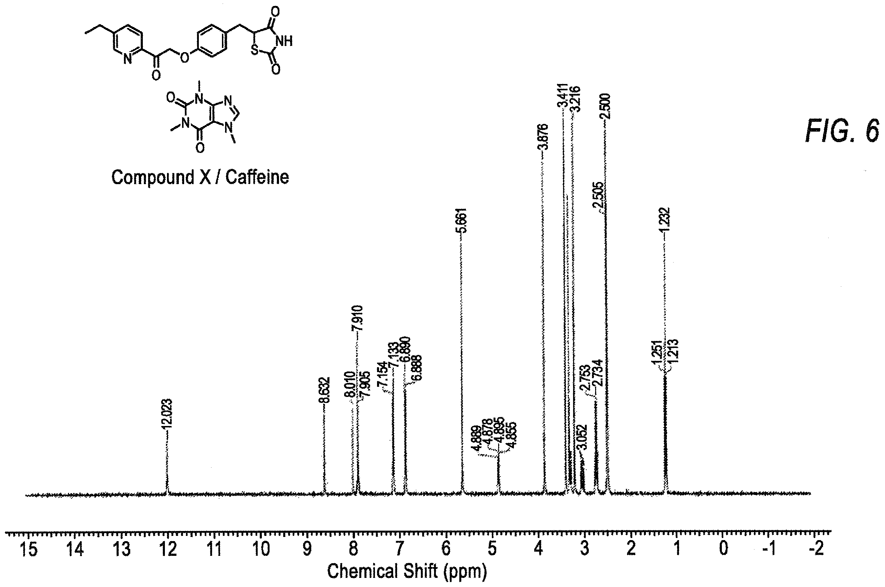

The ¹H NMR spectra of 5-(4-(2-(5-ethylpyridin-2-yl)-2-oxoethoxy)benzyl)-1,3-thiazolidine-2,4-dione, caffeine, and the co-crystal are provided in Figures 4-6. These spectra were obtained using a Bruker 400 mHz NMR spectrometer, wherein the analyte was dissolved in D6-DMSO.

### Co-Crystal B:

### To caffeine (0.194g, 1mmol) and 5-(4-(2-(3-methoxyphenyl)-2-oxoethoxy)benzyl)thiazolidine-2,4-dione (*) having the structure:

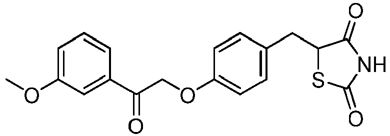

(0.371g, 1mmol) is added acetonitrile (20mL). The mixtures is warmed in a 75 °C oil bath until the solids dissolve. Warming continues for about 10 minutes, then the solution is filtered and cooled to room temperature. The solvent is evaporated until crystallization is complete. Co-crystalline solid is isolated by filtration and dries in vacuo.

### Example 13: Assays.

### Assays for Measuring Reduced PPARγ Receptor Activation

Whereas activation of the PPAPγ receptor is generally believed to be a selection criteria to select for molecules that may have anti-diabetic and insulin sensitizing pharmacology, this invention finds that activation of this receptor should be a negative selection criterion. Molecules will be chosen from this chemical space because they have reduced, not just selective, activation of PPARγ. The optimal compounds have at least a 10-fold reduced potency as compared to pioglitazone and less than 50% of the full activation produced by rosiglitazone in assays conducted in vitro for transactivation of the PPAPγ receptor. The assays are conducted by first evaluation of the direct interactions of the molecules with the ligand binding domain of PPARγ. This can be performed with a commercial interaction kit that measures the direct interaction by florescence using rosiglitazone as a positive control.

PPAPγ binding is measured by a TR-FRET competitive binding assay using Invitrogen LanthaScreen™ TR-FRET PPAPγ Competitive Binding Assay (Invitrogen #4894). This assay uses a terbium-labeled anti-GST antibody to label the GST tagged human PPAPγ ligand binding domain (LBD). A fluorescent small molecule pan-PPAR ligand tracer binds to the LBD causing energy transfer from the antibody to the ligand resulting in a high TR-FRET ratio. Competition binding by PPAPγ ligands displace the tracer from the LBD causing a lower FRET signal between the antibody and tracer. The TR-FRET ratio is determined by reading the fluorescence emission at 490 and 520nm using a Synergy2 plate reader (BioTek). The ability of several exemplary compounds described herein and of the compound of the present invention to bind to PPAPγ was also measured using a commercial binding assay (Invitrogen Corporation, Carlsbad, CA) that measures the test compounds ability to bind with PPAR-LBD/Fluormone PPAR Green complex. These assays were performed on three occasions with each assay using four separate wells (quadruplicate) at each concentration of tested compound. The data are mean and SEM of the values obtained from the three experiments. Rosiglitazone was used as the positive control in each experiment. Compounds were added at the concentrations shown, which ranged from 0.1-100 micromolar.

PPAPγ activation in intact cells may be measured by a cell reporter assay using Invitrogen GeneBLAzer PPAPγ Assay (Invitrogen #1419). This reporter assay uses the human PPAPγ ligand binding domain (LBD) fused to the GAL4 DNA binding domain (DBD) stably transfected into HEK 293H cells containing a stably expressed beta-lactamase reporter gene under the control of an upstream activator sequence. When a PPAPγ agonist binds to the LBD of the GAL4/PPAR fusion protein, the protein binds to the upstream activator sequence activating the expression of beta-lactamase. Following a 16 hour incubation with the agonists the cells are loaded with a FRET substrate for 2 hours and fluorescence emission FRET ratios are obtained at 460 and 530 nm in a Synergy2 plate reader (BioTek).

In addition to showing the reduced activation of the PPAPγ receptor in vitro, the compounds will not produce significant activation of the receptor in animals. Compounds dosed to full effect for insulin sensitizing actions in vivo (see below) will be not increase activation of PPAPγ in the liver as measured by the expression of a P2, a biomarker for ectopic adipogenesis in the liver [Matsusue K, Haluzik M, LambertG, Yim S-H, Oksana Gavrilova O, Ward JM, Brewer B,Reitman ML, Gonzalez FJ. (2003) Liver-specific disruption of PPAR in leptin-deficient mice improves fatty liver but aggravates diabetic phenotypes. J. Clin. Invest.; 111: 737] in contrast to pioglitazone and rosiglitazone, which do increase a P2 expression under these conditions.

### Mitochondrial Membrane Competitive Binding Crosslinking Assay

A photoaffinity crosslinker was synthesized by coupling a carboxylic acid analog of pioglitazone to a p-azido-benzyl group containing ethylamine as in Amer. J. Physiol 256:E252-E260. The crosslinker was iodinated carrier free using a modification of the Iodogen (Pierce) procedure and purified using open column chromatography (PerkinElmer). Specific crosslinking is defined as labeling that is prevented by the presence of competing drug. Competitive binding assays are conducted in 50 mM Tris , pH8.0. All crosslinking reactions are conducted in triplicate using 8 concentrations of competitor ranging from 0-25 µM. Each crosslinking reaction tube contains 20 µg of crude mitochondrial enriched rat liver membranes, 0.1 µCi of 125I-MSDC-1101, and -/+ competitor drug with a final concentration of 1% DMSO. The binding assay reaction is nutated at room temperature in the dark for 20 minutes and stopped by exposure to 180,000 µJoules. Following crosslinking, the membranes are pelleted at 20,000 × g for 5 minutes, the pellet is resuspended in Laemmli sample buffer containing 1% BME and run on 10-20% Tricine gels. Following electrophoresis the gels are dried under vacuum and exposed to Kodak BioMax MS film at - 80 °C. The density of the resulting specifically labeled autoradiography bands are quantitated using ImageJ software (NIH) and IC₅₀ values determined by non-linear analysis using GraphPad PrismTM . Selected compounds in this assay demonstrated an IC₅₀ of less than 20 µM, less than 5 µM or less than 1 µM. The crosslinking to this protein band is emblematic of the ability of the ability of the PPAR-sparing compounds to bind to the mitochondria, the key organelle responsible for the effectiveness of these compounds for this utility.

### Example 14: Additional Biological Properties.

5XFAD mice harbor 5 familial mutations (3 in the amyloid precursor protein; 2 in presenilin 1) and develop robust plaque pathology as early as 6 weeks. These mice were treated beginning at 2 months of age for a period of 4 weeks with control chow or chow containing Compound X to deliver 390 mg/kg for 4 weeks.

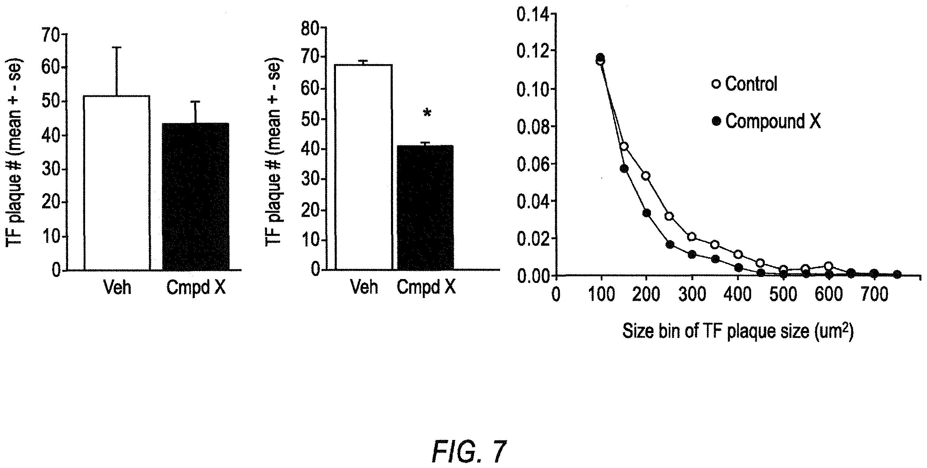

Referring to Figure 7, thioflavin S stained plaques were counted in the hippocampus of the 5XFAD mice. The data indicates that the size and number of plaques in the mice administered Compound X is less than the control group. Note that the plaques having less than 100 micron size were excluded from the graph, and those amounted to about 70% of all the plaques in both Control and Compound X treated groups.

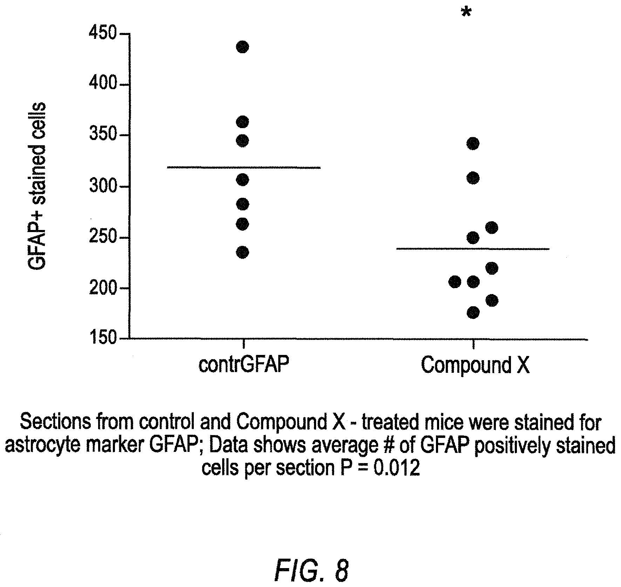

Referring to Figure 8, sections from control and Mitoglitazone treated mice were stained for astrocyte marker GFAP; Data shows average number of GFAP positively stained cells per section. P = 0.012.

## Claims

1. A sodium salt of Compound X 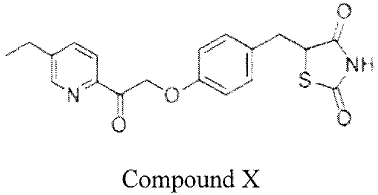 for use in treating Alzheimer's Disease.

## Patentansprüche

1. Ein Natriumsalz der Verbindung X 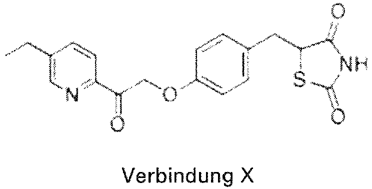 zur Verwendung in der Behandlung der Alzheimer-Erkrankung.

## Revendications

1. Un sel de sodium du composé X 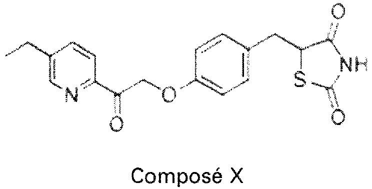 pour l'utilisation dans le traitement de la maladie d'Alzheimer.
